# EUROPÄISCHE PATENTSCHRIFT

(11) **EP 2 212 308 B1**
(45) Veröffentlichungstag und Bekanntmachung des Hinweises auf die Patenterteilung: **22.05.2013**
(21) Anmeldenummer: 08855468.8
(22) Anmeldetag: 03.11.2008
(51) Int. Cl.: C07D 311/92, C07D 493/04, C09K 19/34

(54) **BENZO[F]CHROMEN- UND PYRANO[3,2-F]CHROMEN-DERIVATE ZUR ANWENDUNG IN FLÜSSIGKRISTALLINEN MEDIEN**
BENZO[F]CHROME AND PYRANO[3,2-F]CHROME DERIVATIVES FOR USE IN LIQUID CRYSTAL MEDIA
DÉRIVÉS DE BENZO[F]CHROMÈNE ET DE PYRANO[3,2-F]CHROMÈNE DESTINÉS À ÊTRE EMPLOYÉS DANS DES SUBSTANCES DE CRISTAUX LIQUIDES

(30) Priorität: 27.11.2007 DE 102007057028
(43) Veröffentlichungstag der Anmeldung: 04.08.2010
(73) Patentinhaber: Merck Patent GmbH, 64293 Darmstadt (DE)
(72) Erfinder: JANSEN, Axel, 64293 Darmstadt (DE); KLASEN-MEMMER, Melanie, 67259 Heuchelheim (DE)
(86) Internationale Anmeldenummer: PCT/EP2008/009242
(87) Internationale Veröffentlichungsnummer: WO 2009/068152

(56) Entgegenhaltungen:
- EP-A- 1 930 396
- WO-A-2007/079840
- WO-A-2008/012180
- MATSUMOTO J ET AL: "Generation of quinone methide from aminomethyl(hydroxy)arenes precursors in aqueous solution" TETRAHEDRON, ELSEVIER SCIENCE PUBLISHERS, AMSTERDAM, NL, Bd. 61, Nr. 24, 13. Juni 2005 (2005-06-13), Seiten 5735-5740, XP025383543 ISSN: 0040-4020 [gefunden am 2005-06-13]
- ZHANGJIE SHI ET AL: "DIRECT FUNCTIONALIZATION OF ARENES BY PRIMARY ALCOHOL SOLFONATE ESTERS CATALYZED BY GOLD(III)" JOURNAL OF THE AMERICAN CHEMICAL SOCIETY, AMERICAN CHEMICAL SOCIETY, WASHINGTON, DC., US, Bd. 126, 1. Januar 2004 (2004-01-01), Seiten 13596-13597, XP002434778 ISSN: 0002-7863
- WENKERT, ERNEST ET AL: "A new synthesis of the hydrophenanthrene nucleus" JOURNAL OF THE AMERICAN CHEMICAL SOCIETY , 82, 4675-80 CODEN: JACSAT; ISSN: 0002-7863, 1960, XP002524099
- BILGIC, SEVIM ET AL: "Synthesis of chromans from the reaction of o-quinone methide precursor with substituted styrenes" JOURNAL OF CHEMICAL RESEARCH , (2), 76-79 CODEN: JCROA4, 2007, XP009115434
- BUYUKKIDAN, BULENT ET AL: "The synthesis of some novel substituted benzochromene derivatives" JOURNAL OF CHEMICAL RESEARCH, SYNOPSES , (11), 749-751 CODEN: JRPSDC; ISSN: 0308-2342, 2003, XP009115447

## Beschreibung

Die vorliegende Erfindung betrifft Benzo[f]chromen- und Pyrano[3,2-f]-chromen-Derivate, ein Verfahren zu ihrer Herstellung, flüssigkristalline Medien enthaltend diese Derivate sowie elektrooptische Anzeigeelemente enthaltend diese flüssigkristallinen Medien. Insbesondere betrifft die Erfindung Benzo[f]chromen- und Pyrano[3,2-f]chromen-Derivate mit negativer dielektrischer Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete für herkömmliche Mischungen sind insbesondere Anzeigen für Uhren und Taschenrechner sowie große Anzeigetafeln, wie sie in Bahnhöfen, Flughäfen und Sportarenen verwendet werden. Weitere Anwendungsgebiete sind Displays von tragbaren und stationären Computern, Navigationssystemen und Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante ε_{∥} parallel zu den Moleküllängsachsen größer als die Dielektrizitätskonstante ε^{⊥} senkrecht zu den Moleküllängsachsen, so ist die dielektrische Anisotropie Δε = ε_{∥} - ε_{⊥} größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch den Einbau geeigneter polarer Gruppen, wie z.B. von Nitrilgruppen oder Fluor, in die Flüssigkristallmoleküle lässt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Bei den am weitesten verbreiteten TN-Zellen (abgeleitet aus dem Englischen: "twisted nematic", verdrillt nematisch) ist eine nur etwa 5 bis 10 µm dicke flüssigkristalline Schicht zwischen zwei planparallelen Glasplatten angeordnet, auf die jeweils eine elektrisch leitende, transparente Schicht aus Zinnoxid oder Indium-Zinnoxid (ITO) als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so dass sie im spannungsfreien Zustand einheitlich mit der gleichen Orientierung flach oder mit demselben kleinen Anstellwinkel (englisch: "tilt angle") auf der Innenseite der Displayfläche aufliegen. Auf der Außenseite des Displays sind zwei Polarisationsfolien, die nur linear polarisiertes Licht ein- und austreten lassen, in einer bestimmten Anordnung aufgebracht.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen anzustreben. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtern sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist in diesem Fall negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In der Druckschrift DE 10 2004 004 228 A1 werden Benzochromenderivate folgender Struktur offenbart:

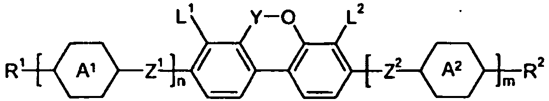

Diese Tricyclen unterscheiden sich von der vorliegenden Erfindung durch die Lage der Heteroatome in den Ringen, die Substitution und den Sättigungsgrad der Ringe. Weitere Benzochromenderivate, die teilweise ungesättigt sind und optional mehrere Heteroatome besitzen werden außerdem in der Druckschrift WO 2007/079840 A2 offenbart.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem zu nennen ein hoher Klärpunkt, eine geringe Rotationsviskosität, eine optische Anisotropie im Anwendungsintervall, sowie die Eigenschaften, die zur Erzielung von Mischungen mit den gewünschten flüssigkristallinen Phasen über einen breiten Temperaturbereich dienen.

Diese Aufgabe wird erfindungsgemäß gelöst durch Verbindungen der allgemeinen Formel I 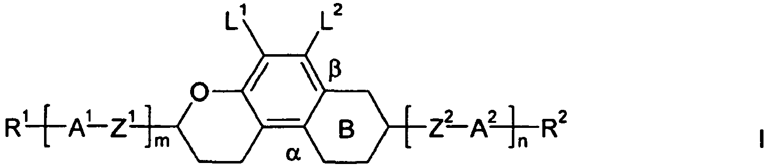 worin
- m und n: jeweils unabhängig voneinander 0, 1, 2 oder 3 sind, bevorzugt 0, 1 oder 2, wobei m + n ≤ 4;
- L¹ und L²: jeweils unabhängig voneinander H, Halogen, CN, CF₃, CHF₂, CH₂F und CH₃ bedeuten, bevorzugt H, F, Cl, CN oder CF₃, wobei einer der Reste L¹ oder L² oder beide ein F bedeuten,
- A¹ und A²: jeweils unabhängig voneinander
(a) 1,4-Phenylen, worin =CH- ein- oder zweimal durch =N-ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann,
(b) 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F und/oder -Cl substituiert sein können, oder
(c) Bicyclo[1.1.1]pentan-1,3-diyl, Bicyclo[2.2.2]octan-1,4-diyl oder Spiro[3.3]heptan-2,6-diyl,
bedeutet;
- Z¹ und Z²: jeweils unabhängig voneinander eine Einfachbindung, -CF₂O-, -OCF₂-, -CH₂CH₂-, -CF₂CF₂-, -CF₂CH₂-, -CH₂CF₂-, -(CO)O-, -O(CO)-, -CH₂O-, -OCH₂-, -CF=CH-, -CH=CF-, -CF=CF-, -CH=CH-, -C≡C- oder eine Kombination aus zwei dieser Gruppen, wobei O-Atome nicht direkt verknüpft sind, bedeuten;
- R¹ und R²: unabhängig voneinander, Wasserstoff, einen unsubstituierten, einen einfach mit -CN oder einen einfach oder mehrfach mit -F, -Cl, und/oder -Br substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -(CO)O-, -O(CO)-oder -O(CO)O- so ersetzt sein können, dass Heteroatome nicht direkt verknüpft sind, -F, -Cl, -Br, -CN, -SCN oder -SF₅ bedeuten; und
- Ring B: 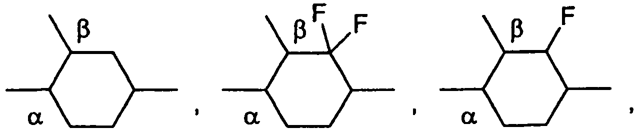 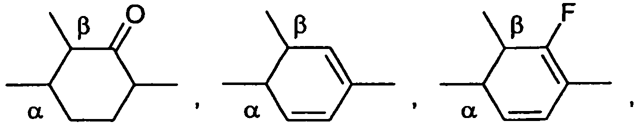 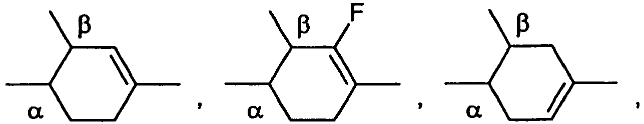 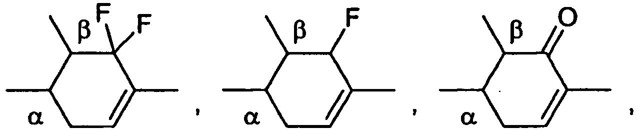 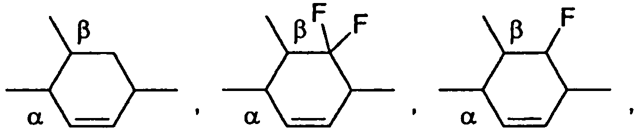 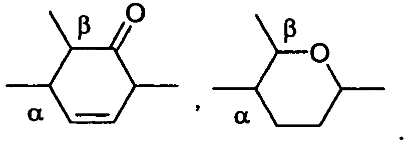 wobei
A¹ und A² bzw. Z¹ und Z² jeweils die gleiche oder unterschiedliche Bedeutungen haben können, wenn m oder n größer als 1 sind, wobei
R¹ und R² nicht gleichzeitig H bedeuten, und
wobei
für den Fall, dass
R¹ = H und m = 0 oder R² = H und n = 0 bedeuten, dann
der Ring B ein Pyran der Formel 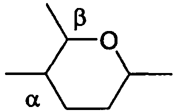 bedeutet.

Die Verbindungen besitzen überwiegend ein negatives Δε und eignen sich daher insbesondere für eine Verwendung in VA-TFT-Displays. Vorzugsweise besitzen die erfindungsgemäßen Verbindungen ein Δε < -2 und besonders bevorzugt ein Δε < -4. Sie zeigen eine sehr gute Verträglichkeit mit den üblichen, in Flüssigkristallmischungen für Displays verwendeten Substanzen.

Ferner weisen die erfindungsgemäßen Verbindungen der Formel I insbesondere für die Verwendung in VA-TFT-Displays geeignete Werte der optischen Anisotropie Δn auf. Bevorzugt besitzen die erfindungsgemäßen Verbindungen ein Δn von größer als 0,05 und kleiner als 0,40.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die Verbindungen bzw. flüssigkristallinen Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die Rotationsviskositäten der Verbindungen sind vorteilhafterweise klein, besonders für m + n = 0.

Unter den Bedeutungen für die Substituenten L¹ und L² sind H, F und Cl bevorzugt, insbesondere H und F. Bevorzugt bedeutet L¹ ein Fluor. Besonders bevorzugt bedeuten L¹ und L² F.

Für den Fall, dass der Rest R² ein Fluoratom oder fluoriertes Alkyl bedeutet, insbesondere wenn gleichzeitig m 0 bedeutet, dann sind unter der Formel I auch Verbindungen umfasst, die eine insgesamt positive dielektrische Anisotropie besitzen können. Solche Verbindungen eignen sich dann für dielektrisch positive Flüssigkristallmischungen, die in Displays wie z. B. vom Typ des TN-TFT oder IPS ('in-plane-switching') verwendet werden. Die Anforderungen an die übrigen physikalischen Parameter, wie z.B. der Viskosität, sind über die meisten Anwendungen hinweg weitgehend kongruent. Die genannten Verbindungen sind also gleichermaßen für diese Zwecke geeignet, weil sie günstige Werte für die Parameter wie Rotationsviskosität, Δn, etc. haben und sich für den Aufbau flüssigkristalliner Mischungen eignen.

A¹ und A² sind bevorzugt und unabhängig voneinander ein optional substituiertes 1,4-Phenylen, ein optional substituiertes 1,4-Cyclohexylen, worin -CH₂- ein- oder zweimal durch -O- ersetzt sein kann, oder ein gegebenenfalls substituiertes 1,4-Cyclohexenylen. Wenn n oder m 2 sind, können die Ringe A¹ beziehungsweise A² die gleichen oder unterschiedliche Bedeutungen annehmen.

Besonders bevorzugt sind A¹ und A² unabhängig voneinander 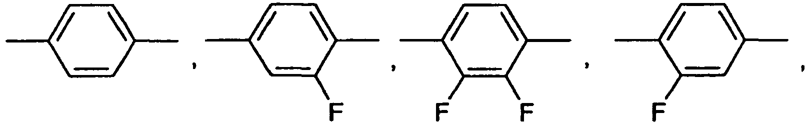 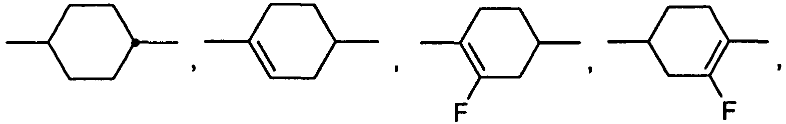 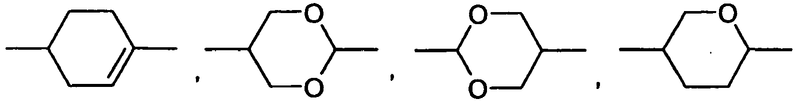 oder

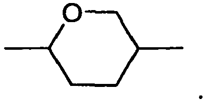

Ganz besonders bevorzugt sind A¹ und A² 1,4-Cyclohexylen und/oder optional mit Fluor substituiertes 1,4-Phenylen.

In einer bevorzugten Ausführungsform der Erfindung ist m + n gleich 1 und
- A¹, A²: bedeuten unabhängig voneinander

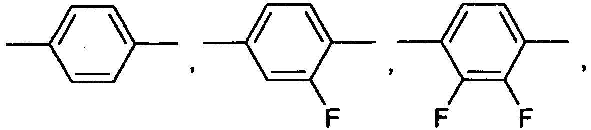

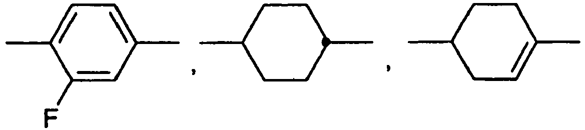

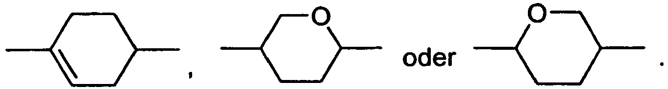

Bevorzugt sind Z¹ und Z² unabhängig voneinander eine Einfachbindung, -CH₂O-, -OCH₂-, -CF₂O-, -OCF₂-, -CF₂CF₂-, -CH=CH-, -CF=CH-, -CH=CF-oder -CF=CF-, besonders bevorzugt unabhängig voneinander eine Einfachbindung, -CF₂O-, -OCF₂-, -CH₂O-, -OCH₂-, -CF₂CF₂-, -CH=CH-, -CF=CH-, -CH=CF- oder -CF=CF-. Ganz besonders bevorzugt sind Z¹ und Z² unabhängig voneinander eine Einfachbindung, -CF₂O-, -OCF₂-, -CH₂O-, -OCH₂- oder -CF=CF-, insbesondere eine Einfachbindung.

Die Parameter m und n besitzen in der Summe m + n bevorzugt einen Wert von 0, 1, 2 oder 3, besonders 0, 1 oder 2. Für m + n = 0 bedeutet bevorzugt wenigstens eine Gruppe aus L¹ und L² F, besonders bevorzugt beide Gruppen. Außerdem bedeuten für m + n = 0 R¹ und R² bevorzugt keinen Wasserstoff.

Bevorzugt bedeuten R¹ und R² jeweils unabhängig voneinander einen Alkanylrest, Alkoxyrest oder Alkenylrest mit 1 bis 7 bzw. 2 bis 7 Kohlenstoffatomen sind, wobei jeder dieser Reste unsubstituiert oder mit Halogen einfach oder mehrfach substituiert ist, oder Fluor oder Wasserstoff, besonders bevorzugt einen Alkanylrest, Alkoxyrest oder Alkenylrest wie beschrieben.

Besonders bevorzugt sind R¹ und R² unabhängig voneinander in der allgemeinen Formel I ein Alkanylrest, Alkoxyrest oder Alkenylrest mit 1 bis 7 bzw. 2 bis 7 C-Atomen, wobei jeder dieser Reste bevorzugt unsubstituiert oder mit Halogen einfach oder mehrfach substituiert ist. Für den Fall, dass m = 0 ist, bedeutet R¹ vorzugsweise eine Alkyl- oder Alkoxygruppe, H oder F, besonders bevorzugt eine Alkylgruppe mit 1-6 C-Atomen.

Sofern R¹ und R² in Formel I jeweils unabhängig voneinander einen Alkanylrest und/oder einen Alkoxyrest (Alkyloxyrest) mit 1 bis 15 C-Atomen darstellen, sind diese geradkettig oder verzweigt. Vorzugsweise ist jeder dieser Reste geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R¹ und R² in Formel I können jeweils unabhängig voneinander auch ein Oxaalkylrest sein, d.h. ein Alkanylrest, in dem mindestens eine der nichtterminalen CH₂-Gruppen des Alkanylrests durch -O- ersetzt ist, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl. In entsprechender Weise können R¹ und R² in Formel I auch unabhängig voneinander Thioalkanyl- bzw. Sulfonalkanylreste sein, d.h. Alkanylreste, in denen eine CH₂-Gruppe durch -S- beziehungsweise -SO₂- ersetzt ist.

R¹ und R² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im allgemeinen sind die jeweiligen E-Isomeren bevorzugt.

In gleicher Weise wie bei einem Alkanylrest kann auch bei einem Alkenylrest wenigstens eine der CH₂-Gruppen durch Sauerstoff, Schwefel oder -SO₂- ersetzt sein. Bei dem Ersatz durch -O- liegt dann ein Alkenyloxyrest (mit entständigem Sauerstoff) beziehungsweise ein Oxaalkenylrest (mit nicht-terminalem Sauerstoff) vor.

R¹ und R² in Formel I können unabhängig voneinander auch ein Alkinylrest mit 2 bis 15 C-Atomen sein, der geradkettig oder verzweigt ist und wenigstens eine C-C-Dreifachbindung aufweist.

R¹ und R² in Formel I können jeweils unabhängig voneinander ein Alkanylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser Rest geradkettig und hat 2 bis 6 C-Atome. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxy-methyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)-propyl oder 4-(Methoxycarbonyl)butyl. Ferner kann ein Alkanylrest auch eine -O-CO-O-Einheit aufweisen. Auch der Ersatz einer CH₂-Gruppe durch lediglich eine -CO-Gruppe (Carbonylfunktion) ist möglich.

R¹ und R² in Formel I können jeweils unabhängig voneinander ein Alkenylrest mit 2 bis 15 C-Atomen sein, in dem eine CH₂-Gruppe bevorzugt in Nachbarschaft zu einer unsubstituierten oder substituierten -C=C-Einheit durch -CO-, -CO-O-, -O-CO- oder -O-CO-O- ersetzt ist, wobei dieser Rest geradkettig oder verzweigt sein kann. Vorzugsweise ist der Rest geradkettig und hat 4 bis 13 C-Atome. Besonders bevorzugt sind dabei Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl oder 8-Methacryloyloxyoctyl. Entsprechend kann auch in einem Alkinylrest in Nachbarschaft zu einer substituierten -C≡C-Einheit eine CH₂-Gruppe durch -CO-, -CO-O-, -O-CO- oder -O-CO-O- ersetzt sein.

R¹ und R² in Formel I können jeweils unabhängig voneinander ein einfach durch -CN substituierter Alkanylrest oder Alkoxyrest mit 1 bis 15 C-Atomen oder ein Alkenylrest oder Alkinylrest mit 2 bis 15 C-Atomen sein, wobei diese vorzugsweise geradkettig sind. Die Substitution durch -CN ist in beliebiger Position möglich.

R¹ und R² in Formel I können jeweils unabhängig voneinander ein Alkanylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R¹ und R² in Formel I können jeweils unabhängig voneinander ein einfach oder mehrfach mit F, Cl und/oder Br substituierter Alkanylrest oder Alkoxyrest mit 1 bis 15 C-Atomen oder Alkenylrest oder Alkinylrest mit 2 bis 15 C-Atomen sein, wobei diese Reste vorzugsweise geradkettig sind und Halogen vorzugsweise -F und/oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor-oder Chlorsubstituent in beliebiger Position sein, vorzugsweise ist er in ω-Position.

R¹ und R² in Formel I können auch jeweils unabhängig voneinander -F, -Cl, -Br, -CN, -SCN, -NCS oder -SF₅ sein. In diesem Fall erhöht sich die dielekrische Anisotropie hin zu positiveren Werten. Für ganz besonders stark negative dielektrische Anisotropien sind diese Substituenten nicht auszuwählen. Für hohes Δε sind sie allerdings bevorzugt.

Halogen bedeutet im Zusammenhang der vorliegenden Erfindung Fluor, Chlor, Brom und Iod, insbesondere Fluor oder Chlor.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen. Sofern es sich bei diesem Alkylrest um einen gesättigten Rest handelt, wird er auch als "Alkanyl" bezeichnet.

Besonders bevorzugt sind erfindungsgemäßen Verbindungen der Formel I ausgewählt aus den Unterformeln IA bis IF 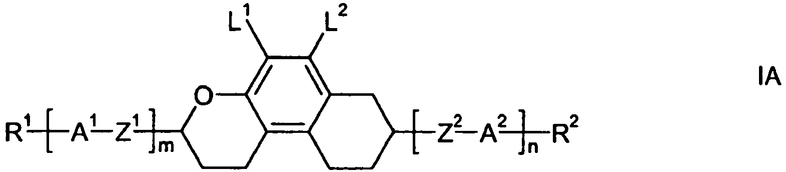 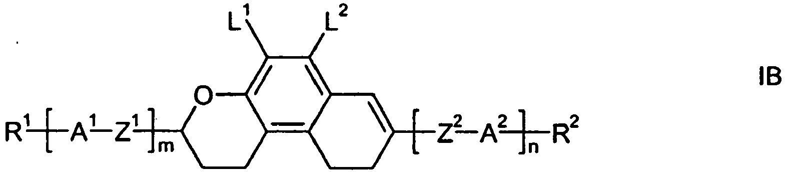 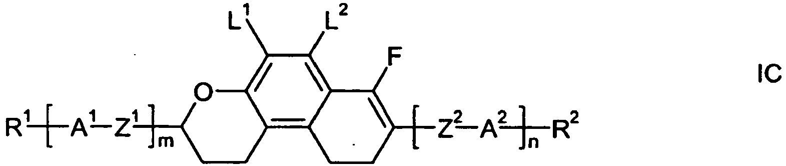 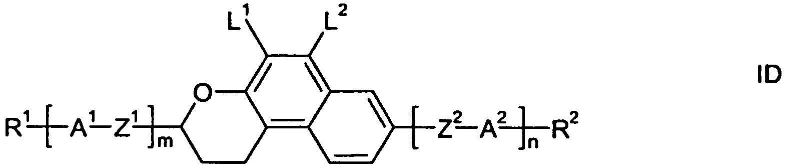 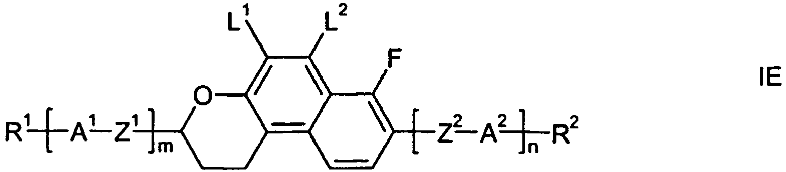 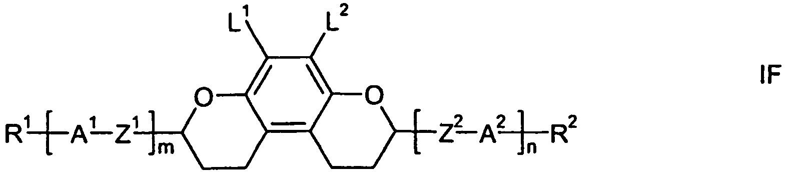 worin R¹, R², A¹, A², L¹, L², m, n, Z¹ und Z² die gleichen sowie die gleichen bevorzugten Bedeutungen, wie für Formel I oben definiert, besitzen.

Soweit A¹ und A², beziehungsweise Z¹ und Z² in den Formeln IA bis IF zweifach vorkommen, können sie jeweils die gleiche oder verschiedene Bedeutung aufweisen.

Ganz besonders bevorzugte Verbindungen der Formel IA, für die n + m = 0 gilt, sind die Folgenden: 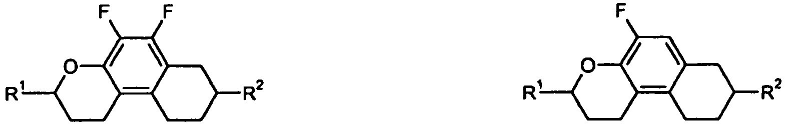 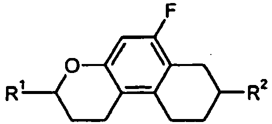 worin
R¹, R² bevorzugt einen Alkyl- oder Alkoxyrest mit bis zu 8 C-Atomen bedeuten.

Vor- und nachstehend bedeutet in den Unterformel der Molekülteil 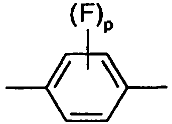 worin p 0, 1, 2, 3, oder 4 bedeutet,
jeweils unabhängig voneinander bevorzugt einen Molekülteil der Formeln 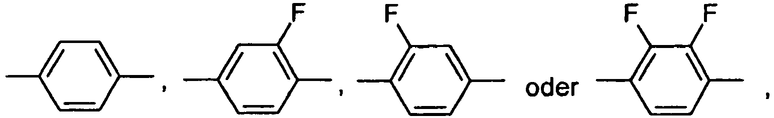 und besonders bevorzugt

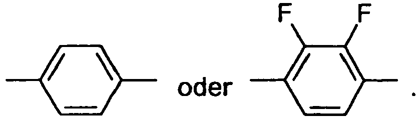

Soweit der Zähler p in den vor- und nachstehenden Formeln mehrmals vorkommt, kann er bei jedem Auftreten jeweils die gleiche oder verschiedene Bedeutung aufweisen, bevorzugt 0, 1 oder 2.

Ganz besonders bevorzugte Verbindungen der Formel IA, für die n+m = 1 gilt sind die Folgenden: 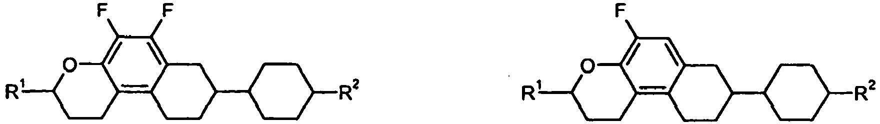 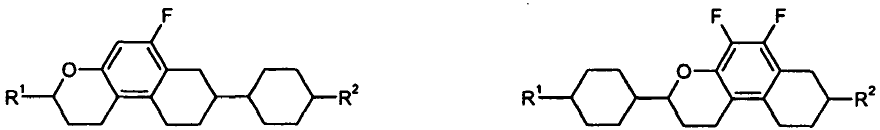 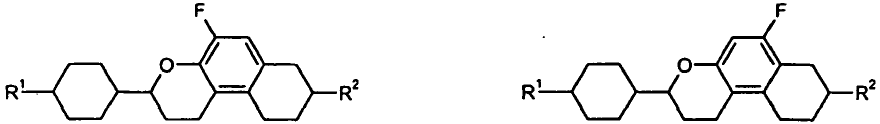 worin
R¹ und R² die gleichen Bedeutungen wie oben definiert besitzen.

Ganz besonders bevorzugte Verbindungen der Formel IA, für die n+m = 2 gilt sind die Folgenden: 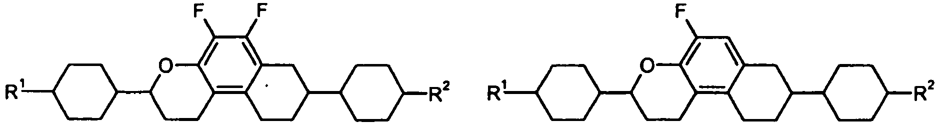 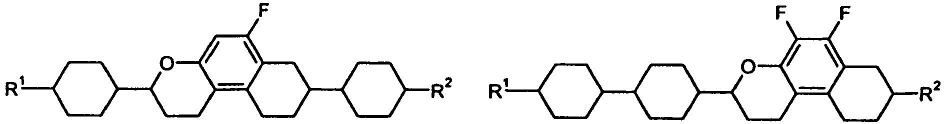 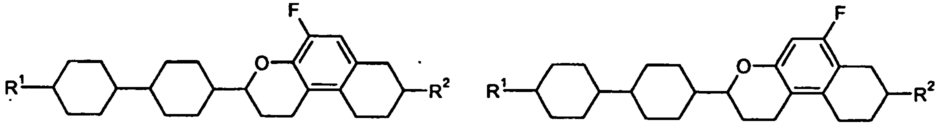 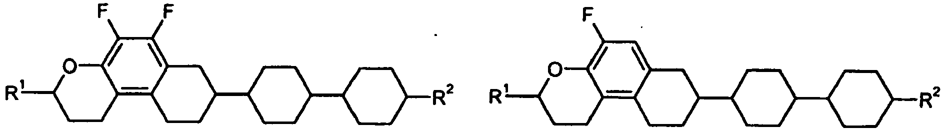 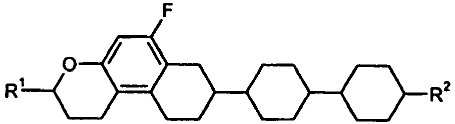 worin
R¹ und R² die gleichen Bedeutungen wie oben definiert besitzen.

Ganz besonders bevorzugte Verbindungen der Formel IB, für die n+m = 0 gilt, sind die Folgenden: 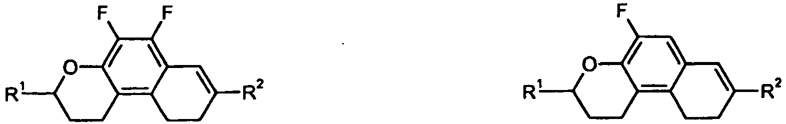 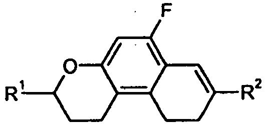 worin
R¹ und R² bevorzugt einen Alkyl- oder Alkoxyrest mit bis zu 8 C-Atomen bedeuten.

Ganz besonders bevorzugte Verbindungen der Formel IB, für die n+m = 1 gilt sind die Folgenden: 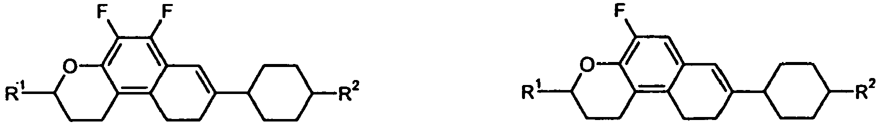 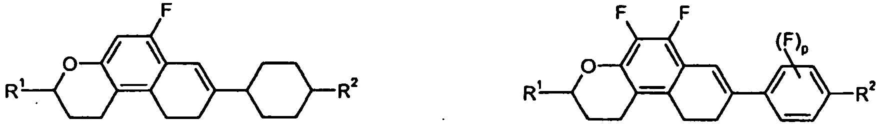 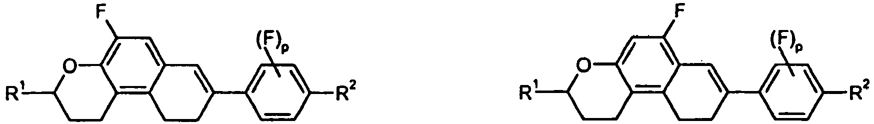 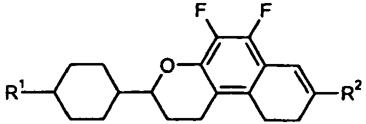 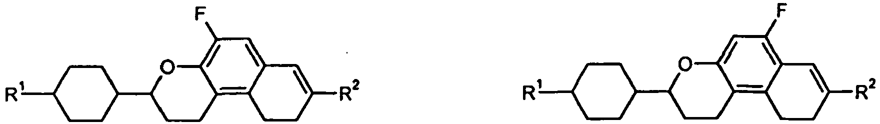 worin
R¹, R² und p die gleichen Bedeutungen wie oben definiert besitzen.

Ganz besonders bevorzugte Verbindungen der Formel IB, für die n+m = 2 gilt sind die Folgenden: 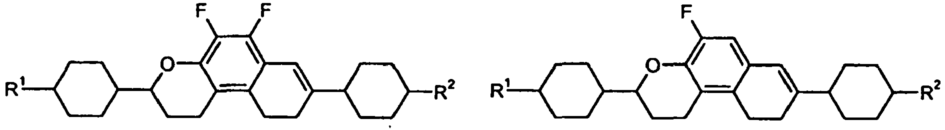 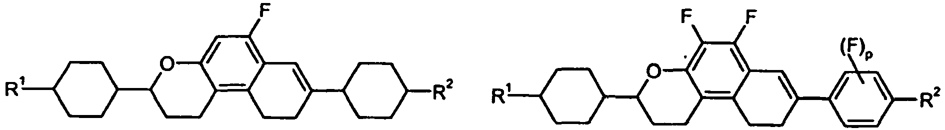 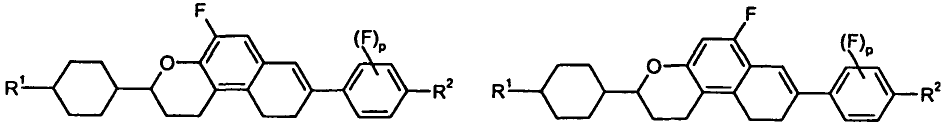 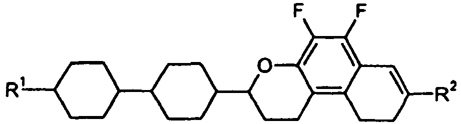 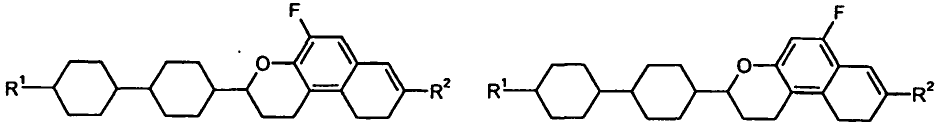 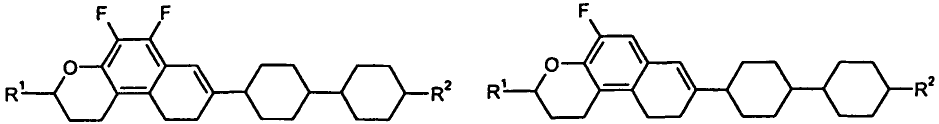 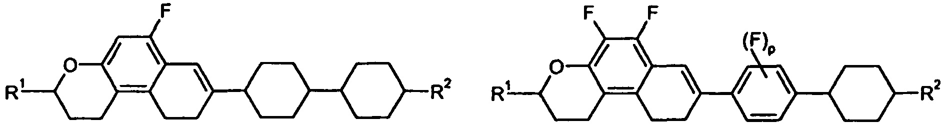 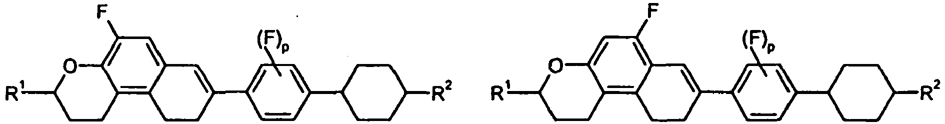 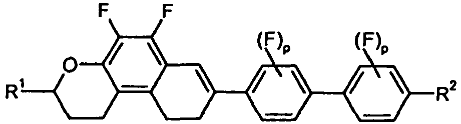 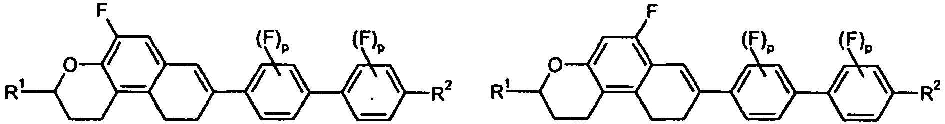 worin
R¹, R² und p die gleichen Bedeutungen wie oben definiert besitzen.

Ganz besonders bevorzugte Verbindungen der Formel IC, für die n+m = 0 gilt, sind die Folgenden: 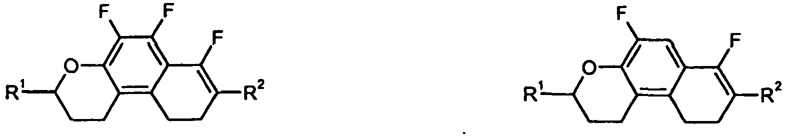 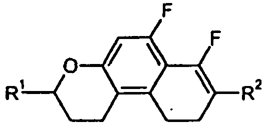 worin
R¹, R² bevorzugt einen Alkyl- oder Alkoxyrest mit bis zu 8 C-Atomen bedeutet.

Ganz besonders bevorzugte Verbindungen der Formel IC, für die n+m = 1 gilt sind die Folgenden: 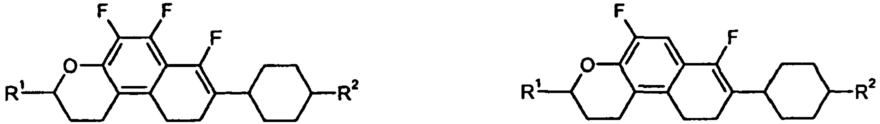 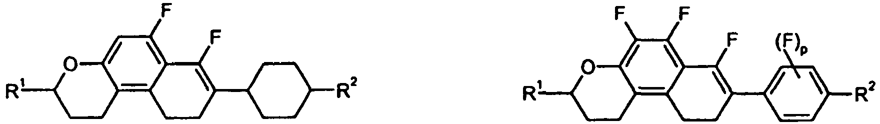 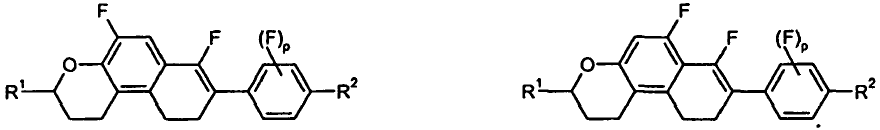 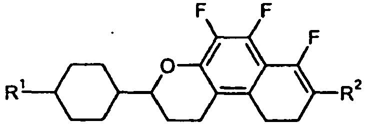 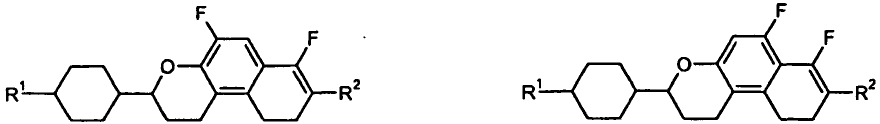 worin
R¹, R² und p die gleichen Bedeutungen wie oben definiert besitzen.

Ganz besonders bevorzugte Verbindungen der Formel IC, für die n+m = 2 gilt sind die Folgenden: 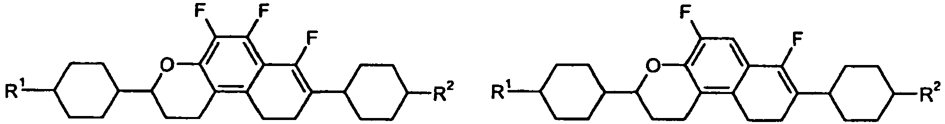 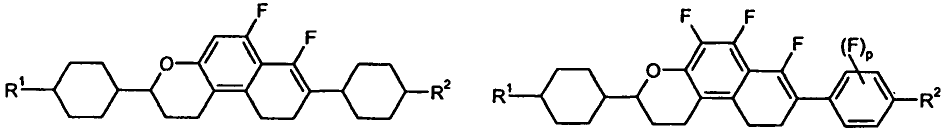 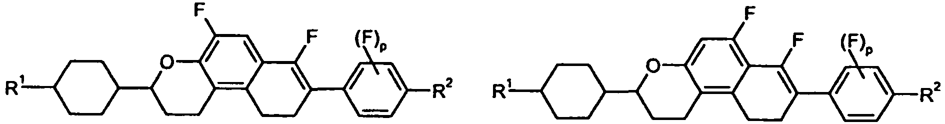 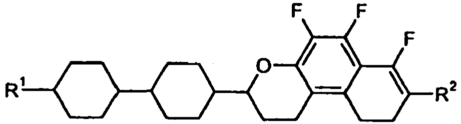 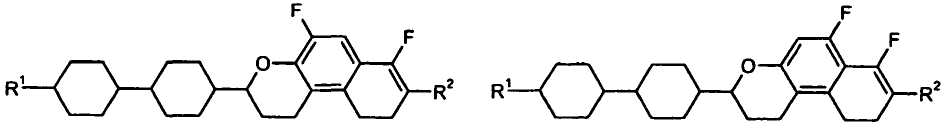 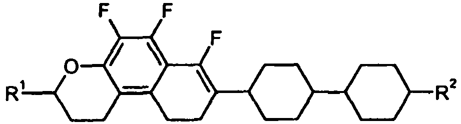 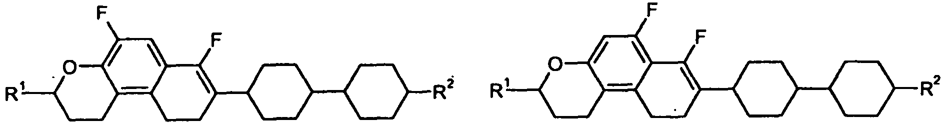 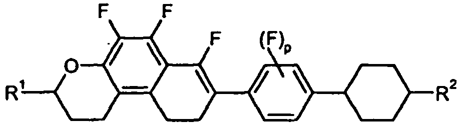 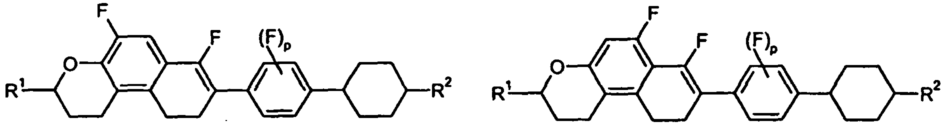 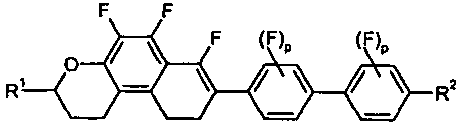 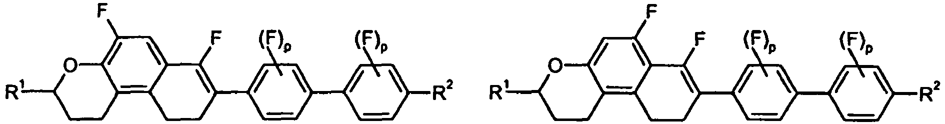 worin
R¹, R² und p die gleichen Bedeutungen wie oben definiert besitzen.

Ganz besonders bevorzugte Verbindungen der Formel ID, für die n+m = 0 gilt, sind die Folgenden: 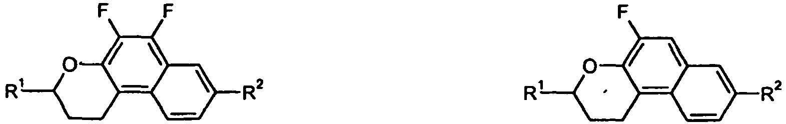 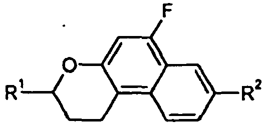 worin
R¹, R² bevorzugt einen Alkyl- oder Alkoxyrest mit bis zu 8 C-Atomen bedeutet.

Ganz besonders bevorzugte Verbindungen der Formel ID, für die n+m = 1 gilt sind die Folgenden: 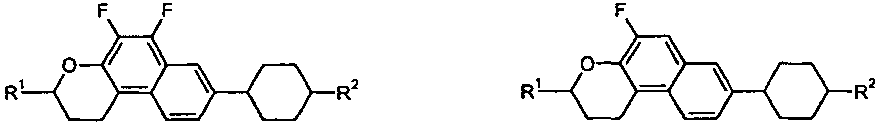 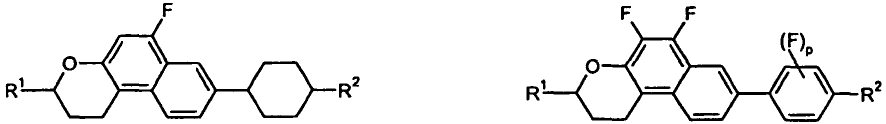 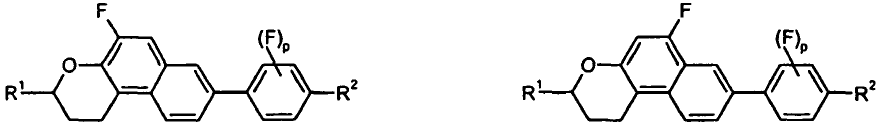 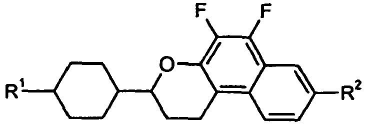 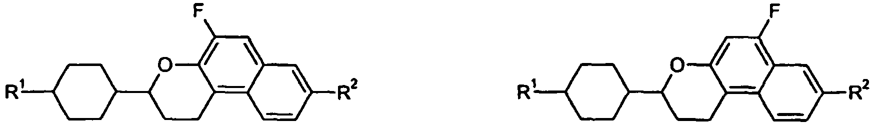 worin
R¹, R² und p die gleichen Bedeutungen wie oben definiert besitzen.

Ganz besonders bevorzugte Verbindungen der Formel ID, für die n+m = 2 gilt sind die Folgenden: 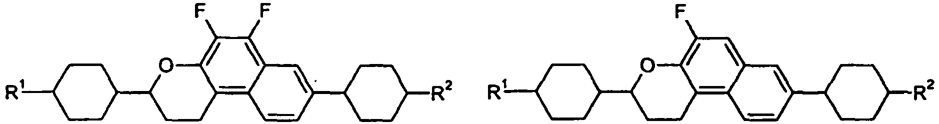 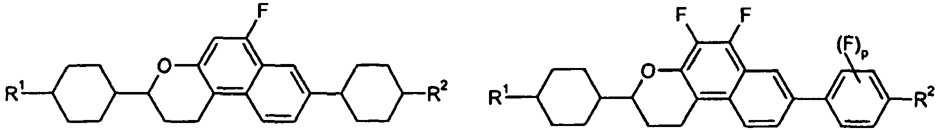 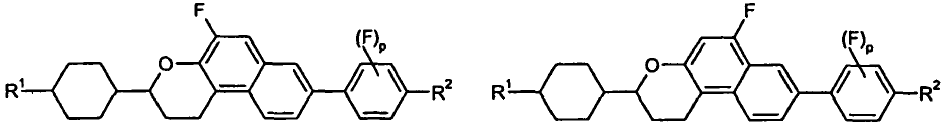 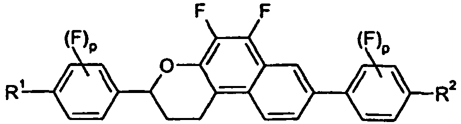 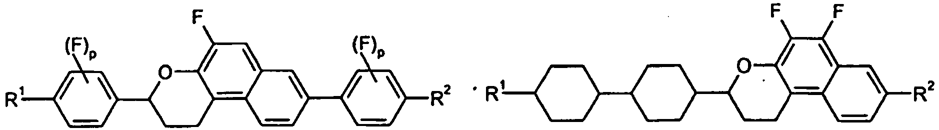 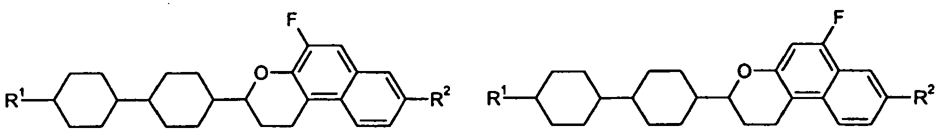 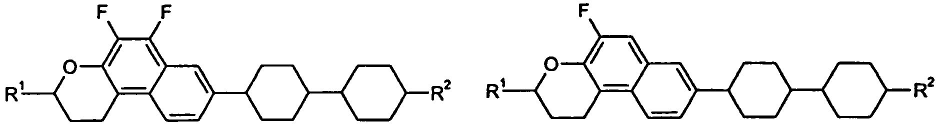 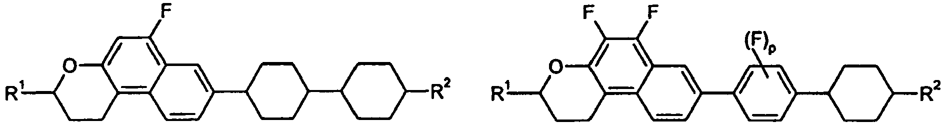 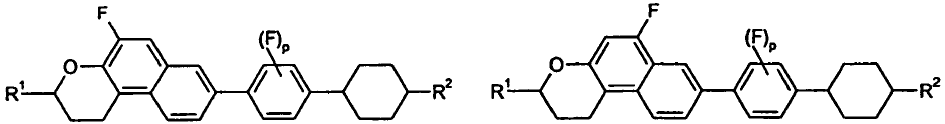 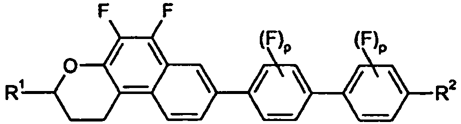 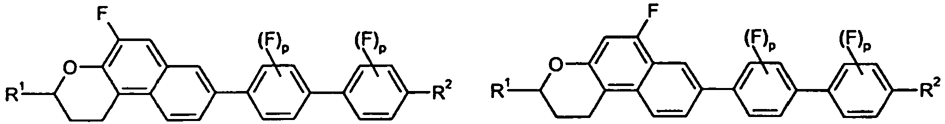 worin
R¹, R² und p die gleichen Bedeutungen wie oben definiert besitzen.

Ganz besonders bevorzugte Verbindungen der Formel IE, für die n+m = 0 gilt, sind die Folgenden: 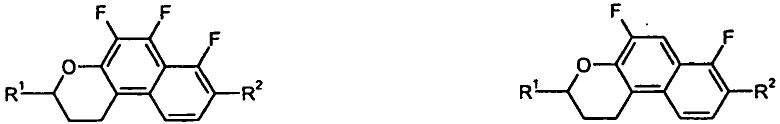 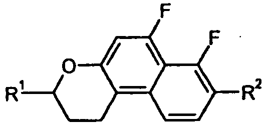 worin
R¹, R² bevorzugt einen Alkyl- oder Alkoxyrest mit bis zu 8 C-Atomen bedeutet.

Ganz besonders bevorzugte Verbindungen der Formel IE, für die n+m = 1 gilt sind die Folgenden: 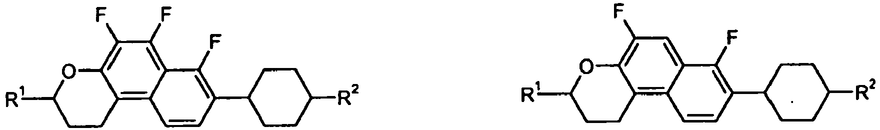 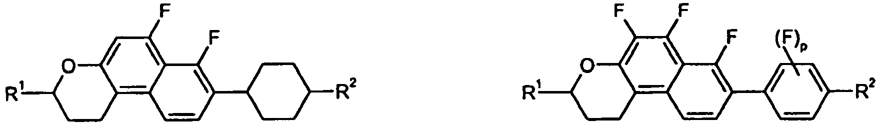 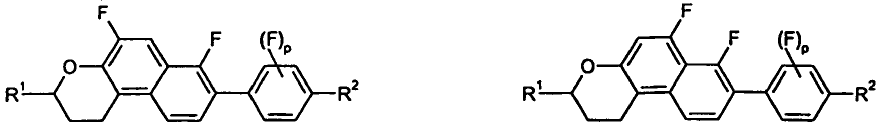 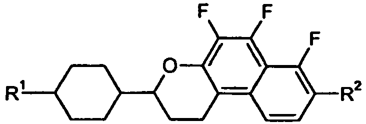 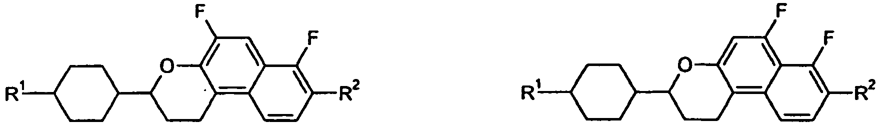 worin
R¹, R² und p die gleichen Bedeutungen wie oben definiert besitzen.

Ganz besonders bevorzugte Verbindungen der Formel IE, für die n+m = 2 gilt sind die Folgenden: 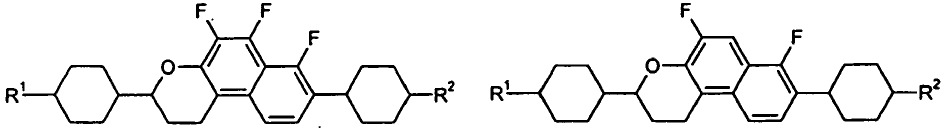 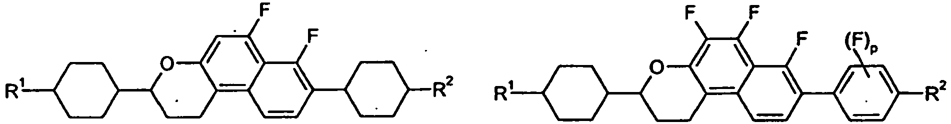 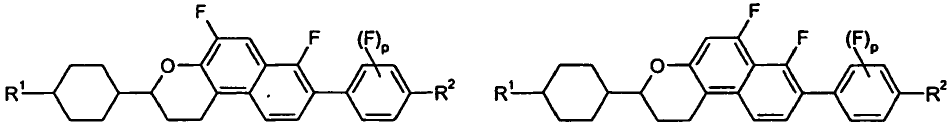 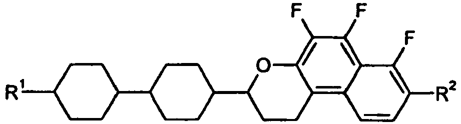 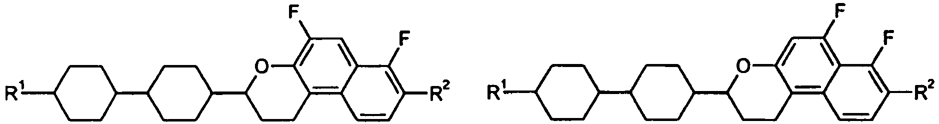 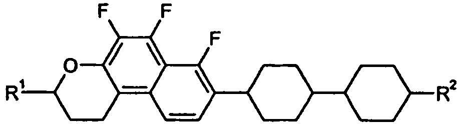 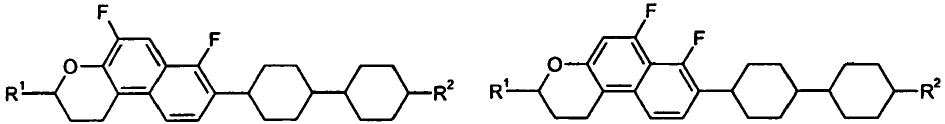 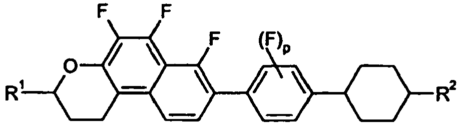 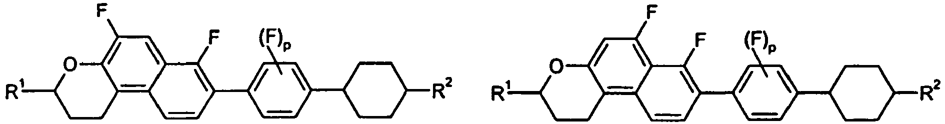 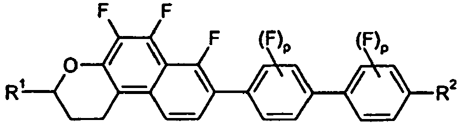 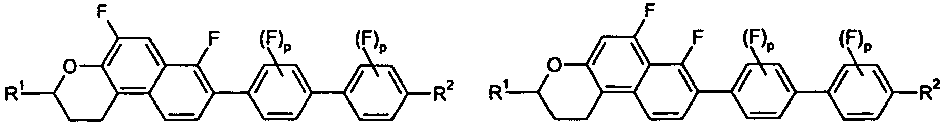 worin
R¹, R² und p die gleichen Bedeutungen wie oben definiert besitzen.

Ganz besonders bevorzugte Verbindungen der Formel IF, für die n+m = 0 gilt, sind die Folgenden: 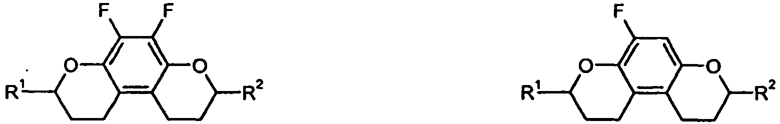 worin
R¹, R² bevorzugt einen Alkyl- oder Alkoxyrest mit bis zu 8 C-Atomen bedeutet.

Ganz besonders bevorzugte Verbindungen der Formel IF, für die n+m = 1 gilt sind die Folgenden: 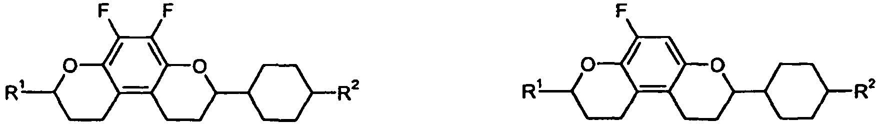 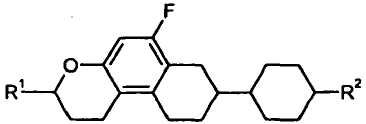 worin
R¹ und R² die gleichen Bedeutungen wie oben definiert besitzen.

Ganz besonders bevorzugte Verbindungen der Formel IF, für die n+m = 2 gilt sind die Folgenden: 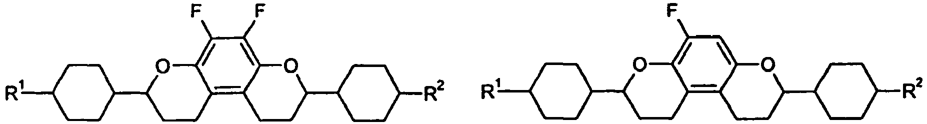 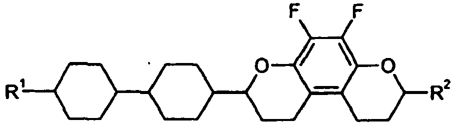 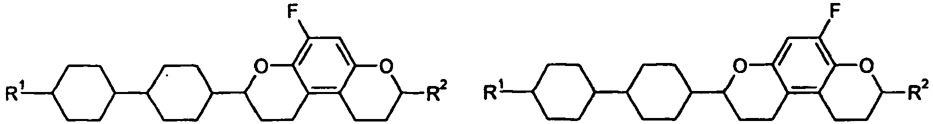 worin
R¹ und R² die gleichen Bedeutungen wie oben definiert besitzen.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Der 1,4-substituierte Cyclohexylring der Formel 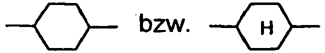 ist in den erfindungsgemäßen Verbindungen und in den übrigen Komponenten für flüssigkristalline Medien bevorzugt trans-konfiguriert, d.h. die beiden Substituenten befindend sich in der thermodynamisch bevorzugten Sesselkonformation beide in äquatorialer Position.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch *in situ* gebildet werden, derart, dass man sie aus dem Reaktionsgemisch nicht isoliert, sondern sofort weiter zu den Verbindungen der allgemeinen Formel I umsetzt.

Die Synthesen verschiedener erfindungsgemäßer Verbindungen der allgemeinen Formel I werden in den Beispielen exemplarisch beschrieben.
Die Ausgangssubstanzen sind nach allgemein zugänglichen Literaturvorschriften oder käuflich zu erhalten.

Besonders geeignete Synthesewege zu den erfindungsgemäßen Verbindungen werden im Folgenden anhand der Schemata I bis XXX erläutert.
Die Substituenten R¹, R², A¹, A², Z¹, Z² und die Zähler m und n besitzen in den folgenden Schemata wenigstens die Bedeutungen wie für die Formel I; R¹ und R² können optional auch eine derivatisierbare Gruppe ausgewählt aus OTs (Tosylat), OTf (Triflat), OMes (Mesylat), -B(OH)₂, -B(OAlkyl)₂ oder -B<(O-C₂₋₁₀-Alkylen-O) bedeuten. Die Substituenten R¹¹ und R²² besitzen ebenfalls die so erweiterte Bedeutung von R¹/R².
Die Substituenten A²² und Z²² sind allgemein wie die Reste A² bzw. Z² definiert; dabei sind die Variablen in der Gruppe -[Z²²-A²²]ₙ-R²² derart ausgewählt, dass die Gruppe zusammen mit der nächstgelegenen Gruppe zwischen -[Z²²-A²²]ₙ-R²² und dem Tricyclus insgesamt einem Rest der Formel -[Z²-A²]ₙ-R² entspricht. Somit soll beispielsweise eine Gruppe der Formel -CH=CH-[Z²²-A²²]ₙ-R²² oder auch der Formel -Ph-[Z²²-A²²]ₙ-R²² formal einem Rest der Formel -[Z²-A²]ₙ-R² entsprechen.

Die Synthese der Verbindungen der Formel I erfolgt bevorzugt ausgehend von den Verbindungen **2** (vgl. **Schema I**).

Verbindungen gemäß **2** werden mit Propargylalkoholen **3** (*Methode A*) [O. Mitsunobu, Synthesis 1981, 1] oder Propargylbromiden **4** (*Methode B*) zu den entsprechenden Verbindungen **5** verethert. Die Propargylarylether **5** untergehen beim Erhitzen in *N,N*-Diethylanilin eine [3.3]-sigmatrope Umlagerung zu den Chromenderivaten **6** [H. Ishii, T. Ishikawa, S. Takeda, S. Ueki, M. Suzuki, Chem. Pharm. Bull. 1992, 40, 1148-1153]**.** Eine. abschließende Hydrierung liefert dann die Zielverbindungen **1,** die den erfindungsgemäßen Verbindungen der Formel I oder einem Zwischenprodukt zu deren Herstellung entsprechen.

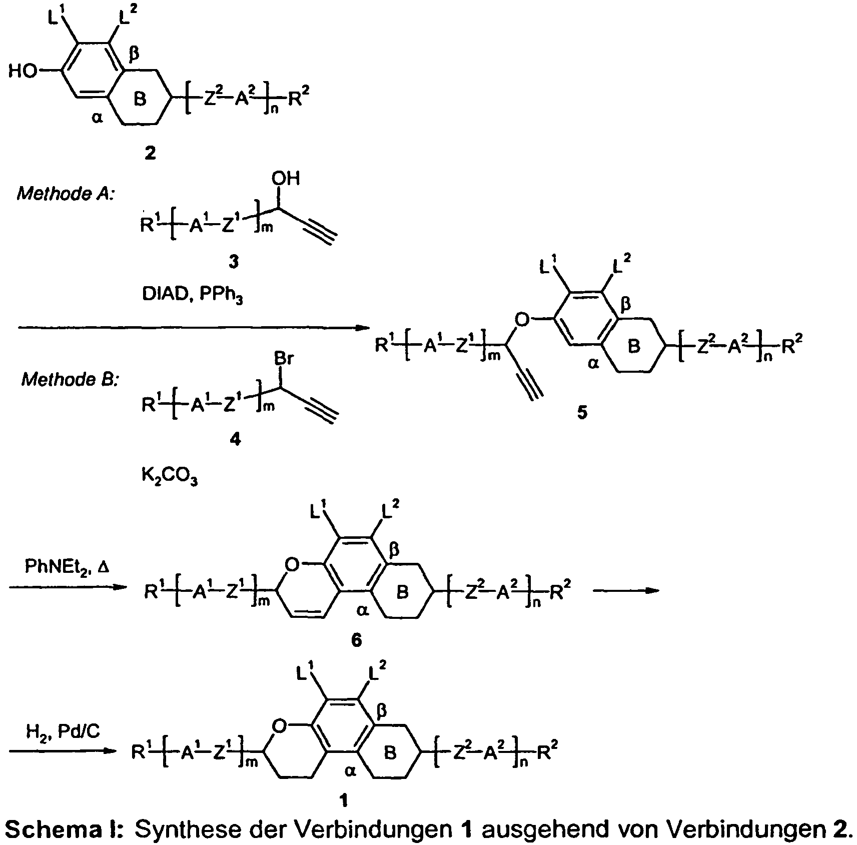

Die Synthese kann durch die Wahl geeigneter Edukte 2 und 3 bzw. 4 an die jeweils gewünschten Verbindungen der Formel 1 angepasst werden. Geeignete Propargylalkohole 3 oder Propargylbromide 4 sind entweder kommerziell erhältlich, oder sie können bereits veröffentlichten Verfahren folgend synthetisiert werden.

Die Synthese der Ausgangsmaterialien 2 wird im Folgenden anhand einiger Beispiele erläutert.

Stellt in den Verbindungen der Formel I der Ring B einen Cyclohexylrest dar und sind L¹ und L² beide gleich F, so beginnt die Synthese der Verbindungen **2** (bzw. hier der Verbindungen **2a**) mit der Verbindung **7**, deren Synthese WO 2004/029015 A1 offenbart (vgl. **Schema II**).

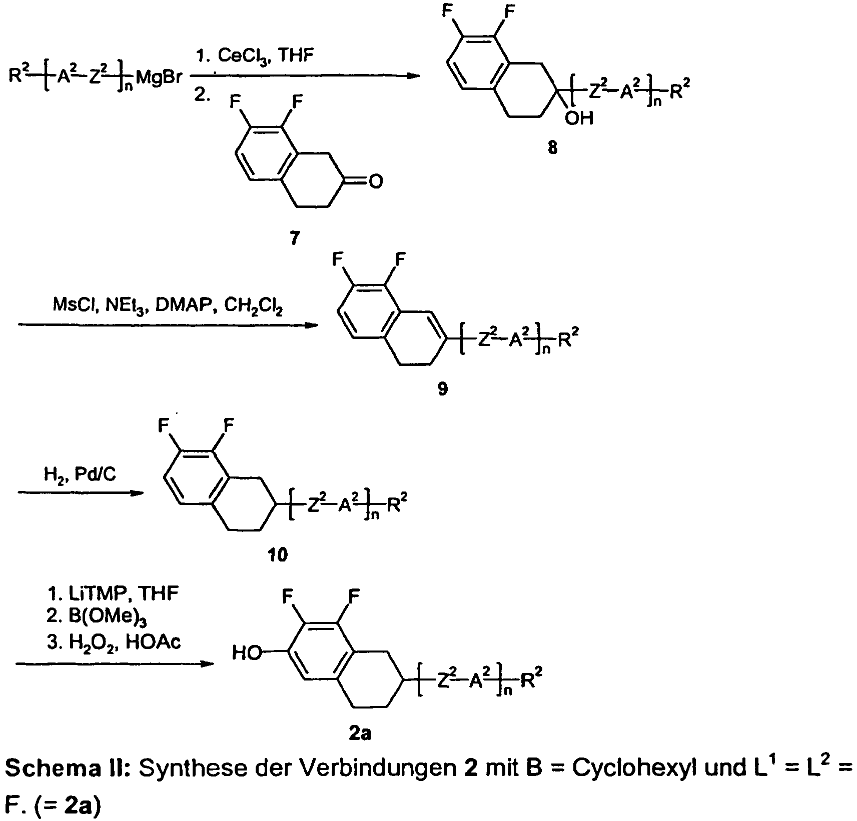

Die Funktionalisierung des β-Tetralons **7** beginnt mit der Cer(III)-chlorid-vermittelten Addition entsprechender *Grignard-* oder lithiumorganischer Verbindungen (vgl. a) N. Takeda, T. Imamoto, Org. Synth. 1999, 76, 228-238. b) M. Scommoda et al. J. Org. Chem. 1996, 61, 4379-4390). Bei der Eliminierung durch Behandlung mit Mesylchlorid und DBU (vgl. oben M. Scommoda et al.) werden die Verbindungen **9** als Hauptkomponente im Isomerengemisch erhalten. Diese werden zu den entsprechenden Tetrahydronaphthalinen **10** hydriert, welche dann über die Reaktionsfolge *ortho*-Metallierung und Hydrolyse und Oxidation des *in situ* gebildeten Boronsäureesters zu den Tetrahydronaphtholen **2a** umgesetzt werden.

Soll in den erfindungsgemäßen Verbindungen der Formel I der Ring B einen Cyclohexenylrest darstellen, und sollen L¹ und L² beide gleich F sein, so wird letztere Reaktionsfolge mit den Zwischenstufen **9** durchgeführt, um zu den entsprechenden Edukten **2** (= **2c** in **Schema VII**) zu gelangen.

Stellt in den erfindungsgemäßen Verbindungen der Formel I der Ring B einen Cyclohexylrest dar und ist einer der Substituenten L¹ und / oder L² gleich H, so erfolgt die Synthese der Verbindungen **2** ausgehend von den Verbindungen **11.** Bevorzugt ist in **11** L¹ oder L² H, der andere Substituent F.

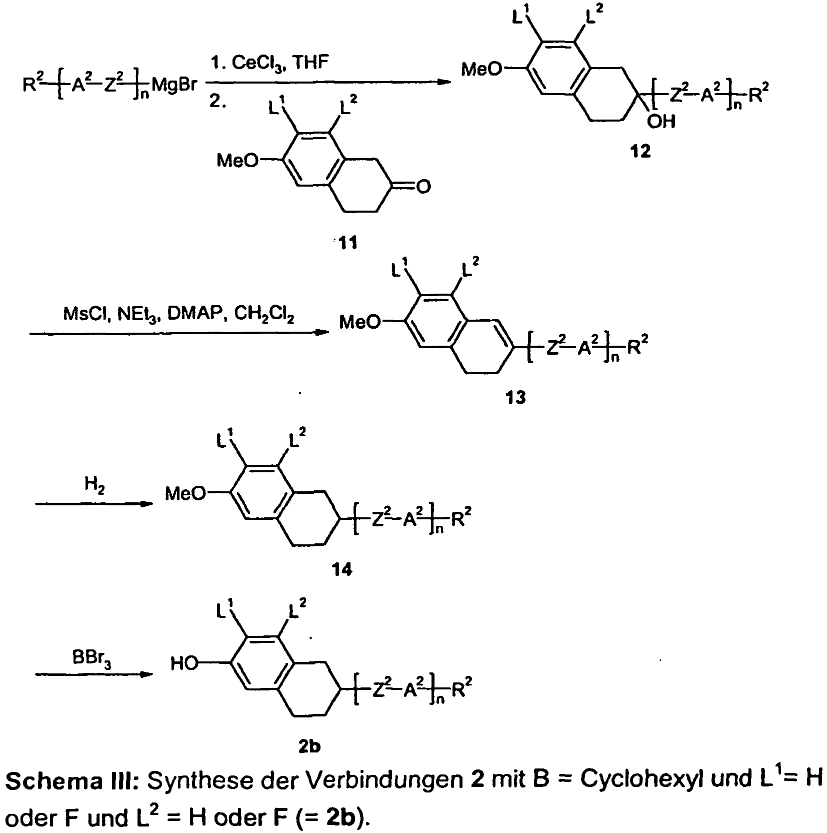

Analog zu **Schema II** werden dazu die Verbindungen **11** (6-Methoxy-naphthalin-2-one) zunächst durch eine Cer(III)-chlorid-vermittelte Addition entsprechender *Grignard-* oder lithiumorganischer Verbindungen (vgl. oben) funktionalisiert. Wiederum werden bei der Eliminierung (vgl. oben) die Verbindungen **13** als Hauptkomponenten in den Isomerengemischen erhalten. Deren Hydrierung ergibt 6-Methoxy-1,2,3,4-tetrahydronaphthaline **14.** Abschließend wird der Methylether mit Bortribromid gespalten (vgl. a) J. F. W. McOmie, D. E. West, Org. Synth., Coll. Vol. V 1973, 412; b) E. H. Vickery, L. F. Pahler, E. J. Eisenbraun, J. Org. Chem. 1979, 44, 4444-4446).

Soll in den Verbindungen **2** der Ring B einen Cyclohexenylrest darstellen, und sollen L¹ und / oder L² gleich H sein, so wird die Methyletherspaltung mit den Zwischenstufen **13** durchgeführt, um zu den entsprechenden Edukten **2** (= **2d** in **Schema X**) zu gelangen.

Für die in **Schema III** dargestellte Synthesefolge werden in den Fällen, in denen nur einer der Substituenten L¹ oder L² gleich F ist und der verbleibende Substituent gleich Wasserstoff ist die Ausgangsmaterialien 7-Fluor-6-methoxy-3,4-dihydro-1*H*-naphthalin-2-on **11a** (= **11** mit L¹ = F, L² = H) und 8-Fluor-6-methoxy-3,4-dihydro-1H-naphthalin-2-on **11b** (= **11** mit L¹ = H, L² = F) benötigt (vgl. Schema **IV**)**.** Diese werden ebenfalls mit dem in der WO 2004/029015 A1 verwendeten Verfahren aus 3-Fluor-4-methoxy-phenylessigsäure **15** bzw. 2-Fluor-4-methoxy-phenylessigsäure **16** hergestellt.

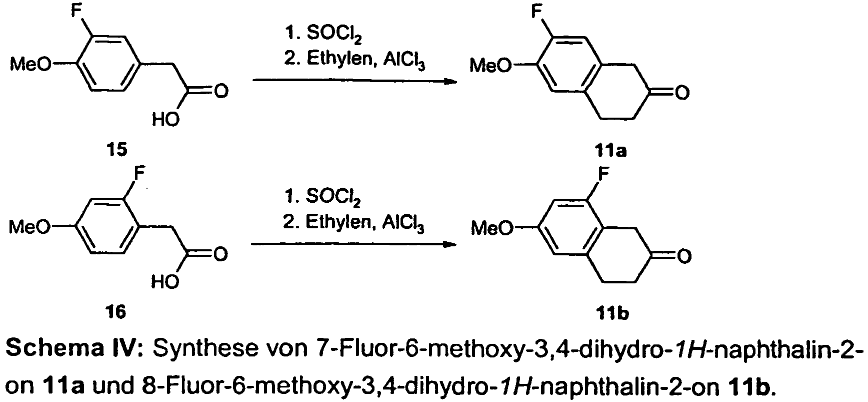

Die Synthese von 3-Fluor-4-methoxy-phenylessigsäure **15** ausgehend von 2-Fluoranisol wurde von *M. Kucha* 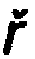 et al. (Collect. Czech. Chem. Commun. 1990, 55, 296-306) beschrieben. 2-Fluor-4-methoxy-phenylessigsäure **16** wird aus 2-Fluor-4-hydroxy-phenylessigsäure [S.-I. Sugita, S. Toda, T. Yoshiyasu, T. Teraji, Mol. Cryst. Liq. Cryst. 1993, 237, 399-406; E. J. Corey, J. P. Dittami, J. Am. Chem. Soc. 1985, 107, 256-257; H. H. Wassermann, J. Wang, J. Org. Chem. 1998, 63, 5581-5586] durch Dimethylierung und Esterverseifung erhalten.

Nicht fluorierte Synthesebausteine **2** (mit L¹ = L² = H) werden ausgehend von 6-Methoxy-3,4-dihydro-1*H*-naphthalin-2-on **(11** mit L¹ = L² = H) erhalten. Eine Synthese von 6-Methoxy-3,4-dihydro-1*H*-naphthalin-2-on (**11** mit L¹ = L² = H) wurde beschreiben [S. N. Suryawanshi, S. N. Fuchs, J. Org. Chem. 1986, 51, 902-921].

Eine weitere, besonders bevorzugte Synthese der Verbindungen **2** geht aus von der Verbindung **17** bzw. den Verbindungen **18.** Die Synthese von **17** erfolgt ausgehend von **7** durch Erzeugung des Enolethers und dessen Reaktion mit Trifluoressigsäureanhydrid. Analog werden die Verbindungen **18** aus den Verbindungen **11** hergestellt (vgl. **Schema V**).

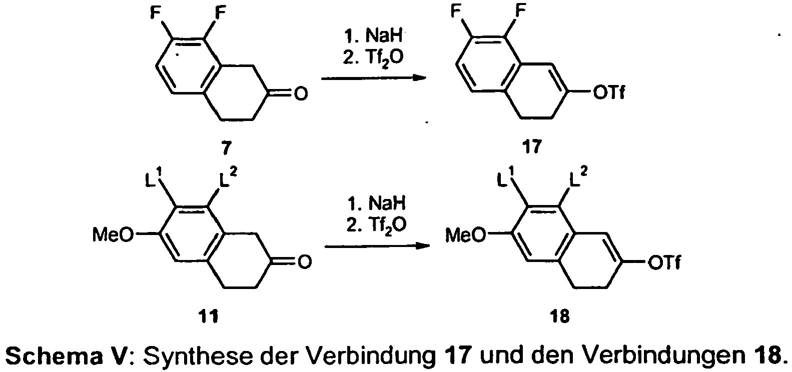

Die Enoltriflate **17** und **18** können in Übergangsmetall-vermittelten Kreuzkupplungsreaktionen [Metal-catalyzed Cross-coupling Reactions (Hrsg.: A. de Meijre, F. Diederich), Wiley-VCH, Weinheim, 2. Aufl. 2004] vielfältig funktionalisiert werden (vgl. **Schema VI** und **Schema IX).** Dabei sind Kreuzkupplungen mit Arylboronsäuren **19** (*Suzuki*-Kupplungen), Alkenylboronsäuren **21** und **23** [C. Sun, R. Bittman, J. Org. Chem. 2006, 71, 2200-2202 und A. Torrado, S. Lopez, R. Alvarez, A. R. de Lera, Synthesis 1995, 285-293], anderen Boronsäuren **25** [T. Oh-e, N. Miyaura, A. Suzuki, J. Org. Chem. 1993, 58, 2201-2208], *Grignard*-Reagentien **26** [B. Scheiper, M. Bonnekessel, H. Krause, A. Fürstner, J. Org. Chem. 2004, 69, 3943-3949] und terminalen Alkinen **27** [Y. Zhao, K. Campbell, R. R. Tykwinski, J. Org. Chem. 2002, 67, 336 - 344.] besonders bevorzugte Methoden, um zu funktionalisierten Dihydronaphthalinderivaten **9** (allg. Formel) bzw. **13** (allg. Formel) zu gelangen. In **Schema VI** sind einige Beispiele für Kreuzkupplungsreaktionen des Enoltriflats **17** dargestellt.

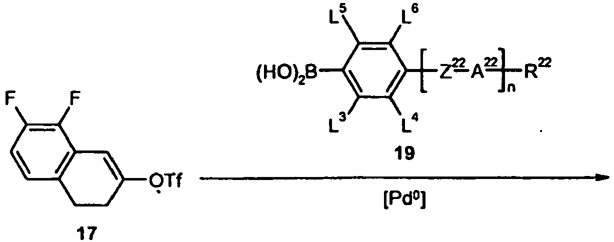

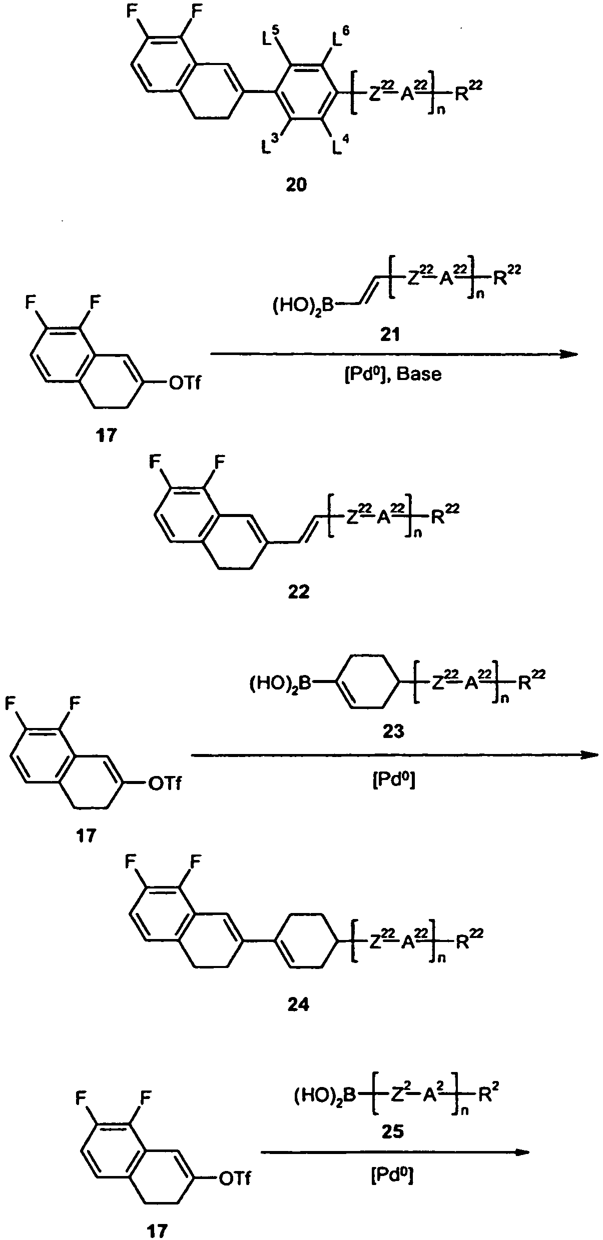

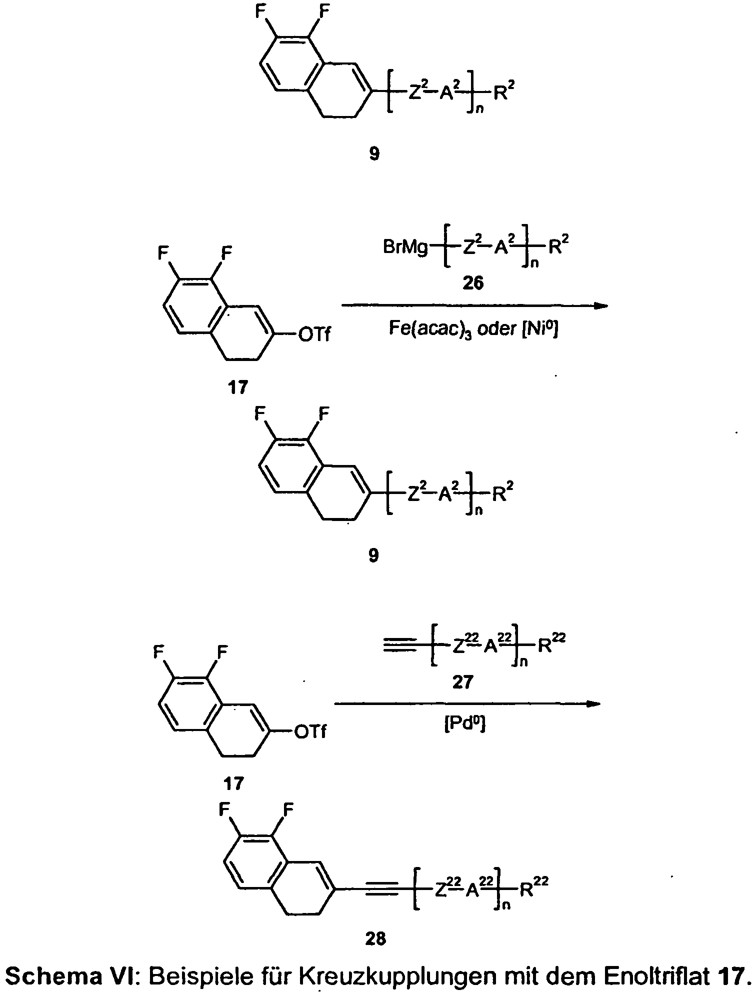

Die Produkte **9** (allg. Formel, aus **Schema III**) aus dieser Funktionalisierung werden wie in **Schema VII** und **Schema VIII** nochmals dargestellt (vgl. auch **Schema II**) zu den gewünschten Verbindungen **2** umgesetzt.

Soll in den erfindungsgemäßen Verbindungen **1** B ein Cyclohexenylrest sein, so werden die Verbindungen **9** über die Reaktionsfolge *ortho-*Metallierung und Hydrolyse und Oxidation des *in situ* gebildeten Boronsäureesters zu den Dihydronaphtholen **2c** umgesetzt (vgl. **Schema VII**)**.**

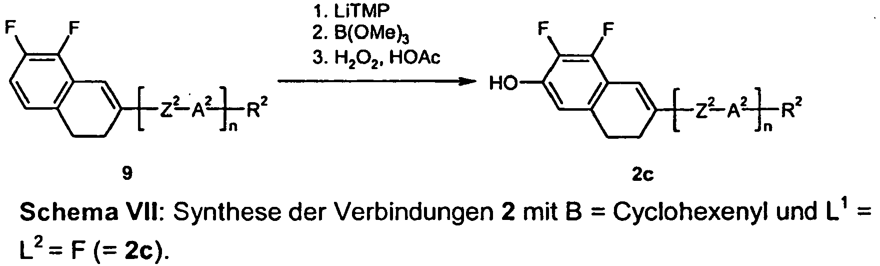

Soll in den erfindungsgemäßen Verbindungen **1** B einen Cyclohexylrest sein, so werden die Verbindungen **9** zunächst zu den Verbindungen **10** hydriert und anschließend wie oben zu den entsprechenden Tetrahydronaphtholen **2a** umgesetzt (vgl. **Schema VIII**)**.**

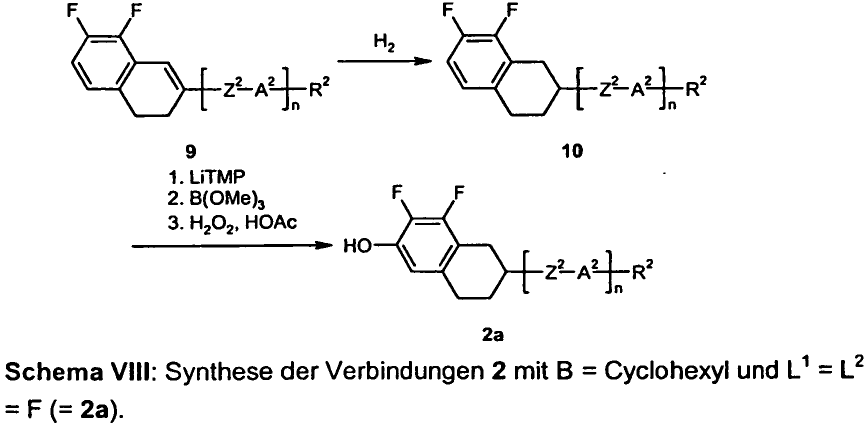

In **Schema IX** sind einige Beispiele für Kreuzkupplungsreaktionen der Enoltriflate **18** dargestellt.

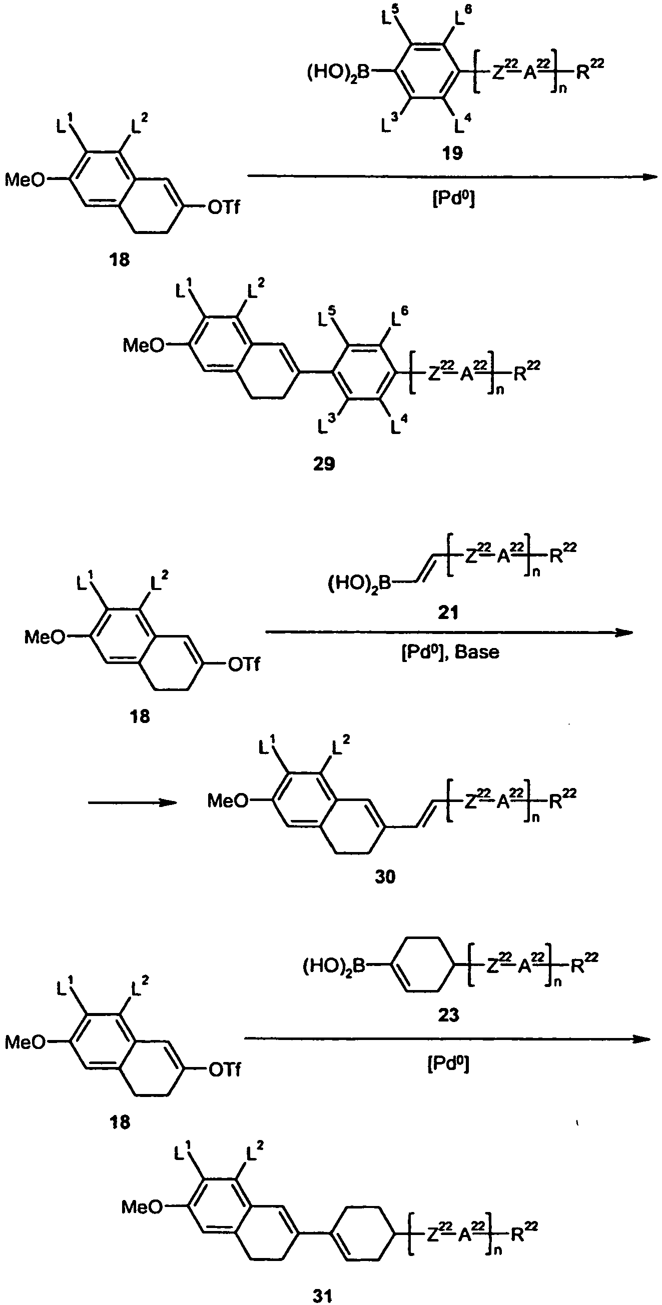

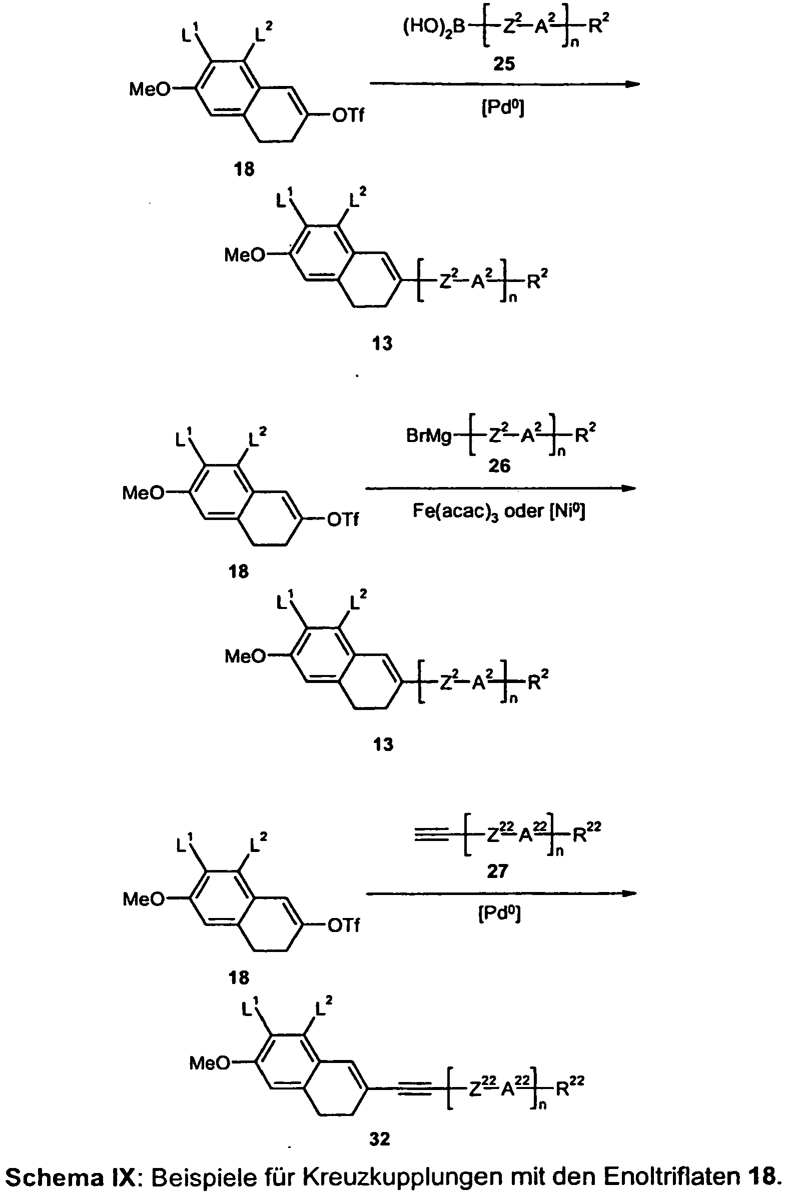

Die Produkte **13** (allg. Formel) aus dieser Funktionalisierung werden wie in **Schema X** und **Schema XI** nochmals dargestellt (vgl. auch **Schema III**) zu den gewünschten Verbindungen **2** umgesetzt.

Soll in den Verbindungen **2** der Ring **B einen** Cyclohexenylrest darstellen, und sollen L¹ und / oder L² gleich H sein, so wird eine Methyletherspaltung mit den Zwischenstufen **13** durchgeführt, um zu den entsprechenden Edukten **2d** zu gelangen (vgl. **Schema X**)**.**

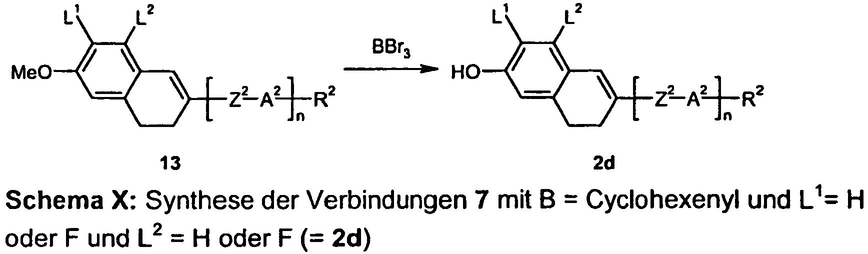

Soll in den Verbindungen 2 der Ring B einen Cyclohexylrest darstellen, und sollen L¹ und / oder L² gleich H oder F sein, so wird vor der Methyletherspaltung eine Hydrierung zu den Verbindungen **14** durchgeführt (vgl. **Schema XI**)**.**

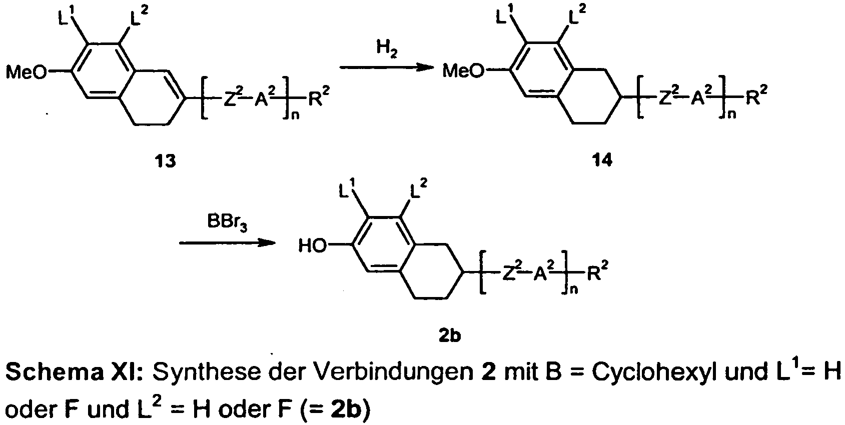

Stellt in den Verbindungen der Formel I der Ring B einen Phenylrest (L³ = H) oder einen fluorierten Phenylrest (L³ = F) dar (vgl. **Schema XII**) und sind L¹ und L² beide gleich F, so geht die Synthese aus von den Verbindungen **33** (L³ = H oder F), deren Synthese die Druckschrift WO 2004/029015 A1 offenbart.

Diese Zwischenstufen **33** bzw. die daraus hervorgehenden Triflate **34** (vgl. **Schema XII**) können vielfältig funktionalisiert werden (vgl. **Schemata XIII, XIV** und **XV**)**.**

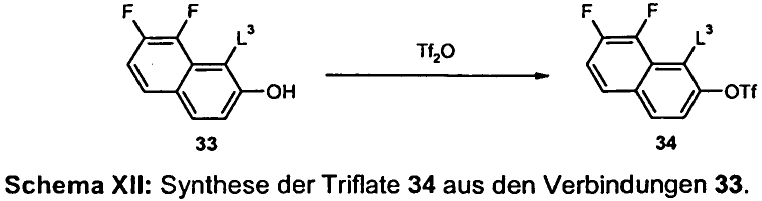

Für den Fall, dass R²-(A²-Z²)ₙ- einen Alkoxyrest darstellen soll, erfolgt eine einfache Veretherung von **33** mit Alkyliodiden oder-bromiden (vgl. **Schema XIII**).

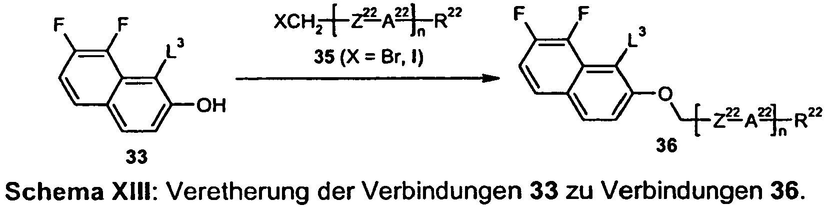

Eine weitere geeignete Methode, um ausgehend von (2,3-Difluorphenyl)-acetylchlorid zu Verbindungen **33** mit L³ = H zu gelangen ist in der Literatur [H. Juteau, Y. Gareau, H. Lachance, Tetrahedron Lett. 2005, 46, 4547-4549] beschrieben. Auf diese Weise wird (2,3-Difluorphenyl)-acetylchlorid z.B mit Trimethysilylacetylen in Gegenwart von Aluminium(III)-chlorid zum entsprechenden 2-Trimethylsilylnaphthol umgesetzt. Abspaltung der Silylgruppe (z.B. mit Kaliumfluorid in DMF) liefert dann die Verbindungen **33.**

Für den Fall, dass R²-(A²-Z²)ₙ- einen Alkenylrest darstellen soll, werden die Triflate **34** mit entsprechenden Alkenylboronsäuren in einer Palladiumkatalysierten Kreuzkupplung umgesetzt (vgl. a) C. Sun, R. Bittmann, J. Org. Chem. 2006, 71, 2200-2202; b) A. Torrado, S. Lopez, R. Alvarez, A. R. de Lera, Synthesis 1995, 285-293). Diese Alkenyle **37** können dann durch Hydrierung zu den entsprechenden gesättigten Verbindungen **38** umgesetzt werden (vgl. **Schema XIV**)**.**

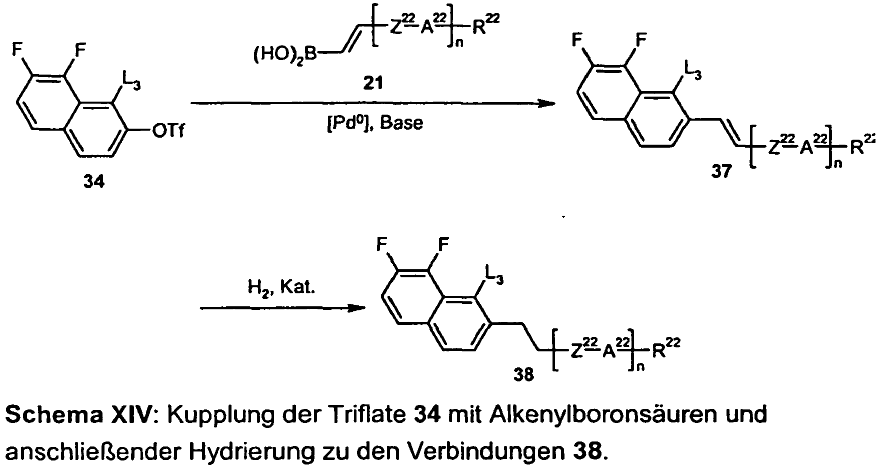

Weiterhin sind Übergangsmetall-vermittelte Kreuzkupplungen der Triflate **34,** z.B. mit Arylboronsäuren (*Suzuki*-Kupplung), terminalen Alkinen (*Sonogashira*-Kupplung) und *Grignard*-Reagentien (*Kumada*-Kupplung oder Eisen(III)-vermittelte Kreuzkupplung) (vgl. **Schema XV,** hier ist nur die Funktionalisierung über eine Kupplung mit *Grignard*-Reagentien dargestellt, für weitere Funktionalisierungsmöglichkeiten vgl. **Schema VI** bzw. **Schema IX**) besonders bevorzugte Methoden um zu funktionalisierten Naphthalinderivaten **39** zu gelangen.

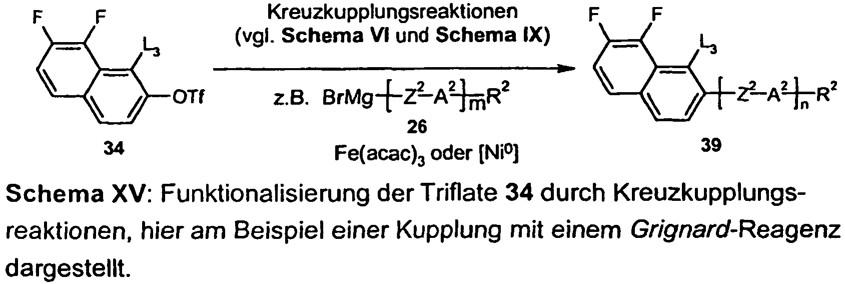

Diese funktionalisierten Naphthalinderivate **39** (allg. Formel) werden dann wieder über die Reaktionsfolge *ortho*-Metallierung, Generierung eines Boronsäureesters sowie Hydrolyse und Oxidation desselben zu den Naphtholen **2** (= **2e**) umgesetzt (vgl. **Schema XVI**)**.** Mit diesen Naphtholen **2** wird dann wie in **Schema I** dargestellt weiterverfahren.

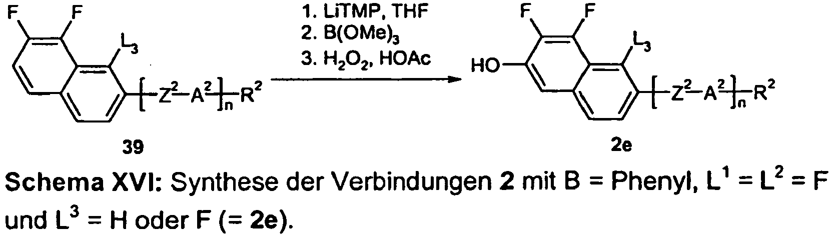

Stellt in den Verbindungen der Formel I der Ring B einen nicht fluorierten Phenylrest (L³ = H) dar und ist L¹ und / oder L² gleich H, wobei der Substituent L¹ oder L² der ungleich H ist bevorzugt F sein soll, so erfolgt die Synthese der Verbindungen **2** (hier **2f**) ausgehend von den Verbindungen **13** (vgl. **Schema XVII**)**.** Die Synthese der Verbindungen **13** wurde schon oben vorgestellt.

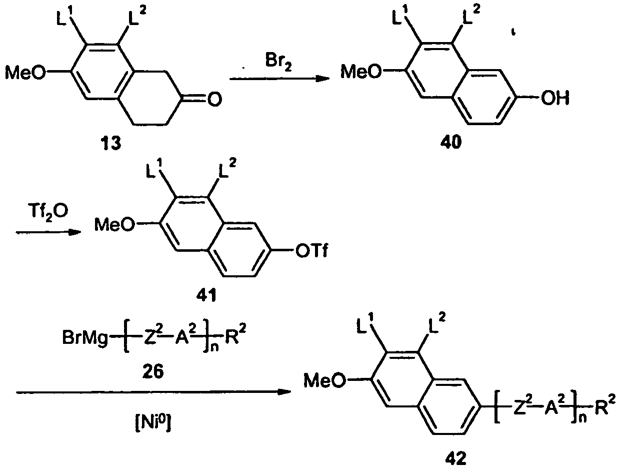

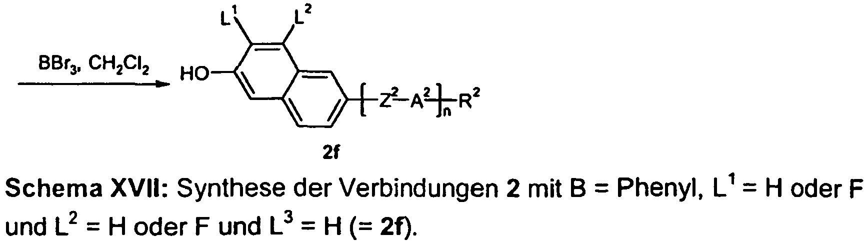

Die Tetralone **13** werden durch Behandlung mit Brom in die entsprechenden Naphthole **40** umgewandelt (vgl. WO 2004/029015 A1). Die aus **40** hervorgehenden Triflate **41** können dann wie zuvor für **34** oben beschrieben (vgl. **Schema XIV** und **Schema XV**) in vielfältiger Weise funktionalisiert werden. In **Schema XVII** ist nur die Funktionalisierung von **41** über eine *Kumada*-Kupplung dargestellt. Weitere Reaktionen der Triflate **41** sind wie zuvor beschrieben möglich (vgl. **Schema VI** und **Schema IX**). Nach Spaltung der Methylether **42,** stehen die für weitere Umsetzungen (vgl. **Schema I**) benötigten Edukte **2f** zur Verfügung.

Für den Fall, dass R²-(A²-Z²)ₙ- in **2f** einen Alkoxyrest darstellen soll, muss die Synthese modifiziert werden. Zum einen können im letzen Schritt der Synthese der Verbindungen **7f** (vgl. **Schema XVII**) Methoden verwendet werden, die die Spaltung von Arylmethylethem in Gegenwart anderer Alkylarylether (A. K. Chakrabortiet al. J. Org. Chem. 2002, 67, 6406-6414; G. Majetichet al. Tetrahedron Lett. 1994, 35, 8727-8730; E. Duran et al. Heterocycles 2002, 57, 825-856) oder Arylallylether (vgl. M. T. Konieczny, G. Maciejewski, W. Konieczny, Synthesis 2005, 1575-1577) zulassen. Ein alternatives bevorzugtes Verfahren ist in **Schema XVIII** dargestellt. Hier erfolgt die Synthese der Zwischenprodukte **2g** ausgehend von Bromtetralonen **43.** Diese werden wiederum durch Behandlung mit Brom [WO 2004/029015 A1] in die entsprechenden 6-Bromnaphthalin-2-ole **44** überführt, welche dann zu den gewünschten Ethern **46** umgesetzt werden können. Die weitere Umsetzung zu den gewünschten Naphtholen **2g** wird dann durch eine Metallierung mit Mg, *Grignard-* oder lithiumorganischen-Verbindungen eingeleitet.

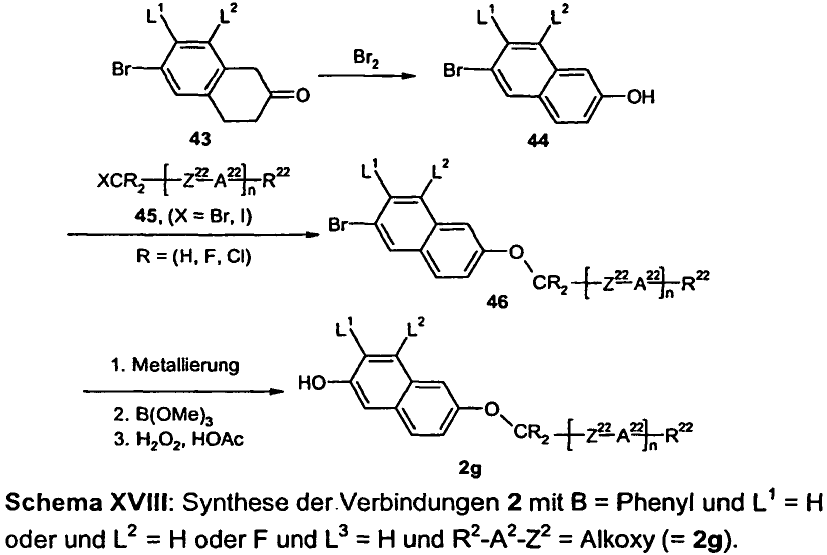

Die Synthese der Bromtetralone **43** erfolgt in Anlehnung an Literaturvorschriften ausgehend von substituierten 4-Bromtoluolen **47** (vgl. **Schema XIX,** WO 2004/029015 A1; M. T. Konieczny et al. Synthesis 2005, 1575-1577; B. P. Imbimbo et al., J. Med. Chem. 2005, 48, 5705-5720). 4-Brom-3-fluortoluol **(47,** L¹ = F, L² = H) und 4-Brom-2-fluortoluol **(47,** L¹ = H, L² = F) sind kommerziell erhältlich. Die Methylgruppe der Bromtoluole **47** wird zunächst mit NBS bromiert und die Brommethylenverbindungen **48** werden mit Kaliumcyanid in die entsprechenden Nitrile überführt. Diese werden dann zu den Phenylessigsäuren **49** hydrolysiert, die dann zu den gewünschten Bromtetralonen **43** umgesetzt werden.

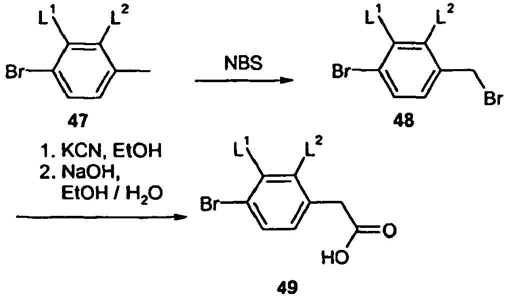

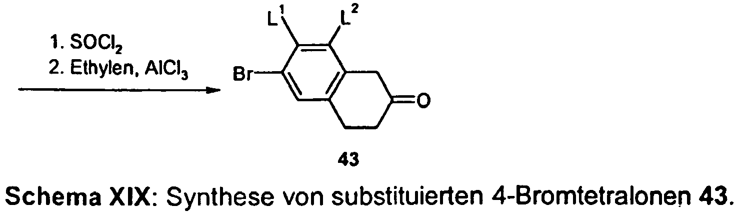

Eine Synthese des nicht fluorierten Synthesebausteins **43** (L¹ = L² = H) wird von D. M. Tschaen et al. J. Org. Chem. 1995, 60, 4324-4330 beschrieben.

Stellt in den Verbindungen der Formel I der Ring B einen fluorierten Phenylrest (L³ = F) dar und sind L¹ und / oder L² gleich H, wobei der Substituent L¹ oder L² der ungleich H ist bevorzugt F sein soll, so erfolgt die Synthese der Verbindungen **2** (speziell **2h** und **2i**) ebenfalls vorteilhaft ausgehend von den Bromnaphtholen **44** (vgl. **Schema XX** und **Schema XXI**).

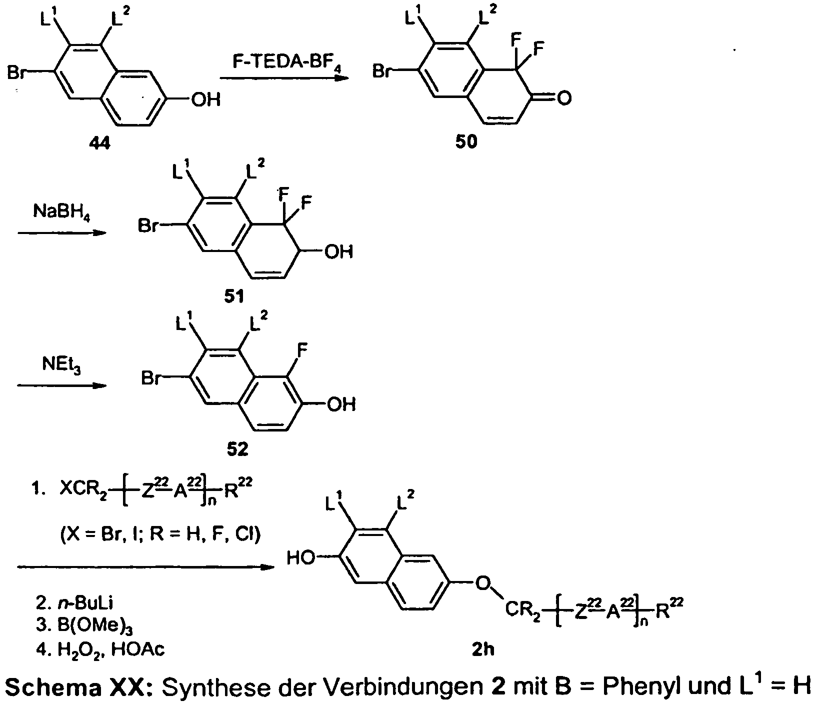

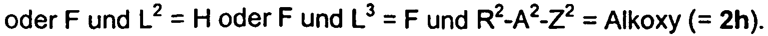

Die Naphthole **44** werden zunächst durch Fluorierung mit Selectfluor^{®} (F-TEDA-BF₄) zu den Difluor-1 H-naphthalin-2-onen **50** umgesetzt (vgl. WO 2004/029015 A1), welche dann zu 1 H-Naphthalin-2-olen **51** reduziert werden. Diese rearomatisieren bei Behandlung mit Base zu den Verbindungen **52** unter Abspaltung von Fluorwasserstoff. Die Verbindungen **52** sind dann zentrale Zwischenstufen auch für die Synthese von Verbindungen in denen R²-A²-Z² einen Alkoxyrest darstellen soll. (**Schema XX,** letzte Reaktion) Dazu wird zunächst mit geeigneten Alkylhalogeniden verethert und anschließend die Umsetzung zum Naphthol **2h** durchgeführt.
Ausgehend von **52** kann eine Funktionalisierung über das Triflat **53** erfolgen. In der dargestellten Kreuzkupplung (vgl. **Schema XXI**) mit verschiedenen *Grignard*-Reagentien gelingt eine effektive Diskriminierung zwischen der Triflat- und der Bromfunktion [T. Kamikawa, T. Hayashi, Tetrahedron Lett. 1997, 38, 7087-7090]. Nach Umsetzung von **54** zum Naphthol **2i**, stehen die für die weitere Synthese (vgl. **Schema I**) benötigten Edukte **2** (speziell **2i**) zur Verfügung. 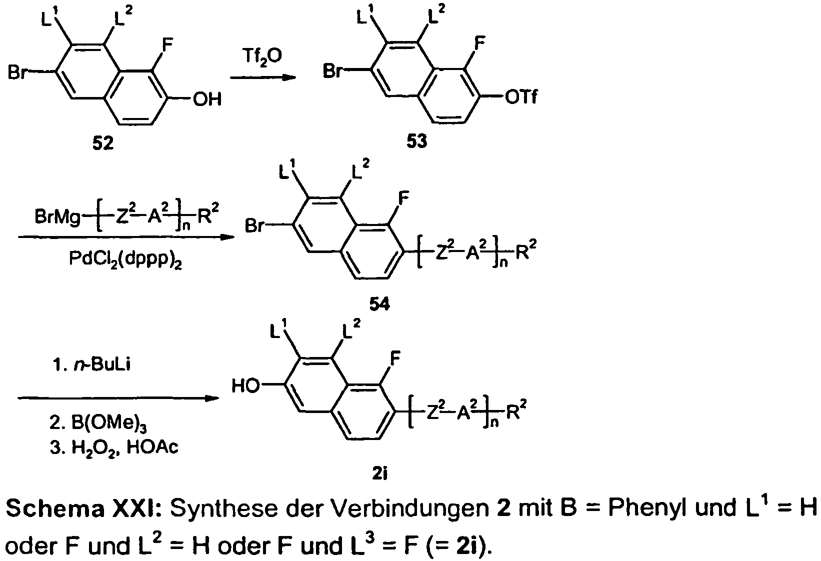 1,2,3,8,9,10-hexahydro-pyrano[3,2-f]chromene, kurz Pyranochromane, sind Verbindungen der Formel I in denen der Ring B einen Sauerstoff-Heterocyclus darstellt. Auch diese Verbindungen werden gemäß **Schema I** aus den entsprechenden Verbindungen **2** hergestellt.

Stellt in den Verbindungen der Formel I der Ring B einen Sauerstoff-Heterocyclus dar und sind L¹ und L² beide gleich F, so werden 7,8-Difluorchroman-6-ole **2j** als Ausgangsmaterialien benötigt. Diese werden ausgehend von 2,3-Difluorphenol (**51**) oder 3,4-Difluor-2-hydroxybenzaldehyd (**55**) [a) N. J. Lawrence, L. A. Hepworth, D. Rennison, A. T. McGown, J. A. Hadfield, J. Fluorine Chem. 2003, 123, 101-108. b) E. Marzi, J. Gorecka, M. Schlosser, Synthesis 2004, 1609-1618] synthetisiert (vgl. Schema **XXII**)**.**

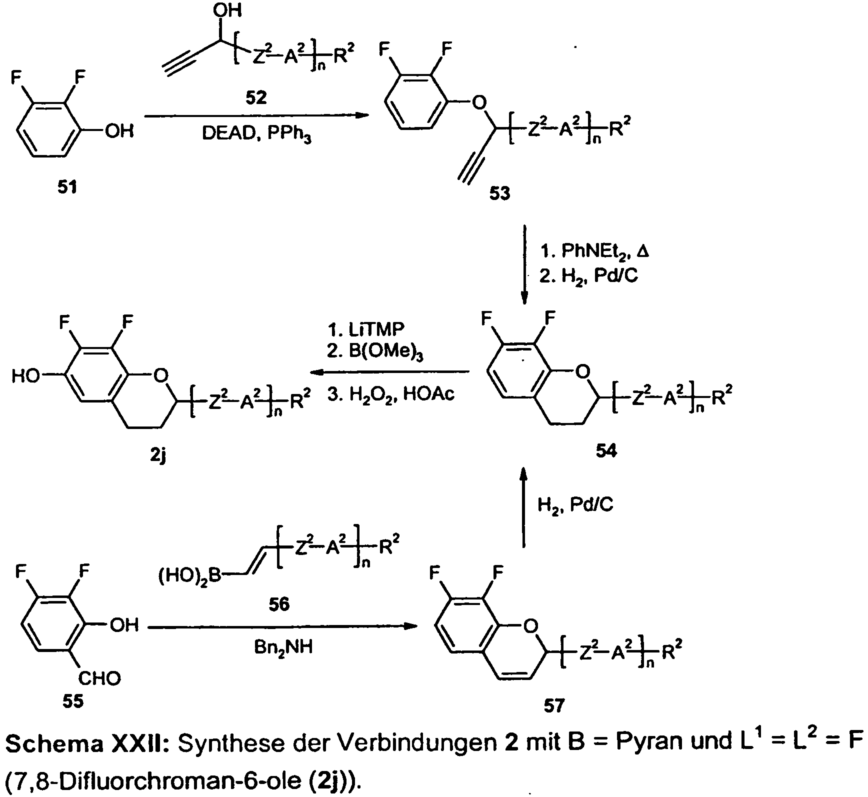

Dazu wird aus 2,3-Difluorphenol (**51**) und einem Propargylalkohol **52** durch *Mitsonobu*-Veretherung zunächst ein Propargyl-Arylether **53** gebildet, der unter geeigneten Reaktionsbedingungen eine thermische [3.3]-sigmatrope Umlagerung zu einem 2H-Chromen eingeht. Diese Chromene können unter schonenden Bedingungen leicht zu den entsprechenden Chromanen **54** hydriert werden.

Alternativ werden diese 7,8-Difluorchromane **2j** aus 3,4-Difluor-2-hydroxybenzaldehyd (**55**) [a) N.J. Lawrence, L.A. Hepworth, D. Rennison, A.T. McGown, J.A. Hadfield, J. Fluorine Chem. 2003, 123, 101-108; b) E. Marzi, J. Gorecka, M. Schlosser, Synthesis 2004, 1609-1618] über eine von Petasis et al. beschriebene [a) N. A. Petasis, I. Akritopoulou, Tetrahedron Lett. 1993, 34, 583-586; b) N.A. Petasis, I.A. Zavialov, J. Am. Chem. Soc. 1997, 119, 445-446; c) N.A. Petasis, I.A. Zavialov, J. Am. Chem. Soc. 1998, 120, 11798-11799] und von Wang und Finn [Q. Wang, M.G. Finn, Org. Lett. 2000, 2, 4063-4065] weiterentwickelte Reaktion erhalten. Dabei werden aus Salicylaldehyden und Vinylboronsäuren in Gegenwart von Dibenzylamin 2*H*-Chromene wie **57** mit hoher Ausbeute erhalten. Diese können dann wiederum leicht zu den entsprechenden Chromanen **54** hydriert werden (s.o.). Die so erhaltenen Zwischenprodukte **54** werden durch *ortho*-Metallierung und Hydrolyse und Oxidation des *in situ* gebildeten Boronsäureesters zu den 7,8-Difluorchroman-6-olen **2j** funktionalisiert.

Stellt in den Verbindungen der Formel **I** der Ring B einen Pyranring dar und ist L¹ gleich F und L² gleich H, so erfolgt die Synthese der Verbindungen **2** (speziell 7-Fluor-chroman-6-ole **2k**) ausgehend von 5-Brom-4-fluor-2-hydroxy-benzaldehyd (**58**)**.** Dieses Edukt **58** ist über literaturbekannte Verfahren aus 3-Fluorphenol durch *ortho*-selektive Formylierung [J. B. Blair et al., J. Med. Chem. 2000, 43, 4701-4710] und anschließende Bromierung [W.A. Caroll et al., J. Med. Chem. 2004, 47, 3163-3179] zugänglich.

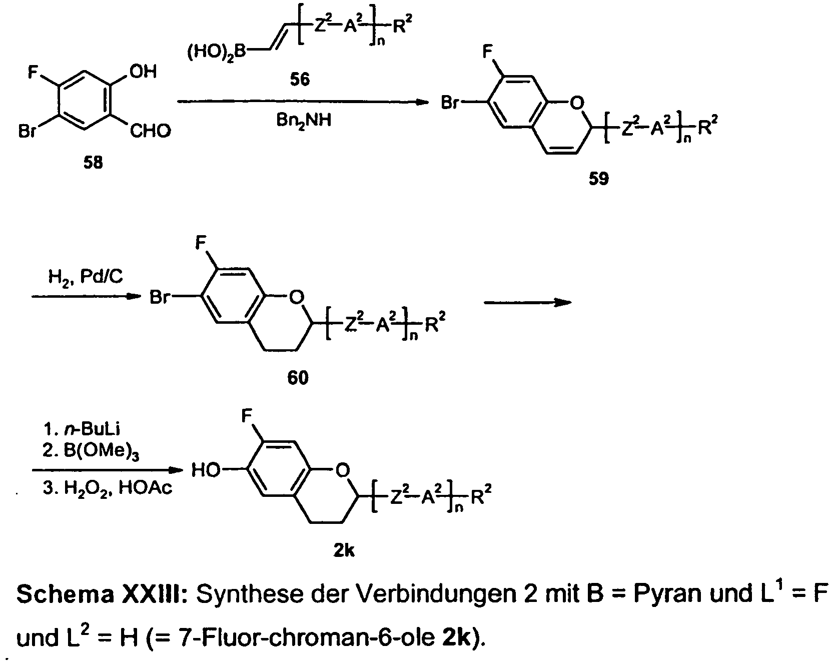

Ausgehend von 5-Brom-4-fluor-2-hydroxy-benzaldehyd (**58**) erfolgt die Synthese der Chromane **60** wiederum vorteilhaft durch die schon beschriebene Reaktion mit Vinylboronsäuren [Q. Wang, M.G. Finn, Org. Lett. 2000, 2, 4063-4065] und anschließender Hydrierung. Die Funktionalisierung zum Chromanol **2k** gelingt wie oben beschrieben, diesmal nach Halogen-Metallaustausch.

Stellt in den Verbindungen der Formel I der Ring B einen Pyranring dar und ist L¹ gleich H und L² gleich F, so erfolgt die Synthese der Verbindungen **2** (speziell 8-Fluor-chroman-6-ole **21**) ausgehend von 2-Fluor-4-bromphenol (**61**)**.**

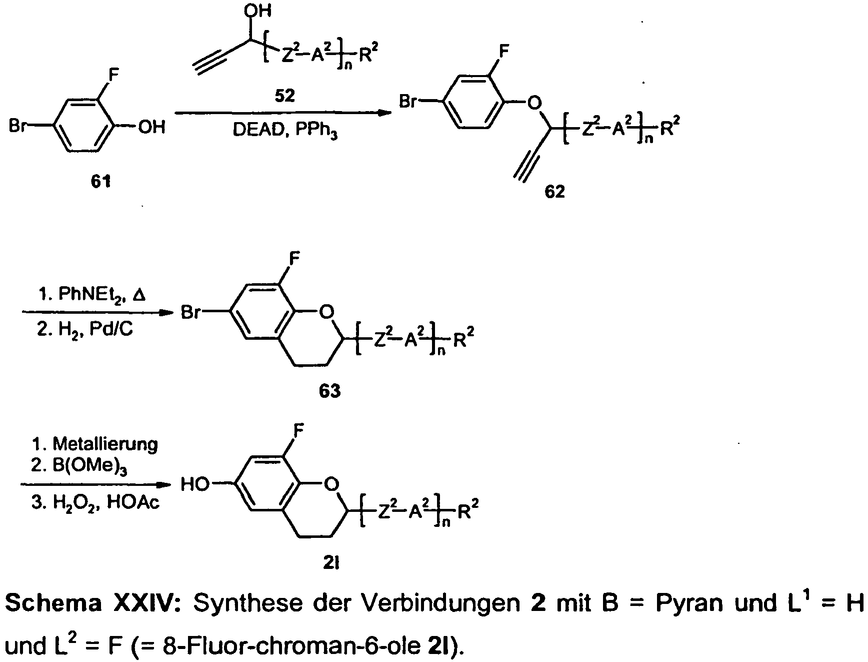

Hier wird der O-Heterocyclus bevorzugt durch eine *Claisen*-Umlagerung über den Propargylarylether **62** anelliert (vgl. **Schema XXIV**). Die Funktionalisierung zum Chromanol **21** gelingt mit der gleichen Reaktionsfolge wie für das Regioisomer **2k.**

Alternativ können die Zwischenprodukte **21** auch ausgehend von 2-Fluor-4-bromphenol (**61**) über den Salicylaldehyd **64** synthetisiert werden (vgl. **Schema XXV**)**.** Dieser ist über eine *Duff*- Reaktion [M. L. Micklatcher, M. Cushman, Synthesis 1999, 1878-1880] aus **61** zugänglich. Die anschließende Synthese des Chromans **63** kann dann über die von *Wang und Finn* [Q. Wang, M. G. Finn, Org. Lett. 2000, 2, 4063-4065] beschriebene Prozedur und anschließender Hydrierung erfolgen.

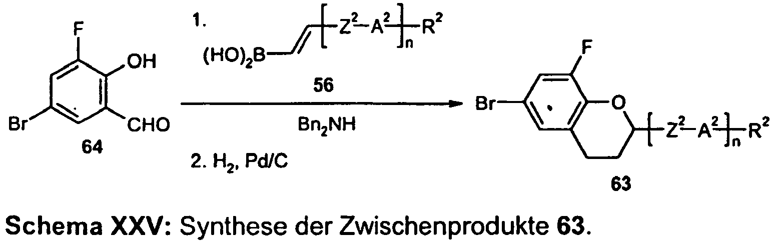

Sollen in den Verbindungen I die Reste R¹[A¹Z¹]ₘ- und / oder R²[A²Z²]ₙ-ungesättigte Reste, Brücken oder Ringsysteme enthalten, so muss die in **Schema I** dargestellte Synthese modifiziert werden (vgl. **Schema XVII).**

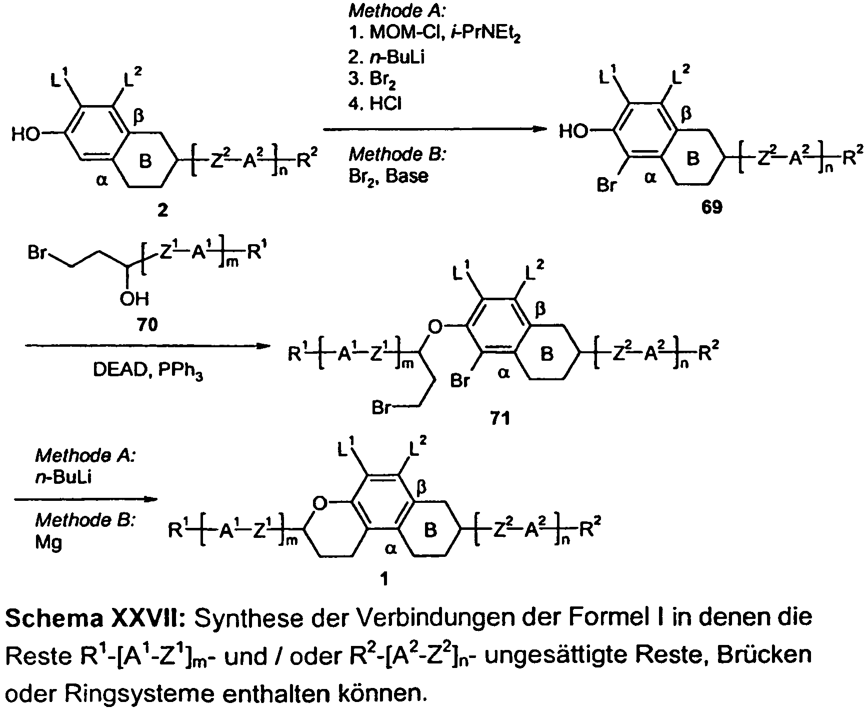

In Anlehnung an Literaturvorschriften [a) C. K. Bradsher, D. C. Reames, J. Org. Chem. 1981, 46, 1384-1388 und b) L. A. Paquette et al., J. Org. Chem. 1994, 59, 2043-2051] erfolgt der Aufbau des gesättigten O-Heterocyclus dann ausgehend von den Verbindungen der Formel **71**. Dazu werden die Verbindungen **2** zunächst in geeigneter Weise bromiert. In einem bevorzugten Verfahren (vgl. **Schema XXVII,** *Methode A*) werden zuerst die MOM-Ether der Substanzen **2** hergestellt. Diese werden mit n-Butyllithium selektiv in der *ortho*-Position metalliert, und die lithiumorganischen Verbindungen werden mit Brom abgefangen. Abschließend erfolgt die Spaltung der MOM-Gruppe unter Erhalt der Verbindungen **69**. Die *ortho*-Metallierung erfolgt analog zu den Methoden in a) F. Mongin, M. Schlosser, Tetrahedron Lett. 1996, 37, 6551-6554; b) R. C. Ronald, M. R. Winkle, Tetrahedron 1983, 39, 2031-2042; c) M. R. Winkle, R. C. Ronald, J. Org. Chem. 1982, 47, 2101-2108; d) M. Dabowski et al. Tetrahedron 2005, 61, 6590-6595 oder e) P. L. Coe et al. J. Chem. Soc. Perkin Trans. 1 1995, 2729-2737.

Die Halogenierung kann auch direkt durch Behandlung mit Brom erfolgen (vgl. **Schema XXVII**, Methode B) (vgl. a) C. W. Holzapfel, D. B. G. Williams, Tetrahedron 1995, 51, 8555-8564; b) K. J. Edgar, S. N. Falling, J. Org. Chem. 1990, 55, 5287-5291; c) R. Johnsson, A. Meijer, U. Ellervik, Tetrahedron 2005, 69, 11567-11663).

Veretherung mit Brompropanolderivaten **70** liefert die Verbindungen **71**. Die aus diesen Verbindungen erzeugte lithiumorganische Verbindung (*Methode A*) oder das aus diesen Verbindungen erzeugte *Grignard-*Reagenz *(Methode B)* cyclisiert unter den Reaktionsbedingungen spontan unter Bildung der Verbindungen **1**.

Geeignete Ausgangsmaterialien **2** in denen der Rest R²[A²Z²]ₙ-ungesättigte Reste, Brücken oder Ringsysteme enthält, sind zum Großteil wie oben beschrieben zugänglich. Lediglich die Synthesen für Verbindungen **2** in denen der Ring B einen Cyclohexylrest darstellt und für Verbindungen in denen der Ring B einen Pyranring darstellt müssen modifiziert werden.

Für die Verbindungen **2** in denen B einen Cyclohexylrest (speziell **2a, 2c**) darstellt kann die Herstellung der Zwischenprodukte **10** und **14** nicht mehr ausgehend von **8** bzw. **12** über eine Eliminierung mit anschließender Hydrierung erfolgen (vgl. **Schema II** und **Schema III). 10** und **14** werden daher direkt über eine ionische Reduktion aus **8** bzw. **12** hergestellt (vgl. **Schema XXVIII**).

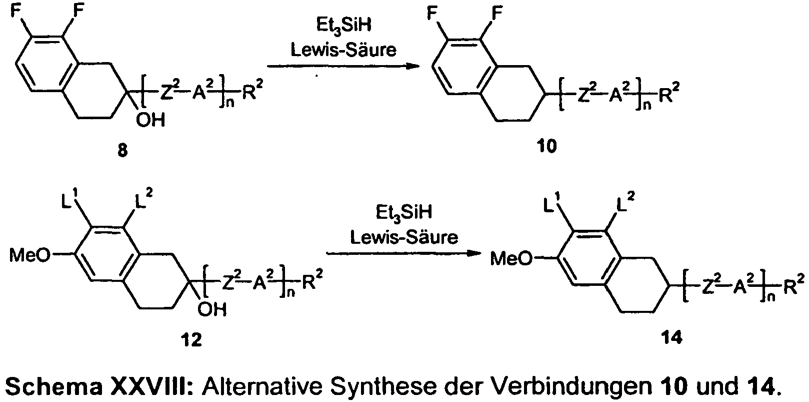

Sollen Verbindungen **2** synthetisiert werden in denen R²[A²Z²]ₙ ungesättigte Reste, Brücken oder Ringsysteme enthält und B einen Pyranring darstellen soll, so wird folgendes Verfahren benötigt (vgl. **Schema XXIX**)

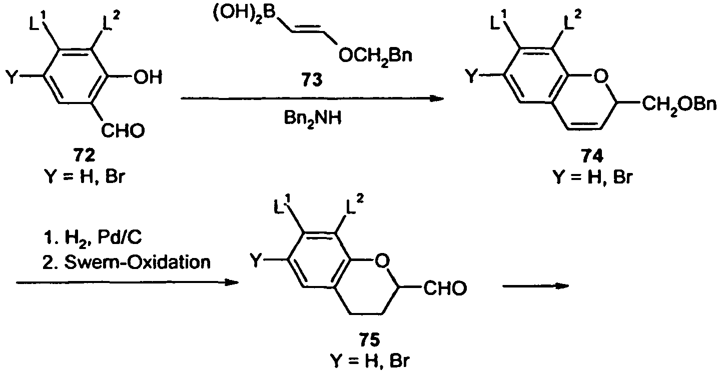

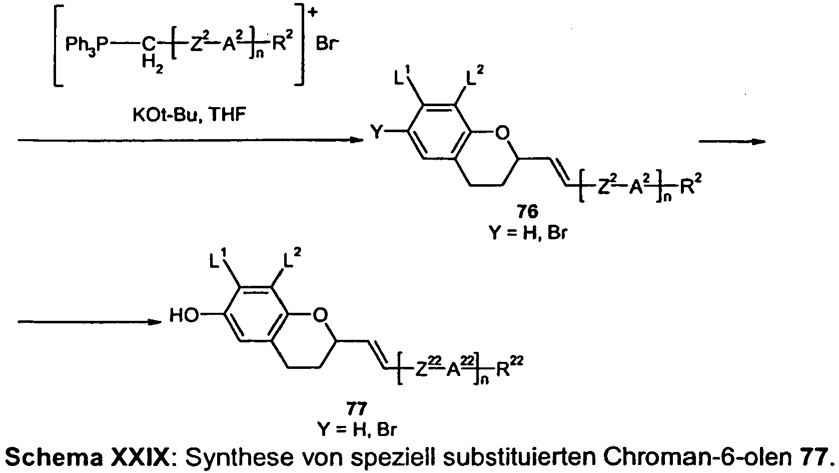

Ausgehend von Salicylaldehyden **72** (Y = H, Br) erfolgt der Aufbau des Chromens **74** mit der Boronsäure **73** [R. A. Batey, A. N. Avinash, A. J. Lough, J. Am. Chem. Soc. 1999, 121, 450-451]. Nach Hydrierung und Oxidation wird die Zwischenstufe **75** erhalten. Hiervon ausgehend kann dann die Gruppe R²[A²Z²]ₙ- aufgebaut werden (z.B. durch *Wittig-*Olefinierung).
Methoden zur Funktionalisierung zu den Chroman-6-olen **77** wurden ausreichend beschrieben.

Für die Synthese von Verbindungen **I** in denen **B** einen fluorierten Cyclohexanring (hier speziell = **1c**) oder einen fluorierten Cyclohexenring (hier speziell = **1d**) darstellt ist folgende Reaktionsfolge ausgehend von den Verbindungen **I** geeignet, in denen B ein Cyclohexenring ist (hier speziell = **1b**) (vgl. **Schema XXX**).

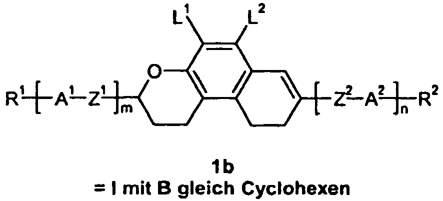

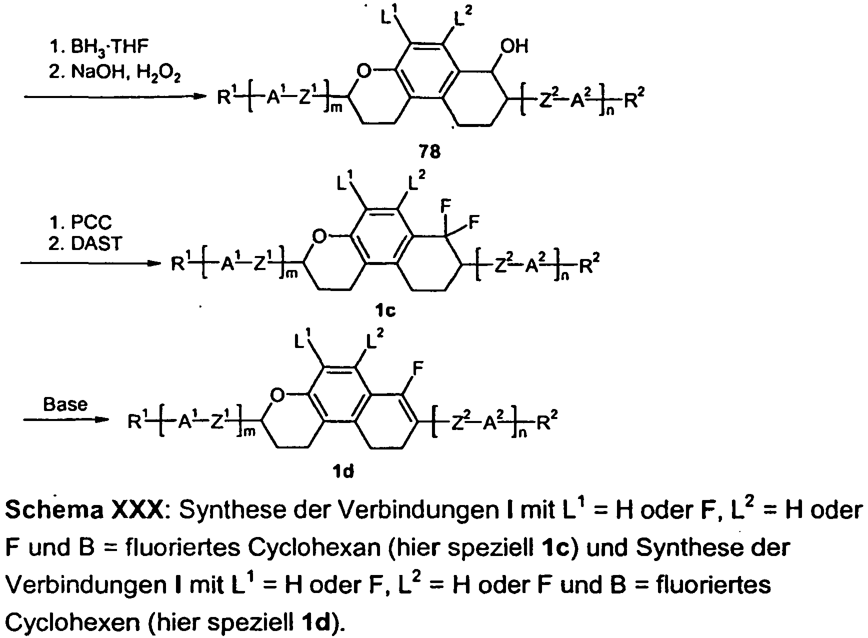

Die dargestellten Reaktionsschemata sind nur als beispielhaft aufzufassen. Der Fachmann kann entsprechende Variationen der vorgestellten Synthesen vornehmen sowie auch andere geeignete Synthesewege beschreiten, um Verbindungen der Formel I zu erhalten.

Gemäß den zuvor dargestellten Synthesen umfasst die vorliegende Erfindung in einer Ausführungsform auch ein oder mehrere Verfahren zur Herstellung von Verbindungen der Formel I. Die Erfindung umfasst somit ein Verfahren zur Herstellung von Verbindungen der Formel I, das dadurch gekennzeichnet ist, dass es einen Verfahrensschritt umfasst, wobei eine Verbindung der Formel 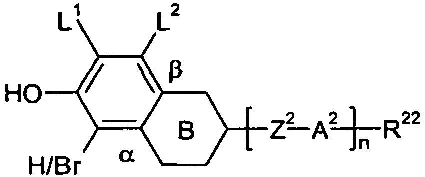 worin
n, A² und Z² wie für Formel I oben definiert sind, und
R²² wie R¹ definiert ist und zusätzlich -OTs, OTf, OMes oder -B(OH)₂, -B(OAlkyl)₂, -B<(O-C₂₋₁₀-Alkylen-O) bedeuten kann,
an der Hydroxylgruppe mit einem geeigneten organischen Rest zu einer Verbindung der Formel 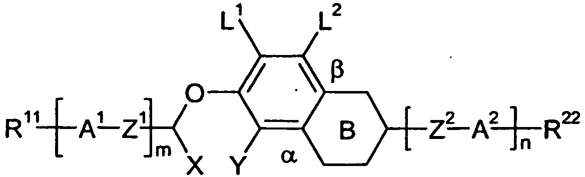 worin m, n, A¹, Z¹, A² und Z² wie für Formel I oben definiert sind,
R¹¹ und R²² wie R¹ definiert sind und zusätzlich -OTs, -OTf, -OMes oder -B(OH)₂ oder -B(OAlkyl)₂ oder -B<(O-C₂₋₁₀-Alkylen-O) bedeuten können,
X -CH₂CH₂Br oder -C≡CH, und
Y H oder Br bedeuten,
verethert und an den Gruppen X und Y cyclisiert wird, vorzugsweise zu einer Verbindung der Formel I. Bevorzugt erfolgt die Cyclisierung anschließend an den ersten Verfahrenschritt. Das Verfahrensprodukt ist eine Verbindung der Formel I oder ein Zwischenprodukt, das sich zur Herstellung von Verbindungen der Formel eignet.
Wie bereits erwähnt, können die Verbindungen der allgemeinen Formel I in flüssigkristallinen Medien verwendet werden.

Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl-oder-cyclohexylester, Phenyl- oder Cyclohexylester der Cyclohexylbenzoesäure, Phenyl- öder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenytethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

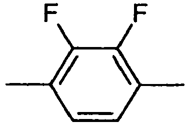

In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (Vlc) beschrieben. In den Verbindungen der Teilformeln (IIc), (IIIc), (IVc), (Vc) und (VIc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchüch. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A:
   0 bis 90 %, vorzugsweise 20 bis 90 %, insbesondere 30 bis 90 %.
Gruppe B:
   0 bis 80 %, vorzugsweise 10 bis 80 %, insbesondere 10 bis 70 %.
Gruppe C:
   0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Die Medien enthalten vorzugsweise eine, zwei, drei, vier oder fünf erfindungsgemäße Verbindungen der Formel I.

Beispiele für die Verbindungen der Formeln (II), (III), (IV), (V) und (VI) sind die nachstehend aufgeführten Verbindungen: 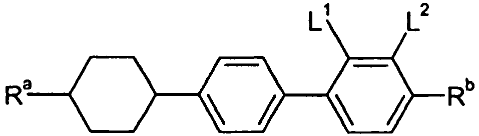 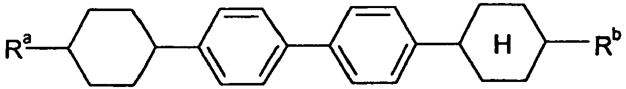 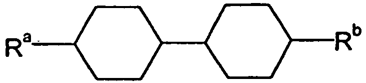 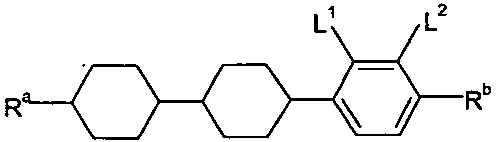 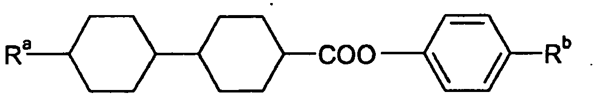 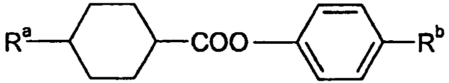 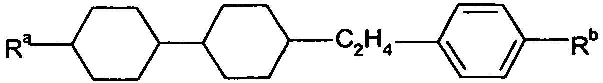 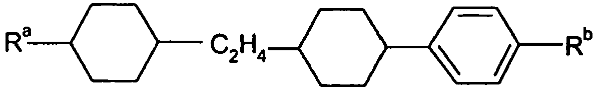 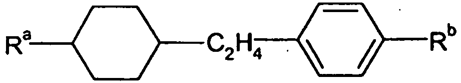 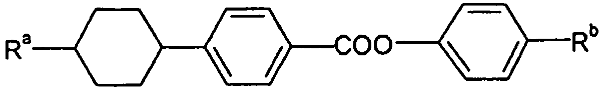 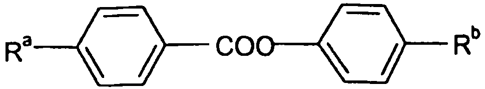 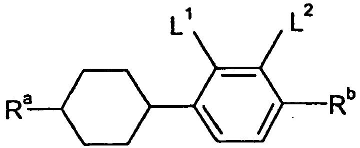 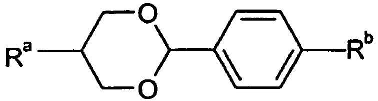 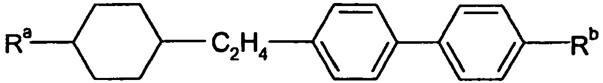 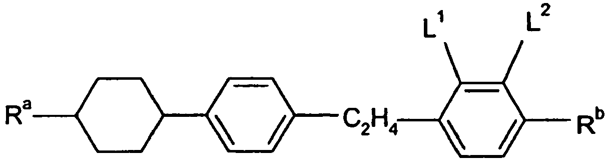 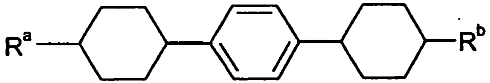 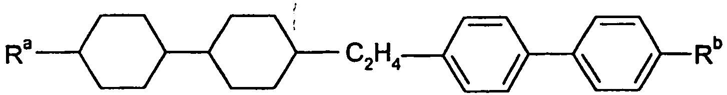 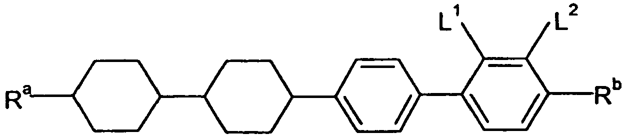 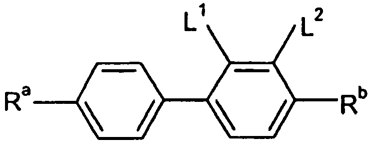 mit R^{a}, R^{b} unabhängig voneinander -CₚH₂ₚ₊₁ oder -OCₚH₂ₚ₊₁ und p = 1, 2, 3, 4, 5, 6, 7 oder 8 sowie L¹, L² unabhängig voneinander -H oder -F, 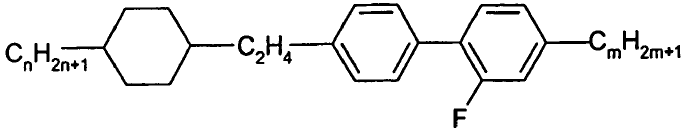 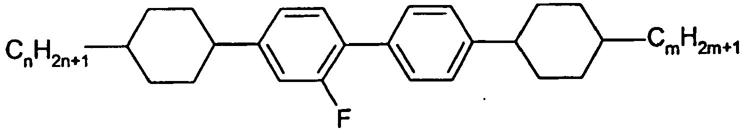 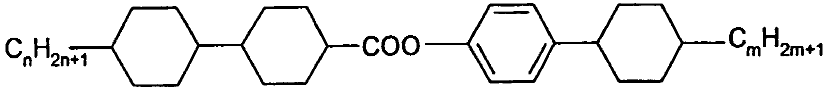 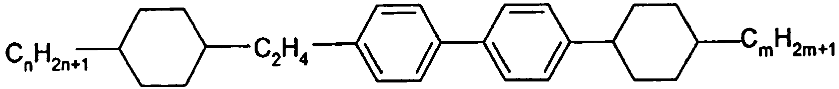 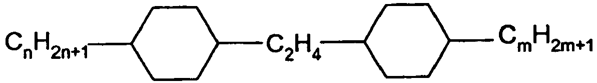 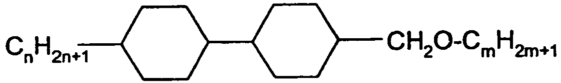 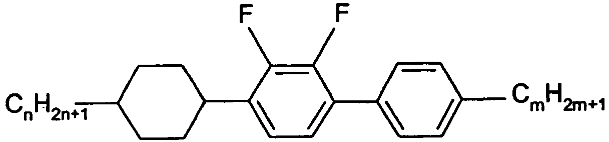 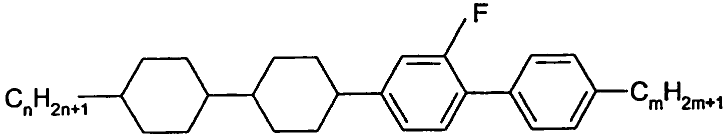 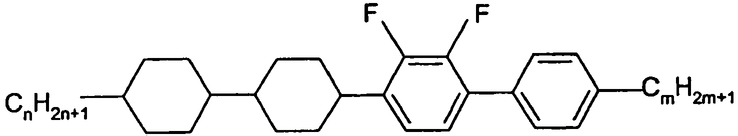 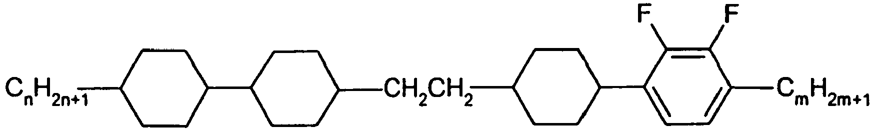 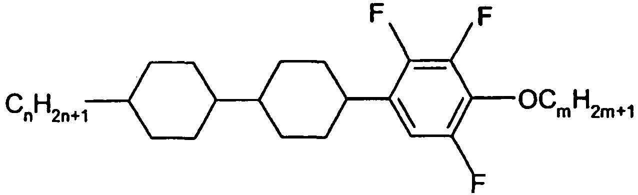 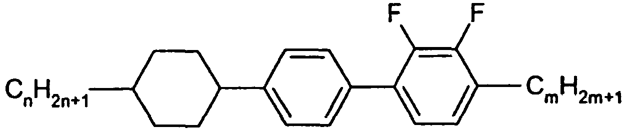 mit m, n unabhängig voneinander 1, 2, 3, 4, 5, 6, 7 oder 8.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Weitere Kombinationen der Ausführungsformen und Varianten der Erfindung gemäß der Beschreibung ergeben sich aus den Ansprüchen.

Die Erfindung wird im Folgenden anhand von Ausführungsbeispielen näher erläutert, ohne dadurch jedoch eingeschränkt werden zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf seine spezielle Problemstellung anzuwenden.

Neben den üblichen und wohlbekannten Abkürzungen werden folgende Abkürzungen verwendet:
K: Kristalline Phase; N: Nematische Phase; Sm: Smektische Phase;
I: Isotrope Phase. Die Zahlen zwischen diesen Symbolen geben die Übergangstemperaturen der betreffenden Substanz wieder.
Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet Δn die optische Anisotropie (589 nm, 20 °C) und Δε die dielektrische Anisotropie (1 kHz, 20 °C). Die dielektrische Anisotropie Δε wird bei 20°C und 1 kHz bestimmt. Die optische Anisotropie Δn wird bei 20°C und einer Wellenlänge von 589,3 nm bestimmt.

Die Δε- und An-Werte, der sowie die Rotationsviskosität (γ₁) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für Δε) bzw. ZLI-4792 (für Δn, γ₁) bestehen (Mischungen Fa. Merck KGaA, Darmstadt).

Nachstehend bedeuten die Abkürzungen:
- DIAD: Diisopropylazodicarboxylat
- MTBE: Methyl-tert-butylether
- THF: Tetrahydrofuran
- DMF: Dimethylformamid
- i. Vak.: im Vakuum (ca. 10⁻² bar)
- ges.: gesättigt
- *n*-BuLi: *n*-Butyllithium, Lösung in Hexan
- Mes: Mesyl-Gruppe
- MOM: Methoxymethyl
- RT: Raumtemperatur
- F-TEDA-BF₄: 1-Chloromethyl-4-fluor-1,4-diazabicyclo[2.2.2]octan-bistetrafluoroborat
- TMP: 2,2,6,6-Tetramethylpiperidin
- dppp: 1,3-Bis(diphenylphosphin)propan
- Tf: Trifyt-Gruppe
- Ts: Tosyl-Gruppe
- NMP: N-Methyl-2-pyrrolidon

### Beispiele

Die Ausgangssubstanzen können nach allgemein zugänglichen Literaturvorschriften oder käuflich erhalten werden.

**Beispiel 1:** 5,6-Difluor-3-(4-propyl-cyclohexyl)-1,2,3,8,9,10-hexahydropyrano[3,2-f]chromen

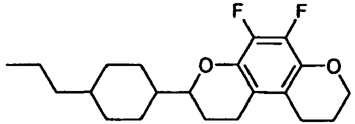

### 1.1 Herstellung von 1,2-Difluor-3-prop-2-inyloxy-benzol

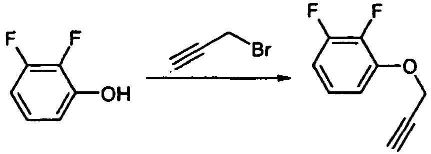

115,0 g (0,88 mol) 2,3-Difluorphenol werden zusammen mit 118,2 ml (17,7 mol) Propargylbromid (80% Lsg. in Toluol) und 146,6 g (138,2 mol) Kaliumcarbonat in 1,6 l Ethylmethylketon 3 h zum Rückfluss erhitzt. Der Ansatz wird filtriert, und der Filterrückstand wird mit MTBE gewaschen. Das Filtrat wird vollständig konzentriert, und der Rückstand wird säulenchromatographisch (SiO₂, *n*-Heptan : MTBE = 3 : 1) gereinigt.

### 1.2 Herstellung von 7,8-Difluoro-2H-chromen

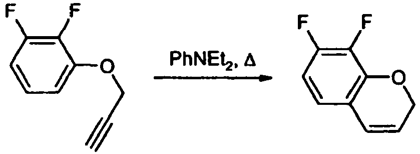

73,0 g (0,43 mol) 1,2-Difluor-3-prop-2-inyloxy-benzol werden im Autoklaven zusammen mit 126,0 g (2,17 mol) Kaliumfluorid in 650 ml N,N-Diethylanilin 3 h auf 200 °C erhitzt. Der Ansatz wird mit Wasser versetzt und mit 25% Salzsäure sauer gestellt. Die Lösung wird mit MTBE extrahiert, und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, *n*-Heptan : MTBE = 10 : 1) gereinigt. Auf diese Weise wird ein Gemisch aus 7,8-Difluoro-2*H*-chromen und dem Ausgangsmaterial 1,2-Difluor-3-prop-2-inyloxy-benzol erhalten.

### 1.3 Herstellung von 7,8-Difluorchroman

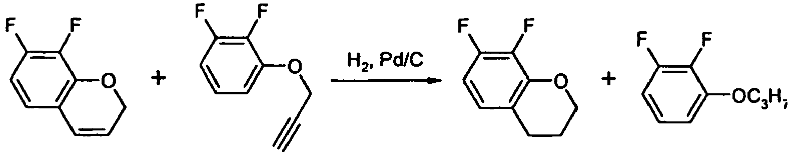

Ein Gemisch aus 7,8-Difluoro-2*H*-chromen und 1,2-Difluor-3-prop-2-inyloxy-benzol (43,2 g) wird in 430 ml THF in Gegenwart von Pd/C (5 % Pd) 1 h bei RT hydriert. Der Ansatz wird vollständig konzentriert, und das Rohprodukt wird säulenchromatographisch (SiO₂, *n*-Pentan : 1-Chlorbutan = 4 : 1) gereinigt. Reines 7,8-Difluorchroman wurde als leicht gelbliche Flüssigkeit erhalten.

### 1.4 Herstellung von 7,8-Difluor-chroman-6-ol

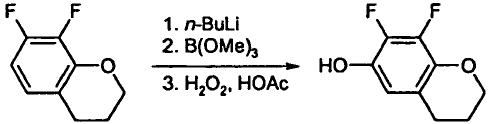

Eine Lsg. von 20,0 g (0,12 mol) 7,8-Difluorchroman in 400 ml THF wird bei -70 °C mit 81,2 ml (0,13 mol) *n*-BuLi (15% Lsg. in Hexan) versetzt. Nach 3 h bei dieser Temperatur werden 14,4 ml (0,13 mol) Trimethylborat zugetropft, und der Ansatz wird auf RT erwärmt. 30 ml verdünnte Essigsäure (ca. 30%) werden zugeben, und der Ansatz wird vorsichtig mit 30 ml wässriger Wasserstoffperoxidlösung (35%) versetzt. Nach beendeter Zugabe wird 17 h bei RT gerührt. Wasser wird zugeben, und der Ansatz wird mit 2 N Salzsäure sauer gestellt. Die Lösung wird mehrfach mit MTBE extrahiert, und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, *n*-Heptan : MTBE = 2:1) gereinigt.

### 1.5 Herstellung von 7,8-Difluor-6-[1-(4-propyl-cyclohexyl)-prop-2-inyloxy]-chroman

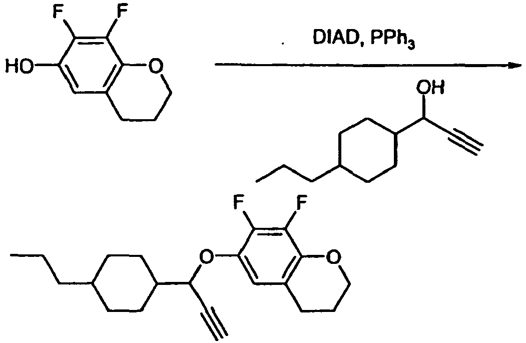

5,0 g (26,9 mmol) 7,8-Difluor-chroman-6-ol werden zusammen mit 5,33 g (29,6 mmol) 1-(4-Propyl-cyclohexyl)-prop-2-yn-1-ol und 8,45 g (32,2 mmol) Triphenylphosphin in 65 ml THF vorgelegt, und unter Eiskühlung werden 6,79 ml (34,9 mmol) DIAD innerhalb von 30 min zugegeben. Nach 19 h bei RT wird halbgesättigte Natriumchloridlösung zugegeben, und die Mischung wird mehrfach mit MTBE extrahiert. Die vereinigten organischen Phasen werden mit Wasser und gesättigter Natriumchloridlösung gewaschen, und die Lösung wird mit Natriumsulfat getrocknet. Der nach dem Entfernen der Lösungsmittel verbleibende Rückstand wird säulenchromatographisch (SiO₂, *n*-Pentan : MTBE = 2:1) gereinigt. 7,8-Difluor-6-[1-(4-propyl-cyclohexyl)-prop-2-inyloxy]-chroman wird als hellbraunes Öl erhalten.

### 1.6 Herstellung von 5,6-Difluor-8-(4-propyl-cyclohexyl)-1,2,3,8-tetrahydropyrano[3,2-f]chromen

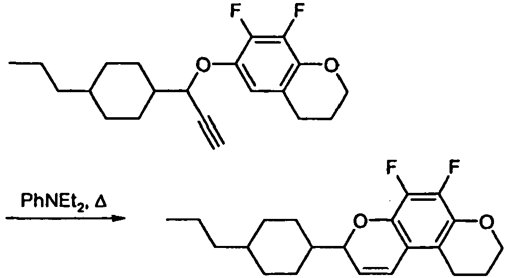

6,0 g (17,2 mmol) 7,8-Difluor-6-[1-(4-propyl-cyclohexyl)-prop-2-inyloxy]-chroman werden zusammen mit 2,0 g (34,4 mmol) Kaliumfluorid in 55 ml N,N-Diethylanilin 6 h auf 200 °C erhitzt. Nach dem Abkühlen wird Wasser zugegeben, und die Mischung wird mit 25% Salzsäure sauer gestellt. Der Ansatz wird mit MTBE extrahiert, und die organische Phase wird mit gesättigter Natriumchloridlösung gewaschen. Die Lösung wird mit Natriumsulfat getrocknet und vollständig konzentriert. Das Rohprodukt wird säulenchromatographisch (SiO₂, 1-Chlorbutan) gereinigt. 5,6-Difluor-8-(4-propyl-cyclohexyl)-1,2,3,8-tetrahydro-pyrano[3,2-f]chromen wird als hellbraunes Öl erhalten.

### 1.7 Herstellung von 5,6-Difluor-3-(4-propyl-cyclohexyl)-1,2,3,8,9,10-hexahydro-pyrano[3,2-f]chromen

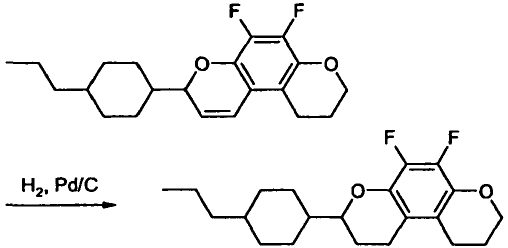

4,0 g (11,5 mmol) 5,6-Difluor-8-(4-propyl-cyclohexyl)-1,2,3,8-tetrahydropyrano[3,2-f]chromen werden in THF und in Gegenwart von Pd/C (5% Pd) bei Normaldruck hydriert. Die Reaktionslösung wird filtriert, und das Filtrat wird vollständig konzentriert. Der Rückstand wird säulenchromatographisch (SiO₂, *n*-Heptan : 1-Chlorbutan = 1 : 1) gereinigt. Die weitere Reinigung erfolgt durch mehrfaches Umkristallisieren aus n-Heptan bei 5 °C. 5,6-Difluor-3-(4-propyl-cyclohexyl)-1,2,3,8,9,10-hexahydro-pyrano[3,2-f]chromen wird als farbloser Feststoff (Schmp. 152 °C) erhalten. 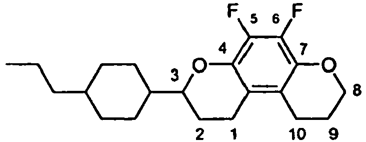
Δε=-10,4
Δn = 0,0663
K 121 l
**¹H-NMR** (250 MHz, CHCl₃): δ = 4,23-4,07 (m, 2H, 8-H), 3,68-3,61 (m, 1H, 3-H), 2,62-2,39 (m, 4H, H*_{aliph.}*), 2,09-1,97 (m, 4H, H_{*aliph*.}), 1,85-1,73 (m, 4H, H*_{aliph.}*), 1,71-1,50 (m, 1H, H*_{aliph.}*), 1,40-1,25 (m, 2H, H*_{aliph.}*), 1,22-1,03 (m, 4H, H*_{aliph.}*), 0,99-0,81 (m, 6H, H*ₐₗᵢₚₕ.*).
**¹⁹F-NMR** (235 MHz, CHCl₃): δ = -161,8 (d, 1F, J = 19,5 Hz), -162,2 (d, 1F, J = 19,5 Hz).
**MS (EI):** m/z(%) = 350 (100, M⁺).

### Beispiel 2: 5,6-Difluor-3-pentyl-8-(4-propyl-cyclohexyl)-1,2,3,8,9,10-hexahydro-pyrano[3,2-f]chromen

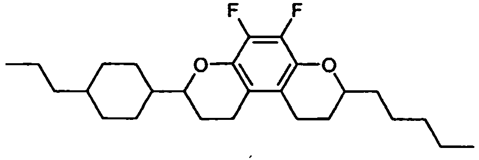

### 2.1 Herstellung von 1-(1-Ethinyl-hexyloxy)-2,3-difluor-benzol

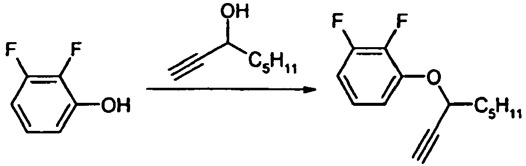

2,4 g (0,33 mol) 2,3-Difluorphenol werden zusammen mit 50,0 ml (0,34 mol) 1-Octin-3-ol und 94,1 g (0,36 mol) Triphenylphosphin in 1,2 I THF vorgelegt, und eine Lösung von 76,1 ml (0,39 mol) DIAD in 100 ml THF wird tropfenweise zugegeben. Nach 19 h bei RT wird mit MTBE verdünnt, und der Ansatz wird mit Wasser gewaschen. Die wässrige Phase wird mit MTBE extrahiert, und die vereinigten organischen Phasen werden mit gesättigter Natriumchloridlösung gewaschen. Die Lösung wird mit Natriumsulfat getrocknet und vollständig konzentriert. Der Rückstand wird säulenchromatographisch (SiO₂, 1-Chlorbutan) gereinigt. 1-(1-Ethinylhexyloxy)-2,3-difluoro-benzol wird als farbloses Öl erhalten.

### 2.2 Herstellung von 7,8-Difluor-2-pentyl-2H-chromen

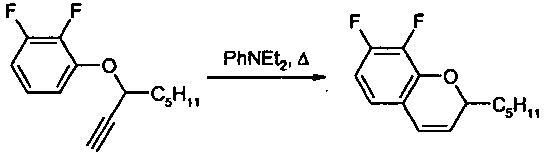

62,0 g (0,26 mol) 1-(1-Ethinyl-hexyloxy)-2,3-difluoro-benzol werden in 390 ml N,N-Diethylanilin 2 h auf 195 °C erhitzt. Der Ansatz wird mit MTBE verdünnt und mehrfach mit 1 N HCl gewaschen. Die organische Phase wird mit Natriumsulfat getrocknet und vollständig konzentriert. Der Rückstand wird säulenchromatographisch (SiO₂, *n*-Pentan : 1-Chlorbutan = 5:1) gereinigt. 7,8-Difluor-2-pentyl-2*H*-chromen wird als braunes Öl erhalten.

### 2.3 Herstellung von 7,8-Difluor-2-pentyl-chroman

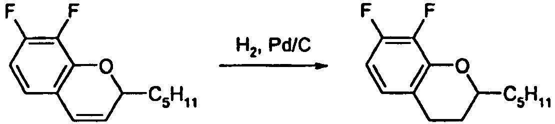

51,0 g (0,21 mol) 7,8-Difluor-2-pentyl-2*H*-chromen werden in 510 ml Toluol in Gegenwart von Pd/C (5% Pd) 1 h bei RT hydriert. Der Ansatz wird vollständig konzentriert: Das Rohprodukt (gelbliche Flüssigkeit) kann direkt für die nächste Stufe verwendet werden.

### 2.4 Herstellung von 7,8-Difluor-2-pentyl-chroman-6-ol

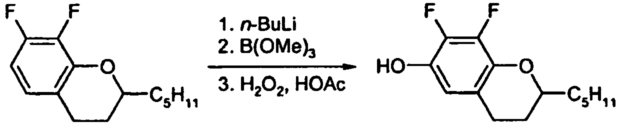

52,4 g rohes (ca. 0,22 mol) 7,8-Difluor-2-pentyl-chroman werden in 400 ml THF vorgelegt und bei -70 °C mit 150,7 ml (0,24 mol) *n*-BuLi (15% Lsg. in Hexan) versetzt. Nach 3 h bei dieser Temperatur werden 26,8 ml (0,24 mol) Trimethylborat zugetropft, und der Ansatz wird auf RT erwärmt. 55 ml verdünnte Essigsäure (ca. 30%) werden zugeben, und der Ansatz wird vorsichtig mit 57 ml Wasserstoffperoxidlösung (35%) versetzt. Nach beendeter Zugabe wird 17 h bei RT gerührt. Wasser wird zugeben, und der Ansatz wird mit HCl sauer gestellt. Die Lösung wird mehrfach mit MTBE extrahiert, und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, *n*-Heptan : MTBE = 2:1) gereinigt.

### 2.5 Herstellung von 7,8-Difluor-2-pentyl-6-[1-(4-propyl-cyclohexyl)-prop-2-inyloxy]-chroman

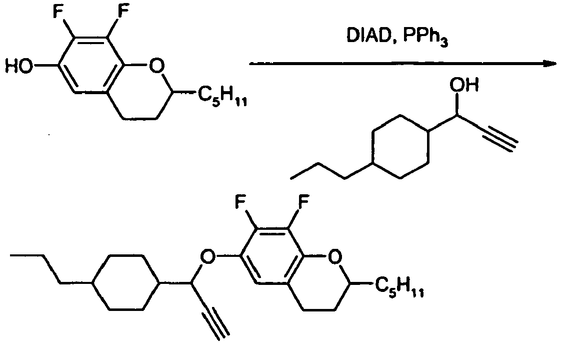

8,0 g (31,2 mmol) 7,8-Difluor-2-pentyl-chroman-6-ol werden zusammen mit 6,75 g (37,4 mmol) 1-(4-Propyl-cyclohexyl)-prop-2-yn-1-ol und 9,83 g (37,6 mmol) Triphenylphosphin in 75 ml THF vorgelegt, und unter Eiskühlung werden 7,89 ml (40,6 mmol) DIAD innerhalb von 30 min zugegeben. Nach 20 h bei RT wird halbgesättigte Natriumchloridlösung zugegeben, und die Mischung wird mehrfach mit MTBE extrahiert. Die vereinigten organischen Phasen werden mit Wasser und gesättigter Natriumchloridlösung gewaschen, und die Lösung wird mit Natriumsulfat getrocknet. Der nach dem Entfernen der Lösungsmittel verbleibende Rückstand wird säulenchromatographisch (SiO₂, *n*-Pentan : MTBE = 2:1) gereinigt. 7,8-Difluor-2-pentyl-6-[1-(4-propyl-cyclohexyl)-prop-2-inyloxy]-chroman wird als hellbraunes Öl erhalten.

### 2.6 Herstellung von 5,6-Difluor-3-pentyl-8-(4-propyl-cyclohexyl)-1,2,3,8-tetrahydro-pyrano[3,2-f]chromen

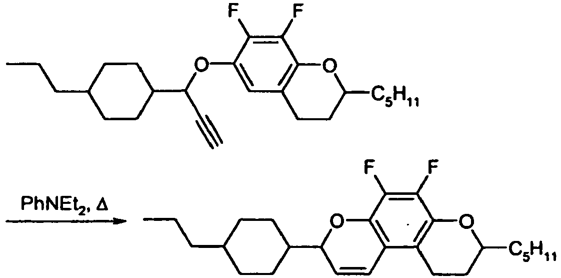

9,7 g (23,2 mmol) 7,8-Difluor-2-pentyl-6-[1-(4-propyl-cyclohexyl)-prop-2-inyloxy]-chroman werden zusammen mit 2,7 g (46,5 mmol) Kaliumfluorid in 75 ml N,N-Diethylanilin 6 h auf 200 °C erhitzt. Nach dem Abkühlen wird Wasser zugegeben, und die Mischung wird mit 25% Salzsäure sauer gestellt. Der Ansatz wird mit MTBE extrahiert, und die organische Phase wird mit gesättigter Natriumchloridlösung gewaschen. Die Lösung wird mit Natriumsulfat getrocknet und vollständig konzentriert. Das Rohprodukt wird säulenchromatographisch (SiO₂, 1-Chlorbutan : Pentan = 4 : 1) gereinigt. 5,6-Difluor-3-pentyl-8-(4-propyl-cyclohexyl)-1,2,3,8-tetrahydro-pyrano[3,2-f]chromen wird als hellbraunes Öl erhalten.

### 2.7 Herstellung von 5,6-Difluor-3-pentyl-8-(4-propyl-cyclohexyl)-1,2,3,8,9,10-hexahydro-pyrano[3,2-f]chromen

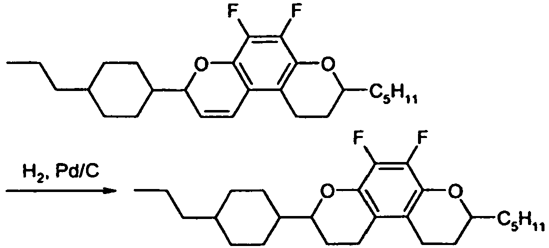

4,8 g (11,5 mmol) 5,6-Difluor-3-pentyl-8-(4-propyl-cyclohexyl)-1,2,3,8-tetrahydro-pyrano[3,2-f]chromen werden in THF und in Gegenwart von Pd/C (5% Pd) bei Normaldruck hydriert. Die Reaktionslösung wird filtriert, und das Filtrat wird vollständig konzentriert. Der Rückstand wird säulenchromatographisch (SiO₂, *n*-Heptan : 1-Chlorbutan = 8:1) gereinigt. Weitere Reinigung erfolgt durch Umkristallisieren aus *n*-Heptan bei 5 °C. 5,6-Difluor-3-pentyl-8-(4-propyl-cyclohexyl)-1,2,3,8,9,10-hexahydro-pyrano[3,2-f]chromen wird als farbloser Feststoff erhalten. Die weitere Auftrennung des erhaltenen Diastereoisomerengemisches erfolgt beispielsweise durch präparative HPLC.

### Isomer 1 (Schmp. 128 °C):

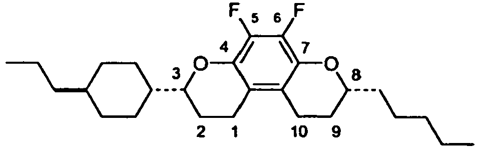

Δε=-11,2
Δn = 0,0742
K128 I
**¹H-NMR** (500 MHz, CHCl₃): δ = 3,94-3,90 (m, 1 H, 8-H), 3,67-3,64 (m, 1 H, 3-H), 2,60-2,54 (m, 2H, H_{*aliph*.}), 2,48- 2,41 (m, 2H, H_{*aliph*.}), 2,06-1,99 (m, 3H, H_{*aliph*.}), 1,82-1,68 (m, 6H, H*_{aliph.}*), 1,61-1,49 (m, 4H, H_{*aliph*.}), 1,46-1,40 (m, 1 H, H_{*aliph*.}),1.35-1,28 (m, 6H, H*_{aliph.}*), 1,26-1,07 (m, 4H, H_{*aliph*.}), 0,96-0,87 (m, 8H, H*_{aliph.}*).
**¹⁹F-NMR** (235 MHz, CHCl₃): δ = -163,1 (m, 2F).
**MS (EI)**: m/z(%) = 420 (100, M⁺).

### Isomer 2 (Schmp. 131 °C):

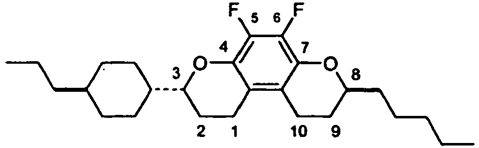

Δε = -9,2
Δn = 0,0894
K 131 I
**¹H-NMR** (500 MHz, CHCl₃): δ = 3,91-3,86 (m, 1 H, 8-H), 3,65-3,61 (m, 1H, 3-H), 2,53-2,49 (m, 4H, H_{*aliph*.}), 2,06-2,00 (m, 3H, H_{*aliph*.}), 1,83-1,66 (m, 6H, H*_{aliph.}*), 1,62-1,50 (m, 4H, H_{*aliph*.}), 1,47-1,40 (m, 1 H, H_{*aliph*.}),1,38-1,28 (m, 6H, H*_{aliph.}*), 1,26-1,07 (m, 4H, H_{*aliph*.}), 0,97-0,87 (m, 8H, H*_{aliph.}*).
**¹⁹F-NMR** (235 MHz, CHCl₃): δ = -163,8 (m, 2F).
**MS (EI)**: m/z(%) = 420 (100, M⁺).

### Beispiel 3: 5,6-Difluor-3-pentyl-8-propyl-1,2,3,8,9,10-hexahydro-pyrano[3,2-f]chromen

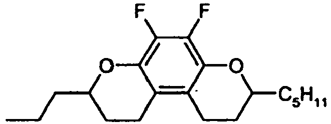

### 3.1 Herstellung von 6-(1-Ethinyl-butoxy)-7,8-difluor-2-pentyl-chroman

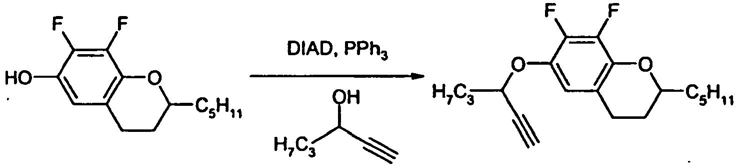

10,0 g (39,0 mmol) 7,8-Difluor-2-pentyl-chroman-6-ol werden zusammen mit 4,0 g (41,0 mmol) 1-Hexin-3-ol und 11,3 g (42,9 mmol) Triphenylphosphin in 140 ml THF vorgelegt, und unter Eiskühlung werden 9,1 ml (46,8 mmol) DIAD innerhalb von 20 min zugegeben. Nach 18 h bei RT wird Wasser zugegeben, und die Mischung wird mehrfach mit MTBE extrahiert. Die vereinigten organischen Phasen werden mit Wasser und gesättigter Natriumchloridlsg. gewaschen, und die Lösung wird mit Natriumsulfat getrocknet. Der nach Entfernung der Lösungsmittel verbleibende Rückstand wird säulenchromatographisch (SiO₂, *n*-Heptan : MTBE = 2 : 1) gereinigt. 6-(1-Ethinyl-butoxy)-7,8-difluor-2-pentyl-chroman wird als gelbes Öl erhalten.

### 3.2 Herstellung von 5,6-Difluor-3-pentyl-8-propyl-1,2,3,8-tetrahydro-pyrano[3,2-f]chromen

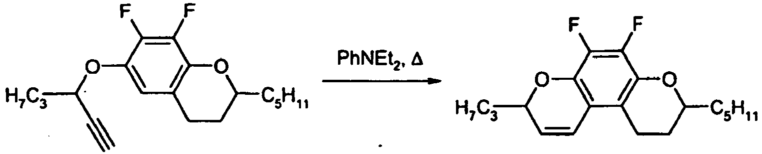

9,0 g (26,8 mmol) 6-(1-Ethinyl-butoxy)-7,8-difluor-2-pentyl-chroman werden in 90 ml N,N-Diethylanilin 3 h auf 205 °C erhitzt. Der Ansatz wird mit MTBE verdünnt und mehrfach mit 3 N HCl gewaschen. Die organische Phase wird mit Natriumsulfat getrocknet und vollständig konzentriert. Der Rückstand wurde säulenchromatographisch (SiO₂, *n*-Heptan : MTBE = 4 : 1) gereinigt. 5,6-Difluor-3-pentyl-8-propyl-1,2,3,8-tetrahydro-pyrano[3,2-f]chromen wird als hellbraunes Öl erhalten.

### 3.3 Herstellung von 5,6-Difluor-3-pentyl-8-propyl-1,2,3,8,9,10-hexahydro-pyrano[3,2-f]chromen

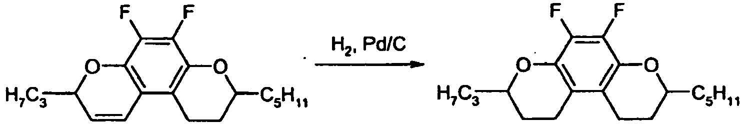

8,0 g (23,8 mmol) 5,6-Difluor-3-pentyl-8-propyl-1,2,3,8-tetrahydro-pyrano[3,2-f]chromen werden in Toluol und in Gegenwart von Pd/C (5 % Pd) 5 h bei Normaldruck hydriert. Die Reaktionslösung wird filtriert, und das Filtrat wird vollständig konzentriert. Der Rückstand wird säulenchromatographisch (SiO₂, *n*-Heptan : 1-Chlorbutan = 1:1) gereinigt. Die weitere Reinigung erfolgt durch Umkristallisieren aus Ethanol. Auf diese Weise wird eine 1:1 Mischung isomerer 5,6-Difluor-3-pentyl-8-propyl-1,2,3,8,9,10-hexahydro-pyrano[3,2-f]chromene erhalten. Eine Trennung erfolgt beispielsweise durch präparative HPLC.

### Isomer 1 (Schmp. 84 °C):

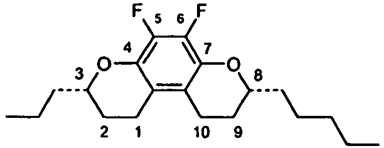

Δε = -10,2
Δn = 0,0463
γ₁ = 295 mPa·s
K 84 l
¹H-NMR (500 MHz, CHCl₃): δ = 3,92-3,80 (m, 2H, 3-H, 8-H), 2,57-2,33 (m, 4H, H*_{aliph.}*), 2,02-1,91 (m, 2H, H*_{aliph.}*), 1,76- 1,60 (m, 4H, H*_{aliph.}*), 1,57-1,36 (m, 8H, H*_{aliph.}*), 1,33-1,17 (m, 6H, H*_{aliph.}*), 0,93-0,78 (m, 6H, H_{*aliph*.}).
**¹⁹F-NMR** (235 MHz, CHCl₃): δ = -163,4 (s, 2F).
**MS (EI)**: m/z(%) = 338 (100, M⁺).

### Isomer 2 (Schmp. 61 °C):

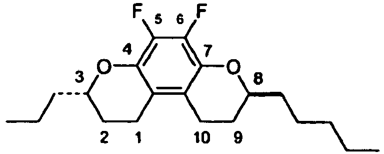

Δε = -11,8
Δn = 0,0464
γ₁ = 488 mPa·s
K 61 l
**¹H-NMR** (500 MHz, CHCl₃): δ = 3,92-3,84 (m, 2H, 3-H, 8-H), 2,56-2,48 (m, 4H, H*_{aliph.}*), 2,08-1,99 (m, 2H, H_{*aliph*.}), 1,84-1,66 (m, 4H, H_{*aliph*.}), 1,64-1,41 (m, 8H, H_{*a*/*iph.*}), 1,36-1,29 (m, 6H, H*_{aliph.}*), 0,97 (t, 3H, J = 7,2 Hz, Me), 0,90 (t, 3H, J = 7,1 Hz, Me).

### Beispiel 4: 5,6-Difluor-3-propyl-8-(4-propyl-cyclohexyl)-2,3,7,8,9,10-hexahydro-1H-benzo[f]chromen

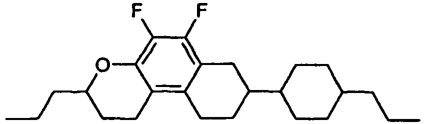

### 4.1 Herstellung von Trifluormethanesulfonsäure-7,8-difluor-3,4-dihydronaphthalin-2-ylester

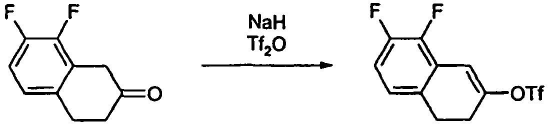

54,0 g (1,35 mol) Natriumhydrid (60% Suspension in Paraffinöl) werden mit Pentan gewaschen und in 1,25 I Diethylether suspendiert. Der Suspension wird eine Lsg. von 120,0 g (0,66 mol) 7,8-Difluor-3,4-dihydro-*1H-*naphthalin-2-on in 750 ml Diethylether langsam zudosiert, und der Ansatz wird nach beendeter Zugabe 30 min bei RT gerührt. Die Mischung wird auf 0 °C gekühlt, und 120,0 ml (0,71 mol) Trifluoressigsäureanhydrid werden tropfenweise zugegeben. Nach beendeter Zugabe wird 2 h bei 0 °C gerührt, und der Ansatz wird zu verd. Salzsäure gegeben. Die Mischung wird mehrfach mit MTBE extrahiert, und die vereinigten organischen Phasen werden mit ges. Natriumchloridlsg. gewaschen. Die Lösung wird absorptiv (SiO₂, MTBE) filtriert, und das Filtrat wird vollständig konzentriert. Der Rückstand wird mit n-Heptan : Toluol = 7 : 3 behandelt, und die organische Phase wird abgetrennt. Die Lösung wird vollständig konzentriert, und der Rückstand wird säulenchromatographisch (SiO₂, n-Heptan : Toluol = 7 : 3) gereinigt. Trifluormethanesulfonsäure-7,8-difluor-3,4-dihydronaphthalin-2-ylester wird als gelbes Öl erhalten.

### 4.2 Herstellung von 5,6-Difluor-3-(4-propyl-cyclohexyl)-1,2-dihydronaphthalin

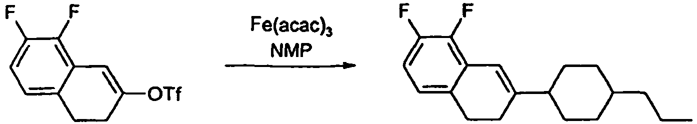

9,0 g (28,6 mmol) Trifluormethanesulfonsäure-7,8-difluor-3,4-dihydronaphthalin-2-ylester werden zusammen mit 550 mg (1,55 mmol) Fe(acac)₃ und 30 ml NMP in 500 ml THF bei -30 °C vorgelegt. Eine aus 30,0 g (0,15 mol) 4-Brom-propylcyclohexan und 3,6 g (0,15 mol) Magnesiumspänen, in 350 ml Diethylether erzeugte Lösung von 4-Propylcyclohexylmagnesiumbromid wird zügig zudosiert. Nach beendeter Zugabe wird 30 min bei -30 °C gerührt, und das Gemisch wird zu ges. Ammoniumchloridlsg. gegeben. Die Mischung wird mehrfach mit MTBE extrahiert, und die vereinigten organischen Phasen werden mit ges. Natriumchloridlsg. gewaschen. Die Lösung wird mit Natriumsulfat getrocknet und vollständig konzentriert. Der Rückstand wird säulenchromatographisch (SiO₂, *n*-Heptan) gereinigt. 5,6-Difluor-3-(4-propyl-cyclohexyl)-1,2-dihydronaphthalin wird als farbloses Öl erhalten.

### 4.3 Herstellung von 7,8-Difluor-2-(4-propyl-cyclohexyl)-1,2,3,4-tetrahydronaphthalin

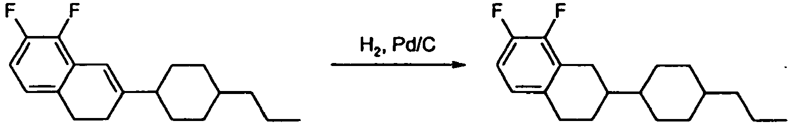

76,0 g (0,26 mol) 5,6-Difluor-3-(4-propyl-cyclohexyl)-1,2-dihydronaphthalin werden in 500 ml THF bei RT und Normaldruck in Gegenwart von Pd-C (5% Pd) 19 h hydriert. Die Reaktionslösung wird filtriert und vollständig konzentriert. Der Rückstand wird ohne weitere Reinigung für die folgende Umsetzung verwendet.

### 4.4 Herstellung von 3,4-Difluor-6-(4-propyl-cyclohexyl)-5,6,7,8-tetrahydronaphthalin-2-ol

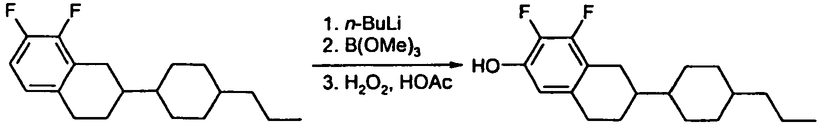

77,0 g (ca. 0,26 mol) rohes 7,8-Difluor-2-(4-propyl-cyclohexyl)-1,2,3,4-tetrahydronaphthalin werden in 500 ml THF vorgelegt und bei -70 °C mit 200,0 ml (0,32 mol) *n*-BuLi (15% Lsg. in Hexan) versetzt. Nach 3 h bei dieser Temperatur werden 37,0 ml (0,33 mol) Trimethylborat zugetropft, der Ansatz wird auf 0 °C erwärmt, und 80 ml verdünnte Essigsäure (ca. 30 %) werden zugeben. Die Mischung wird auf 30 °C erwärmt, und 70 ml Wasserstoffperoxidlösung (35 %) werden vorsichtig zugegeben. Nach beendeter Zugabe wird 2 h bei RT gerührt. Wasser wird zugeben und der Ansatz wird mit verd. Salzsäure sauer gestellt. Die Lösung wird mehrfach mit MTBE extrahiert und die vereinigten organischen Phasen werden nacheinander mit Wasser, ges. Natriumchloridlsg. und Ammoniumeisen(II)-sulfatlsg. gewaschen. Die Lösung wird mit Natriumsulfat getrocknet und vollständig konzentriert. Das Rohprodukt wird säulenchromatographisch (SiO₂, Toluol) gereinigt. Die weitere Reinigung erfolgt durch Kristallisation aus n-Heptan. 3,4-Difluor-6-(4-propyl-cyclohexyl)-5,6,7,8-tetrahydronaphthalin-2-ol wird als farbloser Feststoff erhalten.

### 4.5 Herstellung von 6-(1-Ethinyl-butoxy)-7,8-difluor-2-(4-propyl-cyclohexyl)-1,2,3,4-tetrahydro-naphthalin

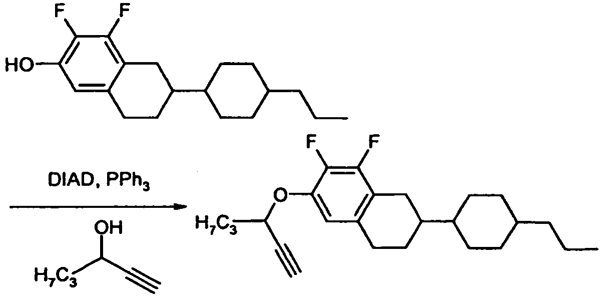

7,0 g (22,7 mmol) 3,4-Difluor-6-(4-propyl-cyclohexyl)-5,6,7,8-tetrahydronaphthalin-2-ol werden zusammen mit 2,70 ml (24,0 mmol) 1-Hexin-3-ol und 6,60 g (25,2 mmol) Triphenylphosphin in 90 ml THF vorgelegt. Unter Eiskühlung werden 5,3 ml (46,8 mmol) DIAD in 10 ml THF innerhalb von 20 min zugegeben. Nach 19 h bei RT wird Wasser zugegeben, und die Mischung wird mehrfach mit MTBE extrahiert. Die vereinigten organischen Phasen werden mit Wasser und gesättigter Natriumchloridlsg. gewaschen, und die Lösung wird mit Natriumsulfat getrocknet. Der nach Entfernung der Lösungsmittel verbleibende Rückstand wird säulenchromatographisch (SiO₂, *n*-Heptan : MTBE = 2 : 1) gereinigt. 6-(1-Ethinyl-butoxy)-7,8-difluor-2-(4-propyl-cyclohexyl)-1,2,3,4-tetrahydro-naphthalin wird als farbloser Feststoff erhalten.

### 4.6 Herstellung von 5,6-Difluor-3-propyl-8-(4-propyl-cyclohexyl)-7,8,9,10-tetrahydro-3H-benzo[f]chromen

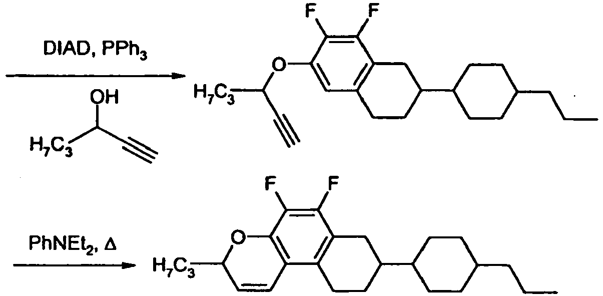

4,5 g (11,6 mmol) 6-(1-Ethinyl-butoxy)-7,8-difluor-2-(4-propyl-cyclohexyl)-1,2,3,4-tetrahydro-naphthalin werden in 45 ml N,N-Diethylanilin 2 h auf 200 °C erhitzt. Nach dem Abkühlen wird der Ansatz zu einem Gemisch aus 2N Salzsäure und Eis gegeben, und die Mischung wird mit MTBE extrahiert. Die organische Phase wird mit 2N Salzsäure und Wasser gewaschen, und die Lösung wird mit Natriumsulfat getrocknet. Der nach Entfernen des Lösungsmittels verbleibende Rückstand wird säulenchromatographisch (SiO₂, *n*-Heptan : Toluol = 9 : 1) gereinigt. 5,6-Difluor-3-propyl-8-(4-propyl-cyclohexyl)-7,8,9,10-tetrahydro-3H-benzo[f]chromen wird als gelber Feststoff erhalten.

### 4.7 Herstellung von 5,6-Difluor-3-propyl-8-(4-propyl-cyclohexyl)-2,3,7,8,9,10-hexahydro-1H-benzo[f]chromen

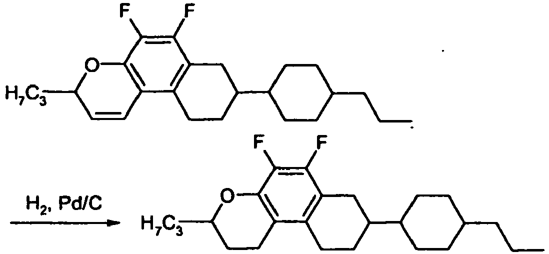

5,0 g (ca. 11,4 mmol) 5,6-Difluor-3-propyl-8-(4-propyl-cyclohexyl)-7,8,9,10-tetrahydro-3H-benzo[f]chromen werden in THF und in Gegenwart von Pd/C (5 % Pd) bei Normaldruck hydriert. Die Reaktionslösung wird filtriert, und das Filtrat wird vollständig konzentriert. Der Rückstand wird säulenchromatographisch (SiO₂, *n*-Heptan : Toluol = 9 : 1) gereinigt. Die weitere Reinigung erfolgt durch Umkristallisieren aus Ethanol. Auf diese Weise wird eine 1:1 Mischung isomerer 5,6-Difluor-3-propyl-8-(4-propylcyclohexyl)-2,3,7,8,9,10-hexahydro-1H-benzo[f]chromene erhalten (Schmp. 89 °C). Eine Trennung kann beispielsweise durch präparative HPLC erfolgen. 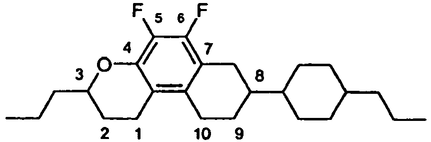
Δε = -7,5
Δn = 0,0879
K 89 N 113 l
**¹H-NMR** (500 MHz, CHCl₃): δ = 4,01-3,90 (m, 1H, 3-H), 2,84-2,77 (m, 1 H), 2,69-2,22 (m, 5H), 2,07-1,93 (m, 2H), 1,86-1,66 (m, 5H), 1,62-1,43 (m, 4H), 1,41-1,26 (m, 4H), 1,24-1,11 (m, 4H), 1,08-0,83 (m, 10H).
**¹⁹F-NMR** (376 MHz, CHCl₃): δ = -146,4 (dd, 1F, J = 21,1 Hz, J = 3,4 Hz), - 165,9 (dd, 1F, J = 21,1 Hz, J = 11,7 Hz).
**MS (EI)**: m/z(%) = 390 (100, M⁺), 321 (78).

### Beispiel 5: 8-Ethoxy-5,6-difluor-3-(4-propyl-cyclohexyl)-2,3-dihydro-1H-benzo[f]chromen

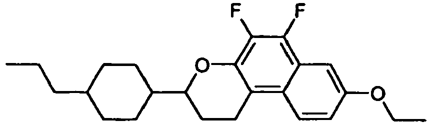

### 5.1 Herstellung von 7,8-Difluor-3-trimethylsilanyl-naphthalin-2-ol

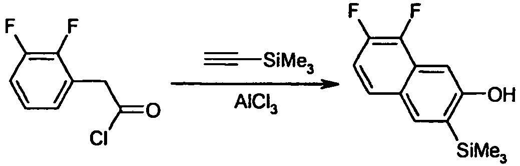

Eine Suspension von 71,5 g (0,53 mol) Aluminium(III)-chlorid in 300 ml Dichlormethan wird bei -20 °C mit einer Lösung von 50,0 g (0,26 mol) (2,3-Difluorphenylessigsäurechlorid in 100 ml Dichlormethan langsam versetzt. Nach 30 min bei dieser Temperatur wird Trimethylsilylacetylen zudosiert, und die Mischung wird 30 min gerührt. Der Ansatz wird zu Eiswasser gegeben und mit Salzsäure sauer gestellt. Die organische Phase wird abgetrennt, und die wässrige Phase wird mit Ethylacetat extrahiert. Die vereinigten organischen Phasen werden nacheinander mit ges. Natriumhydrogencarbonatlsg. und ges. Natriumchloridlsg. gewaschen. Die Lösung wird mit Natriumsulfat getrocknet und vollständig konzentriert. Der Rückstand wird säulenchromatographisch (SiO₂, *n*-Heptan : Ethylacetat = 9 : 1) gereinigt. Auf diese Weise wird 7,8-Difluor-3-trimethylsilanyl-naphthalin-2-ol als braunes Öl erhalten.

### 5.2 Herstellung von (3-Ethoxy-5,6-difluor-naphthalin-2-yl)-trimethylsilan

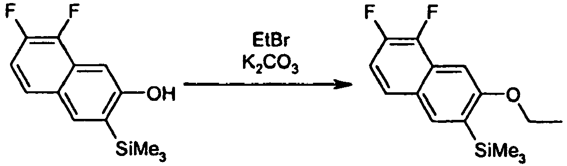

54,7 g (ca. 0,12 mol) 7,8-Difluor-3-trimethylsilanyl-naphthalin-2-ol werden zusammen mit 19,5 ml (0,26 mol) Bromethan und 34,5 g (0,25 mol) Kaliumcarbonat in 400 ml Ethylmethylketon 19 h bei 80 °C gerührt. Der Ansatz wird filtriert, und der Rückstand wird mit Ethylmetyhlketon gewaschen. Das Filtrat wird konzentriert, und der Rückstand in MTBE aufgenommen. Die Lösung wird mit Wasser und ges. Natriumchloridlsg. gewaschen und mit Natriumsulfat getrocknet. Der nach Entfernen des Lösungsmittels verbleibende Rückstand wird säulenchromatographisch (SiO₂, Toluol : Ethylacetat = 9 : 1) gereinigt. (3-Ethoxy-5,6-difluor-naphthalin-2-yl)-trimethylsilan wird als dunkelbrauner Feststoff erhalten.

### 5.3 Herstellung von 7-Ethoxy-1,2-difluornaphthalin

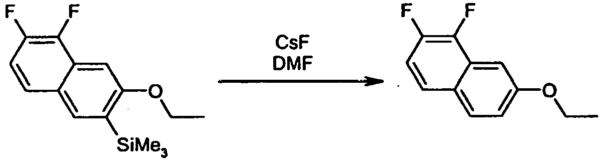

44,0 g (ca. 81,6 mmol) (3-Ethoxy-5,6-difluor-naphthalin-2-yl)-trimethylsilan werden zusammen mit 26,3 g (0,17 mol) Cäsiumfluorid in 300 ml DMF 20 h bei 100 °C gerührt. Nach dem Abkühlen wird mit Wasser verdünnt, und die Mischung wird mehrfach mit MTBE extrahiert. Die vereinigten organischen Phasen werden mit Wasser und ges. Natriumchloridlsg. gewaschen, und die Lösung wird mit Natriumsulfat getrocknet. Das nach Entfernen des Lösungmittels verbleibende braune Öl wird säulenchromatographisch (SiO₂, Toluol : Ethylacetat = 9 : 1) gereinigt. 7-Ethoxy-1,2-difluornaphthalin wird als braunes Öl erhalten.

### 5.4 Herstellung von 6-Ethoxy-3,4-difluor-naphthalin-2-ol

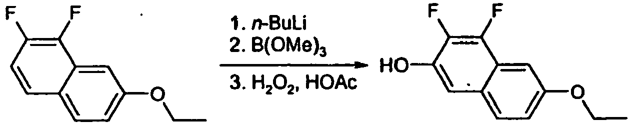

30,0 g (ca. 0,1 mol) 7-Ethoxy-1,2-difluornaphthalin werden in 300 ml THF vorgelegt, und bei -75 °C werden 130,0 ml (0,21 mol) *n*-BuLi (15% Lsg. in Hexan) zugegeben. Nach 1 h bei dieser Temperatur werden 25,0 ml (0,22 mol) Trimethylborat zugetropft, und der Ansatz wird auf -10 °C erwärmt. 50 ml verdünnte Essigsäure (ca. 30%) werden zugeben, und die Mischung wird auf 30 °C erwärmt. 40 ml Wasserstoffperoxidlösung (35 %) werden vorsichtig zugegeben, und die Mischung wird 18 h intensiv gerührt. Wasser wird zugeben, und der Ansatz wird mit Ammoniumeisen(II)-sulfat versetzt. Die Lösung wird mehrfach mit MTBE extrahiert und die vereinigten organischen Phasen werden nacheinander mit Wasser und ges. Natriumchloridlsg. gewaschen. Die Lösung wird mit Natriumsulfat getrocknet und vollständig konzentriert. Das Rohprodukt wird säulenchromatographisch (SiO₂, Toluol : Ethylacetat = 9 : 1) gereinigt. 6-Ethoxy-3,4-difluor-naphthalin-2-ol wird als hellbrauner Feststoff erhalten.

### 5.5 Herstellung von 7-Ethoxy-1,2-difluor-3-[1-(4-propyl-cyclohexyl)-prop-2-inyloxy]-naphthalin

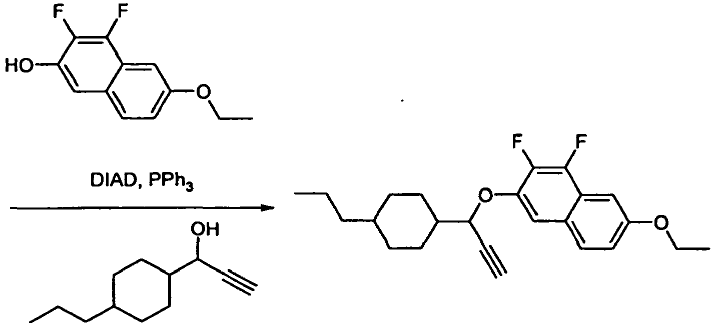

10,5 g (46,8 mmol) 6-Ethoxy-3,4-difluor-naphthalin-2-ol werden zusammen mit 10,0 g (55,5 mmol) 1-(4-Propyl-cyclohexyl)-prop-2-in-1-ol und 15,0 g (57,2 mmol) Triphenylphosphin in 100 ml THF vorgelegt, und unter Eiskühlung werden 11,5 ml (59,1 mmol) DIAD innerhalb von 20 min zugegeben. Nach 18 h bei RT wird Wasser zugegeben, und die Mischung wird mehrfach mit MTBE extrahiert. Die vereinigten organischen Phasen werden mit Wasser und gesättigter Natriumchloridlsg. gewaschen, und die Lösung wird mit Natriumsulfat getrocknet. Der nach Entfernen der Lösungsmittel verbleibende Rückstand wird säulenchromatographisch (SiO₂, *n*-Heptan : MTBE = 2 : 1) gereinigt. Die weitere Reinigung erfolgt durch Umkristallisation aus *n*-Heptan. 7-Ethoxy-1,2-difluor-3-[1-(4-propylcyclohexyl)-prop-2-inyloxy]-naphthalin wird als gelblicher Feststoff erhalten.

### 5.6 Herstellung von 8-Ethoxy-5,6-difluor-3-(4-propyl-cyclohexyl)-3H-benzo[f]chromen

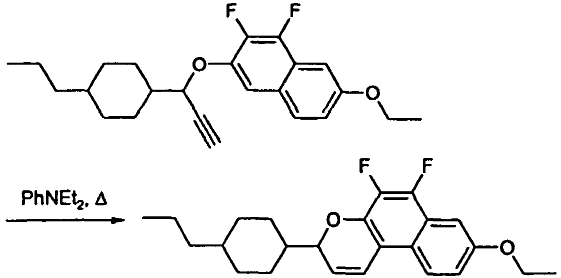

7,75 g (ca. 18,6 mmol) 7-Ethoxy-1,2-difluor-3-[1-(4-propyl-cyclohexyl)-prop-2-inyloxy]-naphthalin werden in 80 ml N,N-Diethylanilin 4,5 h auf 205 °C erhitzt. Der Ansatz wird mit MTBE verdünnt und mehrfach mit Salzsäure gewaschen. Die organische Phase wird mit Natriumsulfat getrocknet und vollständig konzentriert. Der Rückstand wird säulenchromatographisch (SiO₂, *n*-Heptan : MTBE = 4 : 1) gereinigt. Die weitere Reinigung erfolgt durch Umkristallisieren aus n-Heptan. 8-Ethoxy-5,6-difluor-3-(4-propyl-cyclohexyl)-3H-benzo[f]chromen wird als gelber Feststoff erhalten.

### 5.7 Herstellung von 8-Ethoxy-5,6-difluor-3-(4-propyl-cyclohexyl)-2,3-dihydro-1H-benzo[f]chromen

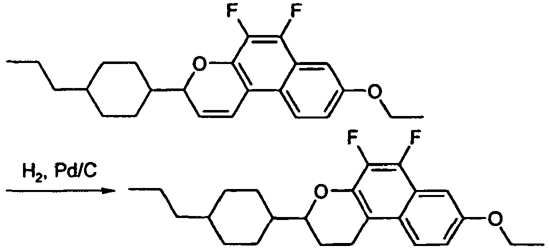

5,1 g (13,1 mmol) 8-Ethoxy-5,6-difluor-3-(4-propyl-cyclohexyl)-3H-benzo[f]chromen werden in THF und in Gegenwart von Pd/C (5 % Pd) bei Normaldruck hydriert. Die Reaktionslösung wird filtriert, und das Filtrat wird vollständig konzentriert. Der Rückstand wird aus Ethylacetat umkristallisiert. 8-Ethoxy-5,6-difluor-3-(4-propyl-cyclohexyl)-2,3-dihydro-1 H-benzo[f]chromen wird als farbloser Feststoff (Schmp. 158 °C) erhalten. 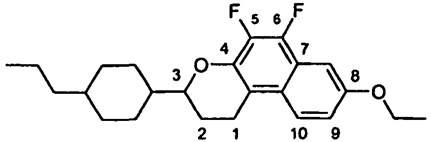
Δε = -5,4
Δn = 0,1550
K 158 N 159 l
**¹H-NMR** (400 MHz, CHCl₃): δ = 7,64 (dd, 1H, J = 9,2 Hz, J = 1,6 Hz, 10-H), 7,24 (d, 1 H, J = 2,4 Hz, 7a-H), 7,12 (dd, 1 H, J = 9,2 Hz, J = 2,4 Hz, 9-H), 4,14 (q, 2H, J = 7,0 Hz, OCH₂CH₃), 3,84-3,79 (m 1H, 3-H), 3,08-3,00 (m 1 H, 1-H), 2,97-2,87 (m 1 H, 1-H), 2,20-2,06 (m, 2H, H_{*aliph*.}), 1,94-1,79 (m, 4H, H_{*aliph*.}), 1,71-1,60 (m, 1H, H_{*aliph*.}), 1,47 (t, 3H, J = 7,0 Hz, OCH₂**CH**₃), 1,38-1,11 (m, 7H, H_{*aliph*.}), 1,00-0,86 (m, 5H, H_{*aliph*.}).
**¹⁹F-NMR** (376 MHz, CHCl₃): δ = -152,5 (dd, 1 F, J = 17,8 Hz, J = 1,2 Hz), - 160,5 (d, 1 F, J = 17,8 Hz).
**MS (EI)**: m/z(%) = 388 (89, M⁺), 237 (100).

## Patentansprüche

1. Verbindungen der Formel I: 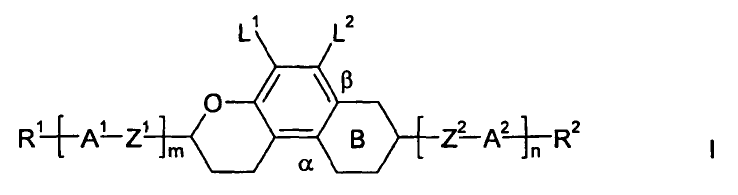 worin
m und n jeweils unabhängig voneinander 0, 1, 2 oder 3 sind, wobei m + n ≤ 4;
L¹ und L² jeweils unabhängig voneinander H, Halogen, CN, CF₃, CHF₂, CH₂F und CH₃ bedeuten, wobei einer der Reste L¹ oder L² ein F bedeutet oder beide der Reste L¹ und L² ein F bedeuten,
A¹ und A² jeweils unabhängig voneinander
(a) 1,4-Phenylen, worin =CH- ein- oder zweimal durch =N-ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁₋C₆-Alkanyl, unsubstituiertem oder mit Fluor und/oder Chlor ein- oder mehrfach substituiertem C₁-C₆-Alkoxy substituiert sein kann,
(b) 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F und/oder -Cl substituiert sein können, oder
(c) Bicyclo[1.1.1]pentan-1,3-diyl, Bicyclo[2.2.2]octan-1,4-diyl oder Spiro[3.3]heptan-2,6-diyl,
bedeuten;
Z¹ und Z² jeweils unabhängig voneinander eine Einfachbindung, -CF₂O-, -OCF₂-, -CH₂CH₂-, -CF₂CF₂-, -CF₂CH₂-, -CH₂CF₂-, -(CO)O-, -O(CO)-, -CH₂O-, -OCH₂-, -CF=CH-, -CH=CF-, -CF=CF-, -CH=CH-, -C≡C- oder eine Kombination aus zwei dieser Gruppen, wobei O-Atome nicht direkt verknüpft sind, bedeuten;
R¹ und R² unabhängig voneinander, Wasserstoff, einen unsubstituierten, einen einfach mit -CN- oder einen einfach oder mehrfach mit -F, -Cl, und/oder -Br substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -(CO)O-, -O(CO)- oder -O(CO)O- so ersetzt sein können, dass Heteroatome nicht direkt verknüpft sind, -F, -Cl, -Br, -CN, -SCN oder -SF₅ bedeuten; und
Ring B 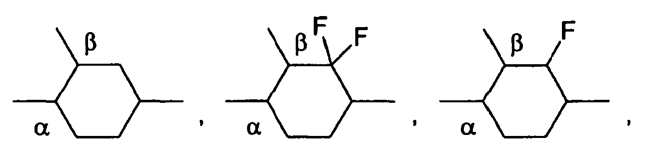 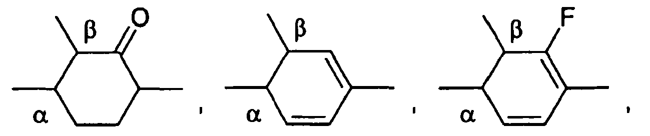 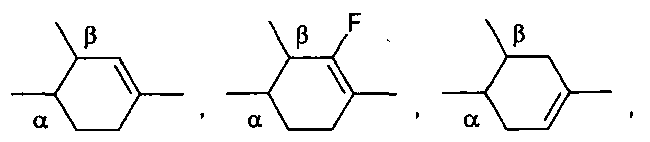 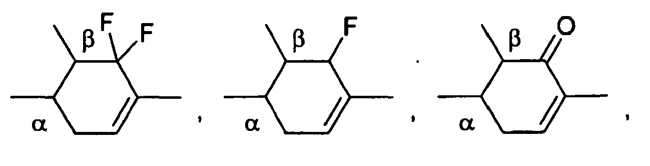 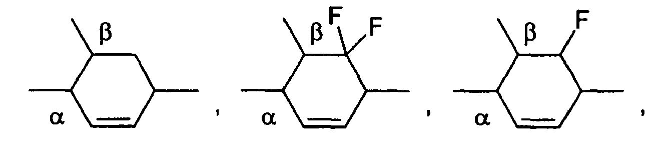 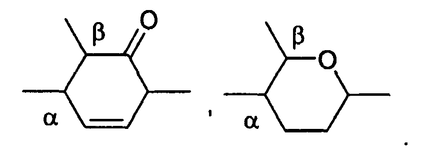 wobei
A¹ und A² bzw. Z¹ und Z² jeweils die gleiche oder unterschiedliche Bedeutungen haben können, wenn m oder n größer als 1 sind,
wobei
R¹ und R² nicht gleichzeitig H bedeuten, und wobei
für den Fall, dass
R¹=H und m=0 oder R²=H und n=0 dann
der Ring B ein Pyran der Formel 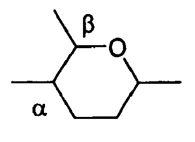 bedeutet.

2. Verbindungen nach Anspruch 1, **dadurch gekennzeichnet, dass** die Substituenten L¹ und L² F bedeuten.

3. Verbindungen nach Anspruch 1 oder 2, **dadurch gekennzeichnet, dass** die Verbindungen der Formel I ausgewählt sind aus der Unterformel IA bis IF 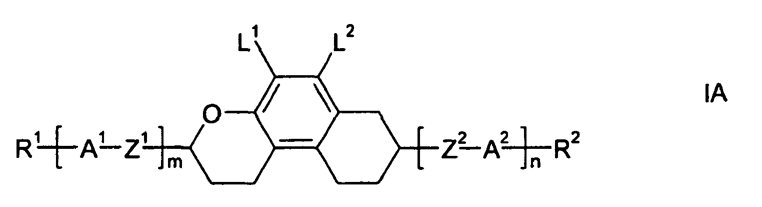 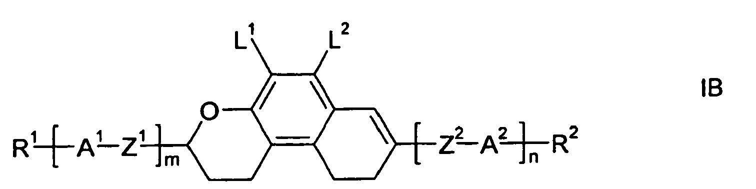 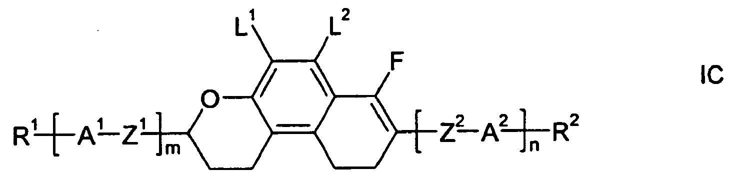 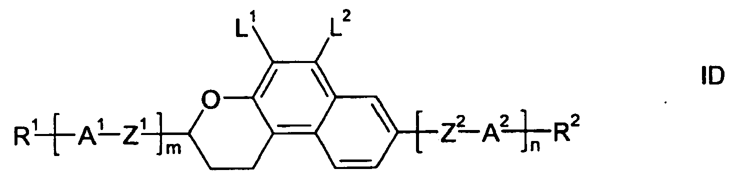 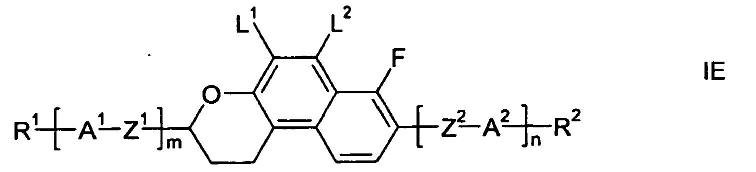 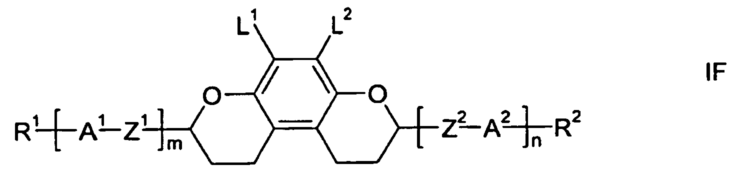 worin R¹, R², A¹, A², L¹, L², Z¹ und Z² die gleichen Bedeutungen wie für die Formel I definiert besitzen.

4. Verbindungen nach einem oder mehreren der Ansprüche 1 bis 3, **dadurch gekennzeichnet, dass**
Z¹ und Z² unabhängig voneinander eine Einfachbindung, -CF₂O-, -OCF₂-, -OCH₂-, -CH₂O-, -CF₂CF₂-, -CH=CH-, -CF=CH-, -CH=CF-oder -CF=CF- bedeuten.

5. Verbindungen nach einem oder mehreren der Ansprüche 1 bis 4, **dadurch gekennzeichnet, dass** A¹ und A² unabhängig voneinander einen Ring der Formeln 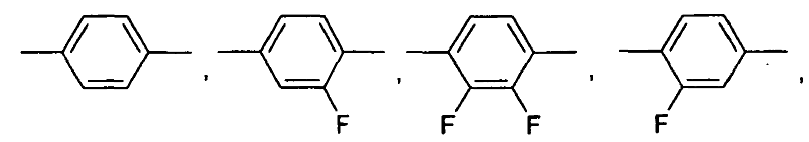 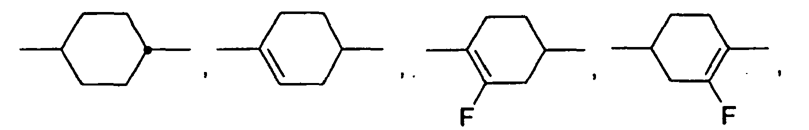 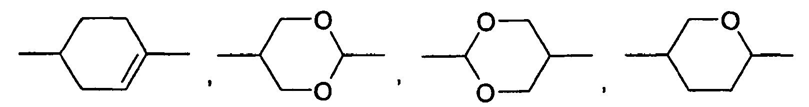 oder 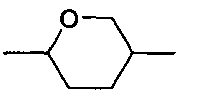 bedeuten.

6. Verbindungen nach einem oder mehreren der Ansprüche 1 bis 5, **dadurch gekennzeichnet, dass**
R¹ und R² jeweils unabhängig voneinander ein Alkanylrest, Alkoxyrest oder Alkenylrest mit 1 bis 7 bzw. 2 bis 7 Kohlenstoffatomen sind, wobei jeder dieser Reste unsubstituiert oder mit Halogen einfach oder mehrfach substituiert ist, oder Fluor oder Wasserstoff bedeutet.

7. Verbindungen nach einem oder mehreren der Ansprüche 1 bis 6, **dadurch gekennzeichnet, dass**
m und n beide null sind, und
R¹ und R² jeweils unabhängig voneinander ein unverzweigter Alkanylrest, Alkoxyrest oder Alkenylrest mit 1 bis 7 bzw. 2 bis 7 Kohlenstoffatomen sind.

8. Verbindungen nach einem oder mehreren der Ansprüche 1 bis 6, **dadurch gekennzeichnet, dass** m + n = 1 ist und
A¹, A² unabhängig voneinander 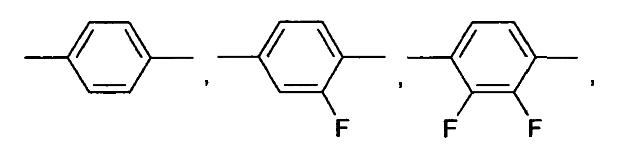 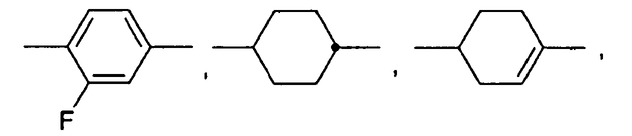 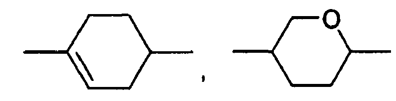 oder 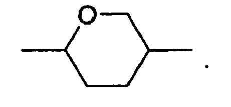
bedeuten.

9. Verwendung einer oder mehrerer Verbindungen nach einem oder mehreren der Ansprüche 1 bis 8 in flüssigkristallinen Medien.

10. Flüssigkristallines Medium enthaltend mindestens zwei Verbindungen, **dadurch gekennzeichnet, dass** es mindestens eine Verbindung nach einem oder mehreren der Ansprüche 1 bis 8 enthält.

11. Elektrooptisches Anzeigeelement, enthaltend ein flüssigkristallines Medium nach Anspruch 10.

12. Verfahren zur Herstellung von Verbindungen der Formel I nach einem oder mehreren der Ansprüche 1 bis 8, **dadurch gekennzeichnet, dass** es einen Verfahrensschritt umfasst, wobei eine Verbindung der Formel 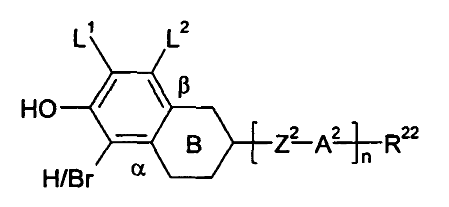 worin
n, A², L¹, L² und Z² wie für Formel I nach einem oder mehreren der Ansprüche 1 bis 8 definiert sind, und
R²² wie R¹ definiert ist und zusätzlich -OTs, OTf, OMes, -B(OH)₂, -B(OAlkyl)₂ oder -B<(O-C₂₋₁₀-Alkylen-O) bedeutet,
an der Hydroxylgruppe mit einem geeigneten organischen Rest zu einer Verbindung der Formel 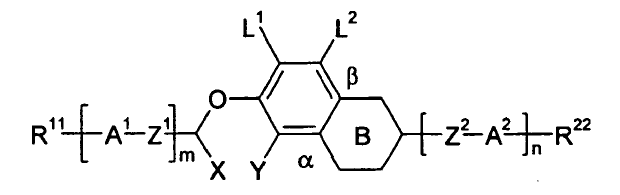 worin m, n, A¹, Z¹, A², L¹, L² und Z² wie für Formel I nach einem oder mehreren der Ansprüche 1 bis 8 definiert sind,
R¹¹ und R²² wie R¹ definiert sind und zusätzlich -OTs, -OTf, -OMes, -B(OH)₂, -B(OAlkyl)₂ oder -B<(O-C₂₋₁₀-Alkylen-O) bedeuten können,
X -CH₂CH₂Br oder -C≡CH-, und
Y H oder Br bedeuten,
verethert wird und in einem weiteren Verfahrensschritt cyclisiert wird.

## Claims

1. Compounds of the formula I: 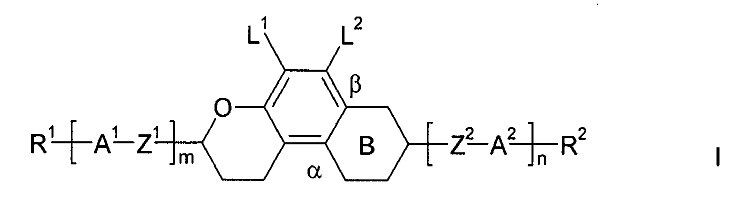 in which
m and n are each, independently of one another, 0, 1, 2 or 3, where m + n ≤ 4;
L¹ and L² each, independently of one another, denote H, halogen, CN, CF₃, CHF₂, CH₂F or CH₃, where one of the radicals L¹ or L² denotes an F or both of the radicals L¹ and L² denote an F;
A¹ and A² each, independently of one another, denote
(a) 1,4-phenylene, in which =CH- may be replaced once or twice by =N- and which may be unsubstituted or mono- to tetrasubstituted, independently of one another, by -CN, -F, -Cl, -Br, -I, C₁-C₆-alkanyl which is unsubstituted or mono-or polysubstituted by fluorine and/or chlorine, or C₁-C₆-alkoxy which is unsubstituted or mono- or polysubstituted by fluorine and/or chlorine,
(b) 1,4-cyclohexylene, 1,4-cyclohexenylene or 1,4-cyclohexadienylene, in which -CH₂- may be replaced once or twice, independently of one another, by -O- or -S- in such a way that heteroatoms are not linked directly, and which may be unsubstituted or mono- or polysubstituted by -F and/or -Cl, or
(c) bicyclo[1.1.1]pentane-1,3-diyl, bicyclo[2.2.2]octane-1,4-diyl or spiro[3.3]heptane-2,6-diyl;
Z¹ and Z² each, independently of one another, denote a single bond, -CF₂O-, -OCF₂-, -CH₂CH₂-, -CF₂CF₂-, -CF₂CH₂-, -CH₂CF₂-, -(CO)O-, -O(CO)-, -CH₂O-, -OCH₂-, -CF=CH-, -CH=CF-, -CF=CF-, -CH=CH-, -C≡C- or a combination of two of these groups, where O atoms are not linked directly;
R¹ and R², independently of one another, denote hydrogen, an alkanyl, alkoxy, alkenyl or alkynyl radical having 1 to 15 or 2 to 15 C atoms respectively which is unsubstituted, monosubstituted by -CN or mono- or polysubstituted by -F, -Cl and/or -Br, where, in addition, one or more CH₂ groups in these radicals may each be replaced, independently of one another, by -O-, -S-, -SO₂-, -CO-, -(CO)O-, -O(CO)- or -O(CO)O- in such a way that heteroatoms are not linked directly, or denote -F, -Cl, -Br, -CN, -SCN or -SF₅; and
ring B denotes 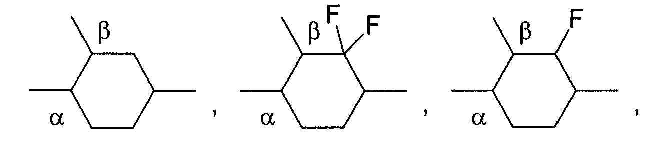 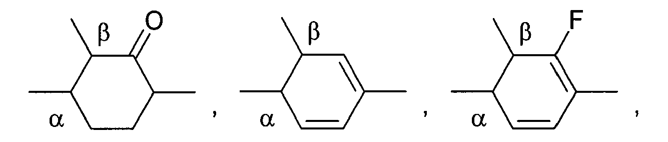 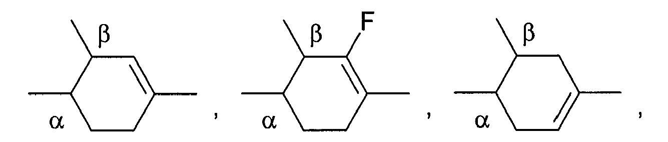 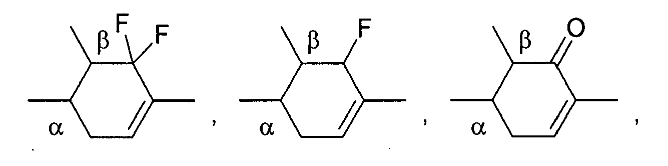 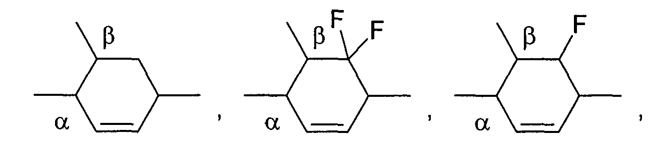 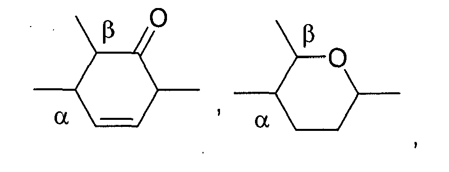 where
A¹ and A² or Z¹ and Z² may each have identical or different meanings if m or n is greater than 1,
where
R¹ and R² do not simultaneously denote H, and where,
in the case where R¹ = H and m = 0 or R² = H and n = 0,
then
ring B denotes a pyran of the formula

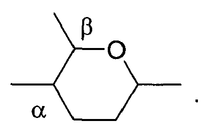

2. Compounds according to Claim 1, **characterised in that** the substituents L¹ and L² denote F.

3. Compounds according to Claim 1 or 2, **characterised in that** the compounds of the formula I are selected from the sub-formulae IA to IF 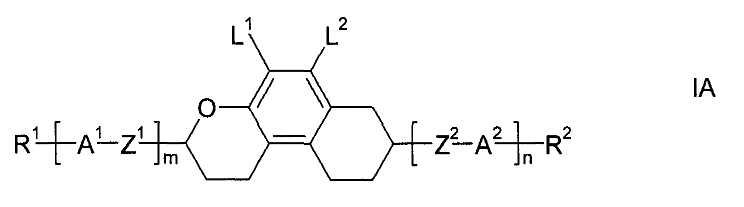 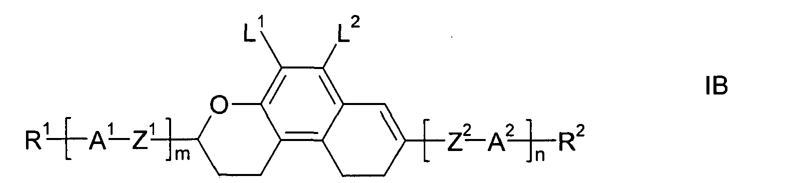 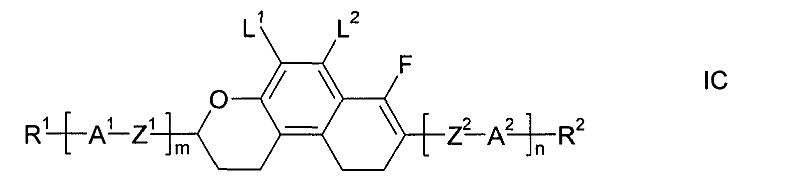 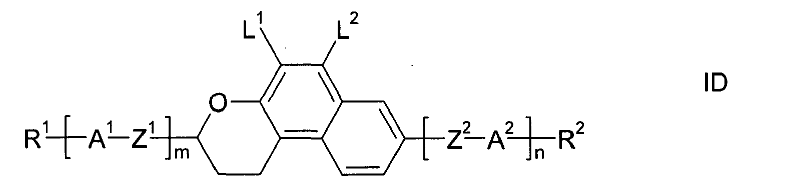 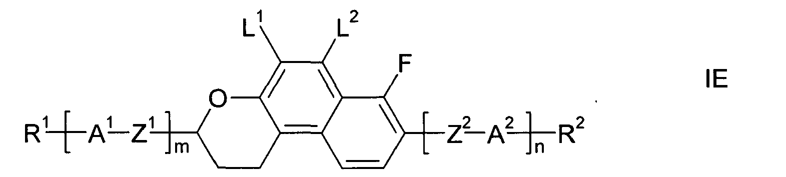 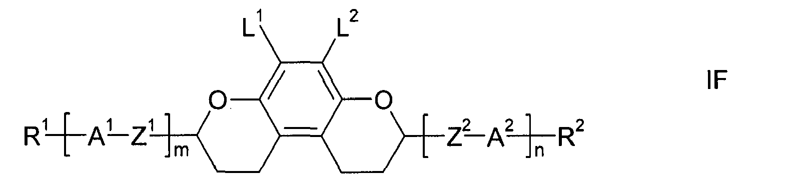 in which R¹, R², A¹, A², L¹, L², Z¹ and Z² have the same meanings as defined for the formula I.

4. Compounds according to one or more of Claims 1 to 3, **characterised in that**
Z¹ and Z², independently of one another, denote a single bond, -CF₂O-, -OCF₂-, -OCH₂-, -CH₂O-, -CF₂CF₂-, -CH=CH-, -CF=CH-, -CH=CF- or -CF=CF-.

5. Compounds according to one or more of Claims 1 to 4, **characterised in that** A¹ and A², independently of one another, denote a ring of the formula 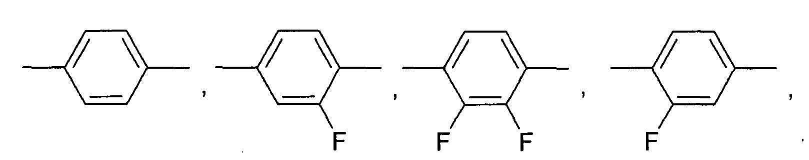 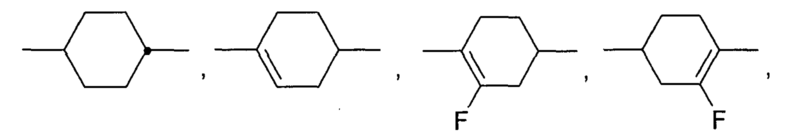 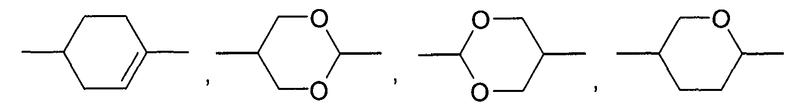 or

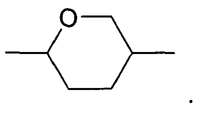

6. Compounds according to one or more of Claims 1 to 5, **characterised in that**
R¹ and R² are each, independently of one another, an alkanyl radical, alkoxy radical or alkenyl radical having 1 to 7 or 2 to 7 carbon atoms respectively, where each of these radicals is unsubstituted or mono- or polysubstituted by halogen, or denote fluorine or hydrogen.

7. Compounds according to one or more of Claims 1 to 6, **characterised in that**
m and n are both zero, and
R¹ and R² are each, independently of one another, an unbranched alkanyl radical, alkoxy radical or alkenyl radical having 1 to 7 or 2 to 7 carbon atoms respectively.

8. Compounds according to one or more of Claims 1 to 6, **characterised in that** m + n = 1, and
A¹, A², independently of one another, denote

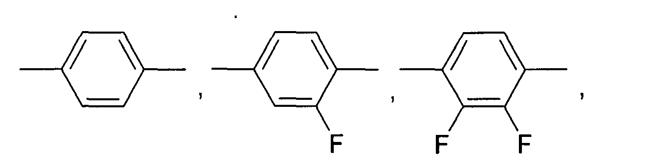

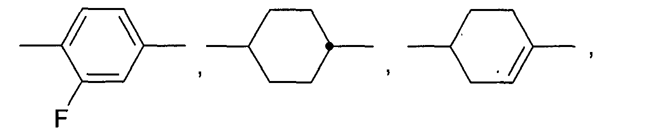

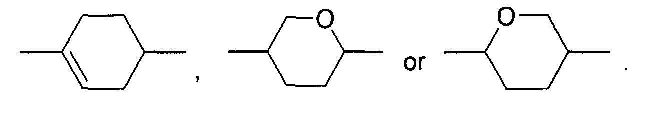

9. Use of one or more compounds according to one or more of Claims 1 to 8 in liquid-crystalline media.

10. Liquid-crystalline medium comprising at least two compounds, **characterised in that** it comprises at least one compound according to one or more of Claims 1 to 8.

11. Electro-optical display element containing a liquid-crystalline medium according to Claim 10.

12. Process for the preparation of compounds of the formula I according to one or more of Claims 1 to 8, **characterised in that** it includes a process step in which a compound of the formula 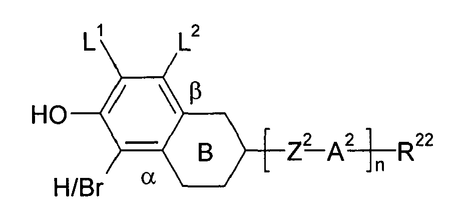 in which
n, A², L¹, L² and Z² are as defined for formula I according to one or more of Claims 1 to 8, and
R²² is as defined for R¹ and additionally denotes -OTs, -OTf, -OMes, -B(OH)₂, -B(O-alkyl)₂ or -B<(O-C₂₋₁₀-alkylene-O),
is etherified on the hydroxyl group using a suitable organic radical to give a compound of the formula 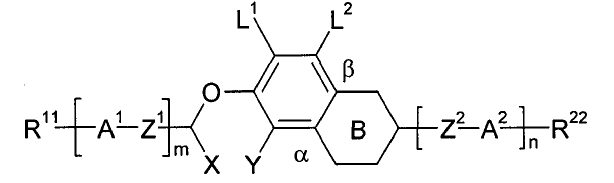 in which m, n, A¹, Z¹, A², L¹, L² and Z² are as defined for formula I according to one or more of Claims 1 to 8,
R¹¹ and R²² are as defined for R¹ and may additionally denote -OTs, -OTf, -OMes, -B(OH)₂, -B(O-alkyl)₂ or -B<(O-C₂₋₁₀-alkylene-O),
X denotes -CH₂CH₂Br or -C≡CH-, and
Y denotes H or Br,
and cyclised in a further process step.

## Revendications

1. Composés de la formule 1 : 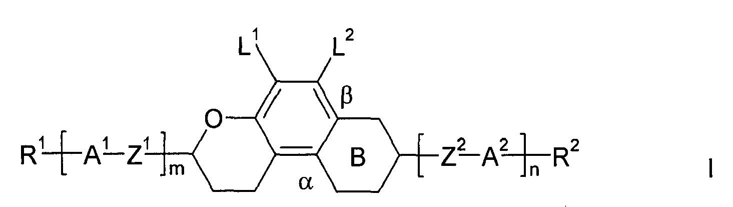 dans laquelle
m et n sont, chacun indépendamment de l'autre, 0, 1, 2 ou 3, où m + n ≤ 4 ;
L¹ et L² représentent, chacun indépendamment de l'autre, H, halogène, CN, CF₃, CHF₂, CH₂F ou CH₃, où l'un des radicaux L¹ ou L² représente un F ou les deux radicaux L¹ et L² représentent un F ;
A¹ et A² représentent, chacun indépendamment de l'autre,
(a) 1,4-phénylène, où =CH- peut être remplacé une fois ou deux fois par =N- et lesquels peuvent être non substitués ou mono- à tétrasubstitués, de manière indépendante, par -CN, -F, -Cl, -Br, -I, C₁-C₆-alkanyle qui est non substitué ou mono- ou polysubstitué par fluor et/ou chlore, ou par C₁-C₆-alcoxy qui est non substitué ou mono- ou polysubstitué par fluor et/ou chlore,
(b) 1,4-cyclohexylène, 1,4-cyclohexénylène ou 1,4-cyclohexadiénylène, où -CH₂- peut être remplacé une fois ou deux fois, de manière indépendante, par -O- ou -S- de telle sorte que des hétéroatomes ne soient pas liés directement, et lesquels peuvent être non substitués ou mono- ou poly-substitués par -F et/ou -Cl, ou
(c) bicyclo[1.1.1]pentane-1,3-diyle, bicyclo[2.2.2]octane-1,4-diyle ou spiro[3.3]heptane-2,6-diyle ;
Z¹ et Z² représentent, chacun indépendamment de l'autre, une liaison simple, -CF₂O-, -OCF₂-, -CH₂CH₂-, -CF₂CF₂-, -CF₂CH₂-, -CH₂CF₂-, -(CO)O-, -O(CO)-, -CH₂O-, -OCH₂-, -CF=CH-, -CH=CF-, -CF=CF-, -CH=CH-, -C=C- ou une combinaison de deux de ces groupes, où des atomes de O ne sont pas liés directement ;
R¹ et R² représentent, indépendamment l'un de l'autre, hydrogène, un radical alkanyle, alcoxy, alkényle ou alkynyle comportant respectivement de 1 à 15 ou de 2 à 15 atomes de C, lequel est non substitué, monosubstitué par -CN ou mono-ou polysubstitué par -F, -Cl et/ou -Br, où, en outre, un ou plusieurs groupes CH₂ dans ces radicaux peuvent chacun être remplacés, indépendamment les uns des autres, par -O-, -S-, -SO₂-, -CO-, -(CO)O-, -O(CO)- ou -O(CO)O- de telle sorte que des hétéroatomes ne soient pas liés directement, ou représentent -F, -Cl, -Br, -CN, -SCN ou -SF₅ ; et
cycle B représente 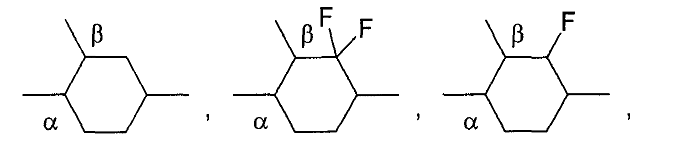 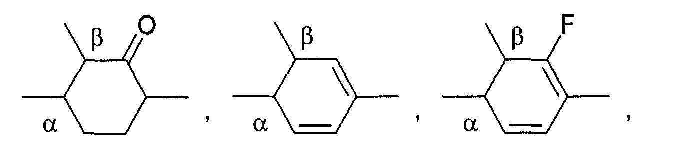 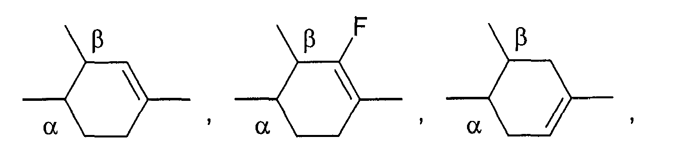 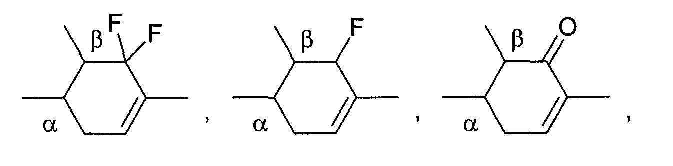 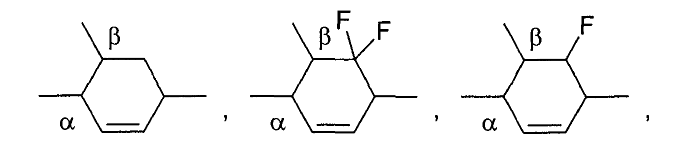 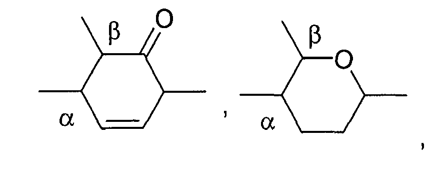 où
A¹ et A² ou Z¹ et Z² peuvent chacun présenter des significations identiques ou différentes si m ou n est supérieur à 1,
où
R¹ et R² ne représentent pas simultanément H, et où,
dans le cas où
R¹ = H et m = 0 ou R² = H et n = 0,
alors
cycle B représente un pyrane de la formule

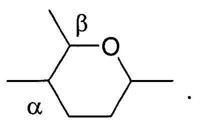

2. Composés selon la revendication 1, **caractérisés en ce que** les substituants L¹ et L² représentent F.

3. Composés selon la revendication 1 ou 2, **caractérisés en ce que** les composés de la formule I sont choisis parmi les sous-formules IA à IF 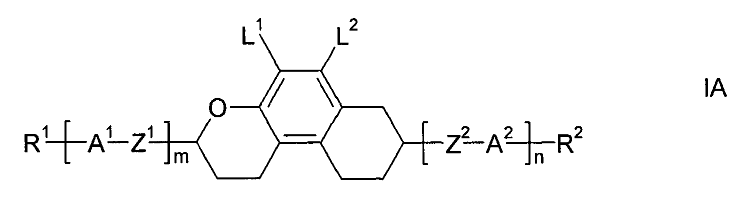 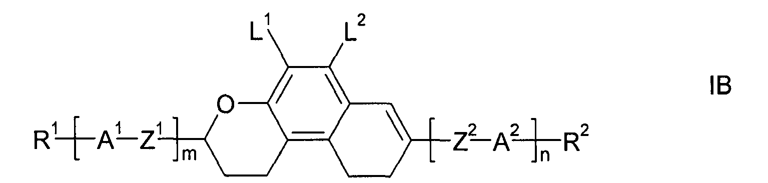 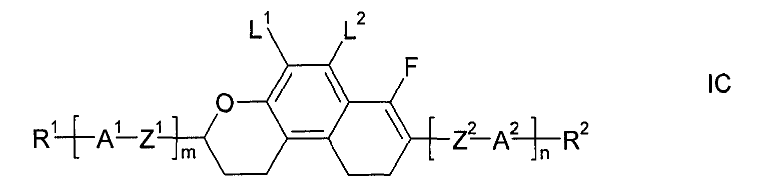 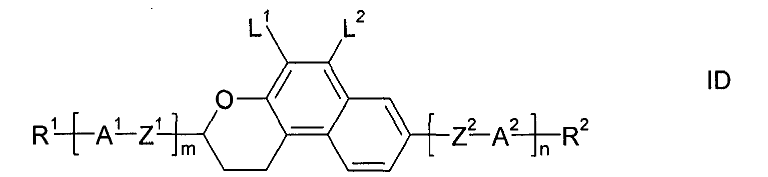 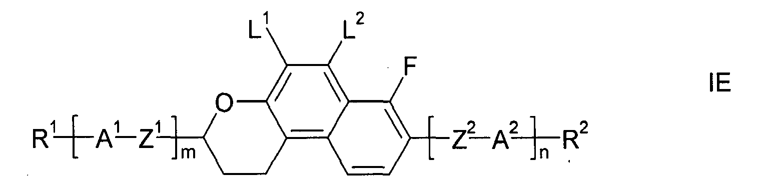 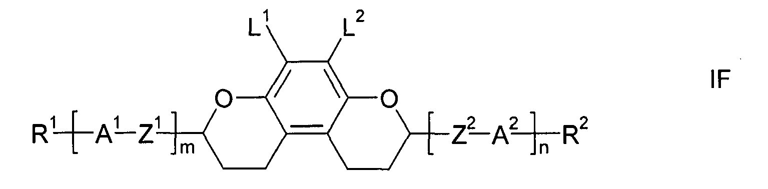 dans lesquelles R¹, R², A¹, A², L¹, L², Z¹ et Z² présentent les mêmes significations que défini pour la formule I.

4. Composés selon une ou plusieurs des revendications1 à 3, **caractérisés en ce que**
Z¹ et Z² représentent, indépendamment l'un de l'autre, une liaison simple, -CF₂O-, -OCF₂-, -OCH₂-, -CH₂O-, -CF₂CF₂-, -CH=CH-, -CF=CH-, -CH=CF- ou -CF=CF-.

5. Composés selon une ou plusieurs des revendications 1 à 4, **caractérisés en ce que** A¹ and A² représentent, indépendamment l'un de l'autre, un cycle de la formule 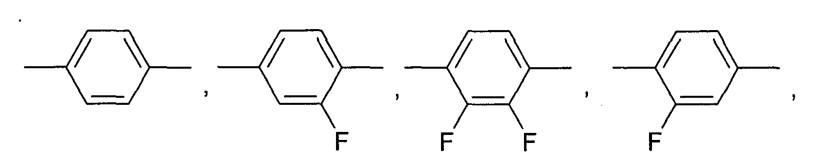 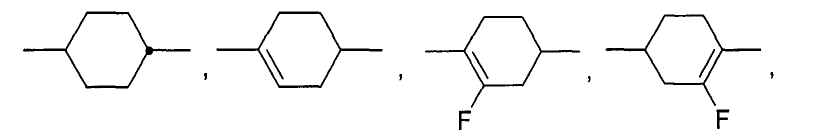 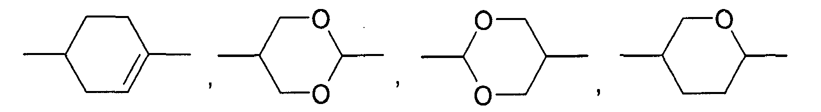 ou

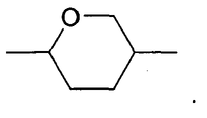

6. Composés selon une ou plusieurs des revendications 1 à 5, **caractérisés en ce que**
R¹ et R² sont, chacun indépendamment de l'autre, un radical alkanyle, un radical alcoxy ou un radical alkényle comportant respectivement de 1 à 7 ou de 2 à 7 atomes de carbone, où chacun de ces radicaux est non substitué ou mono- ou polysubstitué par halogène, ou représentent fluor ou hydrogène.

7. Composés selon une ou plusieurs des revendications 1 à 6, **caractérisés en ce que**
m et n sont tous deux zéro, et
R¹ et R² sont, chacun indépendamment de l'autre, un radical alkanyle non ramifié, un radical alcoxy ou un radical alkényle comportant respectivement de 1 à 7 ou de 2 à 7 atomes de carbone.

8. Composés selon une ou plusieurs des revendications 1 à 6, **caractérisés en ce que** m + n = 1, et
A¹, A² représentent, indépendamment l'un de l'autre,

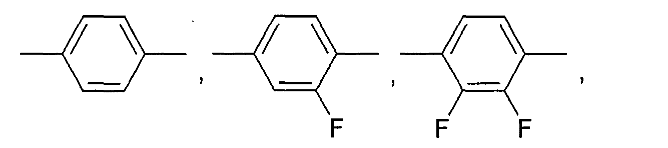

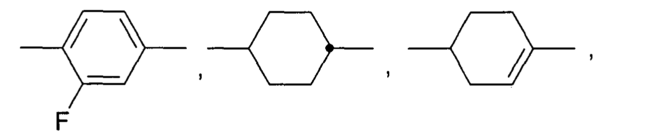

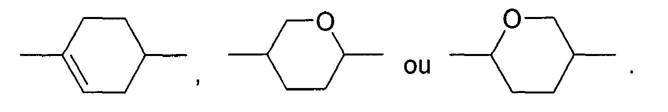

9. Utilisation d'un ou de plusieurs composés selon une ou plusieurs des revendications 1 à 8 dans des milieux cristallins liquides.

10. Milieu cristallin liquide comprenant au moins deux composés, **caractérisé en ce qu'**il comprend au moins un composé selon une ou plusieurs des revendications 1 à 8.

11. Elément d'affichage électro-optique contenant un milieu cristallin liquide selon la revendication 10.

12. Procédé pour la préparation de composés de la formule I selon une ou plusieurs des revendications 1 à 8, **caractérisé en ce qu'**il inclut une étape de procédé selon laquelle un composé de la formule 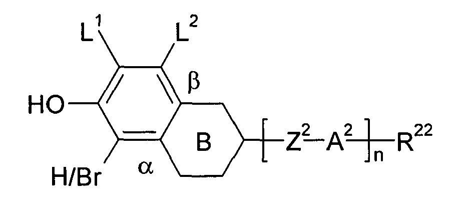 dans laquelle
n, A², L¹, L² et Z² sont comme défini pour la formule I selon une ou plusieurs des revendications 1 à 8, et
R²² est comme défini pour R¹ et additionnellement, représente -OTs, -OTf, -OMes, -B(OH)₂, -B(O-alkyl)₂ ou -B<(O-C₂₋₁₀-alkylène-O),
est éthérifié sur le groupe hydroxyle en utilisant un radical organique approprié pour obtenir un composé de la formule 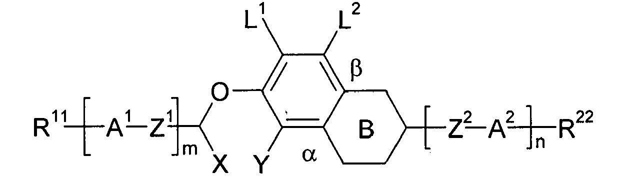 dans laquelle m, n, A¹, Z¹, A², L¹, L² et Z² sont comme défini pour la formule I selon une ou plusieurs des revendications 1 à 8,
R¹¹ et R²² sont comme défini pour R¹ et peuvent additionnellement représenter -OTs, -OTf, -OMes, -B(OH)₂, -B(O-alkyl)₂ ou -B<(O-C₂₋₁₀-alkylène-O),
X représente -CH₂CH₂Br ou -C≡CH-, et
Y représente H ou Br,
et est cyclisé selon une autre étape de procédé.
